# EUROPEAN PATENT APPLICATION

(11) **EP 3 919 076 A1**
(43) Date of publication of application: **08.12.2021**
(21) Application number: 20177903.0
(22) Date of filing: 02.06.2020
(51) Int. Cl.: A61K 39/09, A61K 31/04, A61K 39/00

(54) **SYNTHETIC OLIGOSACCHARIDE VACCINES AGAINST STREPTOCOCCUS PNEUMONIAE WITH MICROPARTICLE ADJUVANT FORMULATIONS**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention relates to a vaccine comprising at least one glycoconjugate of synthetic *Streptococcus pneumoniae* oligosaccharides and a carrier protein, and at least one agonist or activator of toll-like receptors such as TLR1, 2, 4, 6, 7, 8, or 9, wherein the at least one glycoconjugate and the at least one adjuvant are encapsulated in biodegradable polymer particles. The vaccine of present invention is useful for the prevention and/or treatment of diseases caused by *Streptococcus pneumoniae,* in particular *Streptococcus pneumoniae* serotype 3.

## Description

### Specification

The present invention relates to a vaccine comprising at least one glycoconjugate of synthetic *Streptococcus pneumoniae* oligosaccharides and a carrier protein, and at least one agonist or activator of toll-like receptors such as TLR1, 2, 4, 6, 7, 8, or 9, which serves as adjuvant, wherein at least one glycoconjugate and at least one adjuvant are encapsulated in biodegradable polymer particles. The vaccine of the present invention is useful for the prevention and/or treatment of diseases caused by *Streptococcus pneumoniae,* in particular *Streptococcus pneumoniae* serotype 3.

### Background of the invention

The Gram-positive bacterium *Streptococcus pneumoniae* is one of the main pathogens and causes severe invasive diseases like meningitis, pneumonia or bacteremia. Particularly infants, the elderly and immunocompromised patients are at high risk towards pneumococcal infections. About more than 90 serotypes of *Streptococcus pneumoniae* have been identified throughout the world, with a small number of these serotypes accounting for most diseases. The serotypes are identified by their different capsular polysaccharide (CPS) structures. The capsular polysaccharide consists of polymers of repeating oligosaccharide units, which represent the epitope of the bacteria targeted by the vaccine. These capsular polysaccharides or parts of them are often immunogenic and constitute potential candidates for pneumococcal vaccines.

The resistance of *Streptococcus pneumoniae* to antibiotics is a rapidly increasing problem. Thus, pneumococcal vaccines providing protection against pneumococcal infections are becoming increasingly important. Two classes of pneumococcal vaccines are currently available, one based on polysaccharides and the other based on polysaccharides conjugated to a carrier protein. The polysaccharides are thymus-independent type 2 antigens and thus induce low-affinity antibodies. In addition, they evoke no B-cell memory. Pneumococcal conjugate vaccines can circumvent these disadvantages with an increased serotype-specific antibody response to capsular polysaccharides. The conjugate vaccine Prevnar13®(PCV13) contains immunogenic conjugates comprising the purified polysaccharides of 13 different *S*. *pneumoniae* serotypes covalently linked to a protein, such as CRM₁₉₇.

At least 98 *S*. *pneumoniae* serotypes can be distinguished based on their CPS. Currently available CPS-based pneumococcal vaccines contain the serotypes most frequently associated with invasive pneumococcal diseases (IPDs). Although the licensed 23-valent polysaccharide vaccine Pneumovax 23® is not effective in younger children, the conjugate vaccines Prevnar13® and Synflorix® cover thirteen and ten serotypes respectively and are highly successful in all age groups. Nevertheless, the plasticity of the pneumococcal genome means that the pathogen has the potential to adapt to the selective pressure of vaccines. The serotype replacement due to vaccination and regional differences in dominant serotypes necessitate the expansion of existing vaccines to include additional serotypes. New strains, e.g., serotype 19A and 22F, arise and replace previous colonizing strains under this selective pressure.

*Streptococcus pneumoniae* type 3 (ST3) is part of the current pneumococcal vaccines. The capsular polysaccharide of ST3 consists of [→3)-β-D-Glc*p*A-(1→4)-β-D-Glc*p*-(1→] repeating units. Immunization experiments with ST3 oligosaccharides conjugated to CRM₁₉₇ showed that increasing IgG antibody titers were found with increasing chain length (from monosaccharide to tetrasaccharide) and no influence on the immunogenicity by the oligosaccharide/CRM₁₉₇ ratio (Benaissa-Trouw et al., Infection and Immunity, 2001, 69, 4698 - 4701).

*S*. *pneumoniae* serotype 3 (ST3) is an important cause of invasive pneumococcal disease, particularly pneumonia, in both children and adults. Serotype 3 conjugate was added to the formulation of 13-valent pneumococcal vaccine (Prevnar13®, PCV13) and is now included in the routine immunization schedule. However, this particular serotype remains a prominent cause of the invasive pneumococcal disease (IPD) in all age groups in most countries using Prevnar13®. Many studies show no change in the incidence rate of serotype 3 after the introduction of PCV13 (Moore MR, et al. 2015, The Lancet. Infectious diseases 15(3): 301-309). *S*. *pneumoniae* serotype 3 produces a very thick mucoid capsule which protects the bacteria from phagocytosis, inhibits opsonization by complement, helps to escape the neutrophil extracellular traps and prevents macrophage killing. Lack of clinical efficacy against serotype 3 after pneumococcal conjugate vaccination may be a result of reduced induction of immune memory. It was found that the levels of pre-existing ST3-specific antibody are negatively correlated with the B cell memory response to a booster dose of PCV13 containing ST3 glycoconjugate. Therefore, improvement by alternative approaches is needed to advance vaccines for this highly invasive pneumococcal serotype.

Synthetic oligosaccharides based on ST3 capsular polysaccharide repeating units have been shown to protect mice against lethal systemic challenge with ST3 pneumococci. A highly immunogenic tetrasaccharide glycotope based on the disaccharide repeating unit of *S*. *pneumoniae* serotype 3 was synthesized (below). The semi-synthetic oligosaccharide-based glycoconjugate vaccine candidate shows an immunoprotective effect against experimental pneumonia caused by transnasal infection with ST3 strain PN36. However, the vaccine needs further enhancement to achieve long-lived immune memory (Parameswarappa SG, et al. 2016, Cell Chemical Biology 23(11):1407-1416).

Vaccine adjuvants are molecules or compounds that have intrinsic immunomodulatory properties and, when administered in conjunction with an antigen, effectively potentiate the host antigen-specific immune responses compared to responses raised when antigen is given alone. In addition to enhancing the immunogenicity, adjuvants also reduce the total amount of antigens and the number of immunizations required to achieve an adequate level of protective immunity. A broad range of compounds such as mineral salts, saponins, liposomes, and particulate compounds have all been considered as potential vaccine adjuvants.
The adjuvant is a crucial portion of a vaccine and, as has been shown in many studies, significantly changes the immune response induced by the vaccination. Alum-based adjuvants are currently the most popular type of adjuvants used in commercial vaccines. Throughout the conflicting evidence and without a fully defined mechanism for alum's adjuvanticity, the general consensus is that alum increases recognition and uptake of antigen. However, alum cannot be the most effective adjuvant in every case. Alum salts, for instance, promote necrosis and DNA release in vivo with consequent activation of IRF3, and enhanced migration of inflammatory monocytes to draining lymph nodes after IP injection. Moreover phagocytosis of particulates including alum can lead to lysosomal rupture and release of enzymes into the cytosol, including cathepsin B, which can mediate pyroptosis, cell death associated with NLRP3 activation. Moreover, very often alum adjuvants do not allow to reveal the maximum capability of the antigen as the mechanism, by which they improve the immune response, is still not fully understood. Oil-in-water adjuvants are known to be very effective for prophylaxis in human. Typical examples are MF59™ and AS03. MF59™ is a squalene-based oil-water emulsion which was licensed in Europe for influenza vaccines especially for the elderly and young children. AS03 is similar squalene-based oil-in-water emulsion containing the adjuvant alpha tocopherol. However, an issue of side effects such as narcolepsy was raised for AS03.

### Vaccine Delivery System

Appropriate vaccine administration/delivery is the key element to ensure successful vaccination. Typically, most vaccines are administered via the subcutaneous (SC) or intramuscular (IM) routes. Hypodermic injections are associated with pain and distress that might lead to poor patient compliance and require highly trained personnel for administration. They are associated with a risk of disease transmission due to the possibility of needle-stick injuries or reuse of contaminated needles. Insufficient vaccine supply or limitation of vaccine production may also prove problematic in instances when mass vaccination is necessary.
Consequently, antigen delivery by hypodermic injection will bypass the skin's immune cells leading to less efficient vaccination. For this reason, the skin represents an ideal site for vaccine delivery, as vaccination at this site will evoke strong immune responses at much lower doses of antigen than intramuscular vaccines. Other areas of the body have also recently shown promise as targets for vaccine delivery, including the nasal mucosa and the gastrointestinal tract. These alternate sites of delivery offer the prospect for eliciting immune responses that are qualitatively different from those of injected vaccines or that stimulate immune responses at these mucosal sites for more effective defense against pathogens that invade by these routes, e.g., oral or nasal.

Therefore, it is necessary to widely investigate and to find most effective adjuvants and novel vaccine delivery systems in order to enhance immunogenicity against S. *pneumoniae* infections, as well as vaccine stability.

It is the objective of the present invention to provide a vaccine comprising glycoconjugates of synthetic *Streptococcus pneumoniae* oligosaccharides with carrier proteins and at least one adjuvant in an effective vaccine delivery system.

The objective of the present invention is solved by providing a vaccine comprising at least one glycoconjugate of a synthetic *Streptococcus pneumoniae* oligosaccharide with a carrier protein and at least one adjuvant selected from agonists of toll-like receptors, wherein the at least one glycoconjugate and the at least one adjuvant are encapsulated in biodegradable polymer particles as an effective vaccine delivery system.

One of the greatest benefits of the particulate antigen delivery systems is their ability to co-deliver antigens and immunostimulatory molecules simultaneously to the same antigen presenting cell.

The inventive vaccine is useful in the prevention and/or treatment of a disease caused by *Streptococcus pneumoniae,* in particular *Streptococcus pneumoniae serotypes 3,* in a human or an animal host.

The objective of the present invention is solved by the teaching of the independent claims. Further advantageous features, aspects and details of the invention are evident from the dependent claims, the description, the figures, and the examples of the present application.

### Brief description of the invention

The present invention is directed to a vaccine comprising
a) at least one glycoconjugate of a synthetic *Streptococcus pneumoniae* oligosaccharide and a carrier protein;
b) at least one adjuvant, wherein the at least one adjuvant is selected from agonists of toll-like receptors 1, 2, 4, 6, 7, 8, or 9,
   wherein the at least one glycoconjugate and the at least one adjuvant are encapsulated in biodegradable polymer particles.

In one embodiment, the biodegradable polymer particles are preferably selected from poly(lactic-co-glycolic acid) particles, polylactic acid particles, polyglycolic acid particles, poly(lactic-co-glycolic acid) / polyethylene glycol co-polymers particles and tri-block poly(lactic-co-glycolic acid)/ polyethylene glycol/ poly(lactic-co-glycolic acid)/ copolymers particles. More preferably, the biodegradable polymer particles are poly(lactic-co-glycolic acid) particles.

In some embodiments, the synthetic *Streptococcus pneumoniae* oligosaccharide is preferably selected from the group consisting of serotypes 1, 2, 3, 4, 5, and 8. More preferably, the synthetic *Streptococcus pneumoniae* oligosaccharide is of *Streptococcus pneumoniae* serotype 3.

In some embodiments, the carrier protein is preferably selected from CRM₁₉₇, detoxified pneumolysin or an immunogenic fragment of detoxified pneumolysin, pneumococcal surface protein A, an immunogenic fragment of pneumococcal surface protein A, and tetanus toxoid. More preferably, the carrier protein is CRM₁₉₇.

In some embodiments, the at least one adjuvant is selected from monophosphoryl lipid A, an imidazoquinoline compound, preferably resiquimod, a mixture of monophosphoryl lipid A and resiquimod, and isolated bacterial RNA.

In some embodiments, a ratio of the at least one glycoconjugate and the at least one adjuvant is 1 : 1 to 1 : 100 (w/w).

In some embodiments, the vaccine of the present invention comprises the at least one glycoconjugate having the general formula (**III**)

**[H-(C)_{c3}-(D-C)_{c2}-(D)_{c1}-O-L³-NH-W³]ₘ₃- carrier protein** **(III)**

wherein
C represents: 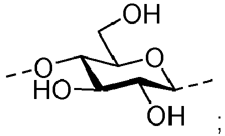
D represents: 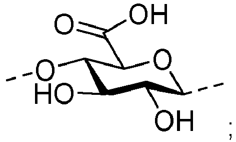
c2 is an integer selected from 1, 2, 3, 4, 5, 6, 7, 8, 9 and 10;
c1 and c3 represent independently of each other an integer 0 or 1;
L³ represents a linker;
m³ is an integer between 2 and 18;
-W³- is selected from: 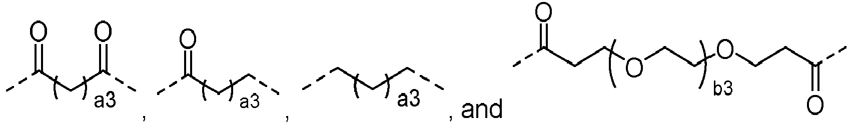
   a3 represents an integer from 1 to 10;
   b3 represents an integer from 1 to 4; and
   carrier protein is preferably selected from CRM₁₉₇, detoxified pneumolysin or an immunogenic fragment of detoxified pneumolysin, pneumococcal surface protein A, an immunogenic fragment of pneumococcal surface protein A, and tetanus toxoid. More preferably, the carrier protein is CRM₁₉₇.

More preferably, the vaccine of the present invention comprises the at least one glycoconjugate having the following formula **(IIIa)** 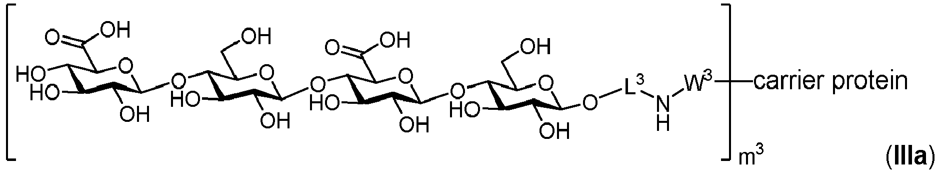
wherein m³ represents an integer from 3 to 12;
L³ represents -CH₂CH₂-;
W³ represents -COCH₂CH₂CH₂CH₂CO-, or -COCH₂CH₂CH₂CH₂CH₂;
carrier protein is preferably selected from CRM₁₉₇, detoxified pneumolysin or an immunogenic fragment of detoxified pneumolysin, pneumococcal surface protein A, an immunogenic fragment of pneumococcal surface protein A, and tetanus toxoid. More preferably, the carrier protein is CRM₁₉₇, and
the at least one adjuvant is selected from monophosphoryl lipid A (MPLA), resiquimod (R848), a mixture of MPLA and R848, and isolated bacterial RNA.

In some embodiments, the vaccine of the present invention further comprises at least one immunogenic component, wherein the at least one immunogenic component is selected from the group consisting of:
detoxified pneumolysin (Ply), or an immunogenic fragment thereof;
a pneumococcal surface antigen A, or an immunogenic fragment thereof,
a pneumococcal surface protein A, or an immunogenic fragment thereof, and a glycosphingolipid.

In some embodiments, the vaccine of the present invention further comprises one or more glycoconjugates of capsular polysaccharides from *Streptococcus pneumoniae* serotypes 1, 3, 4, 5, 6A, 6B, 7F, 9V, 14, 18C, 19A, 19F and 23F individually conjugated to CRM₁₉₇ carrier protein.

In another embodiment, the present invention is directed to a vaccine for use in the prevention and/or treatment of a disease caused by *Streptococcus pneumoniae* in a human or an animal host. Preferably, the vaccine of the present invention is useful in the prevention and/or treatment of a disease caused by *Streptococcus pneumoniae* serotype 3.

In particular, the disease caused by *Streptococcus pneumoniae* includes pneumonia, meningitis, otitis media, bacteremia and acute exacerbation of chronic bronchitis, invasive pneumococcal disease, exacerbation of chronic obstructive pulmonary disease, sinusitis, arthritis and conjunctivitis.

Optionally, said vaccine is in a nasal formulation, preferably nasal drops, nasal powder, a nasal gel or a nasal spray.

### Description of the invention

### Definitions

As used herein, the term **"conjugate"** or **"glycoconjugate"** refers to a conjugate between a protein, i.e. a carrier protein, and an antigen, i.e. a synthetic *Streptococcus pneumoniae* oligosaccharide.

The term **"oligosaccharide"** as used herein refers to a saccharide which has a number of monosaccharides in a range of 2 to 40, preferred 3 to 30, more preferred 4 to 25, still more preferred 4 to 20, and most preferred 4 to 15.
The number of monosaccharides of said oligosaccharide as used herein is smaller than that of the capsular polysaccharide of *Streptococcus pneumoniae.* The polysaccharide has usually more than some hundreds of monosaccharides.

As used herein, the term **"synthetic *Streptococcus pneumoniae* oligosaccharide"** refers to an oligosaccharide of capsular polysaccharide of *Streptococcus pneumoniae* and it is produced synthetically to have the same or similar structure that is produced in the native host cell (i.e., *S*. *pneumoniae*).
The term **"synthetic"** includes for example, saccharides produced by chemical synthesis, but not those obtained by recombinant expression in host cells, such as gram positive bacteria or gram negative bacteria.

As used herein the **"size"** of, for example, a conjugate or pneumococcal saccharide means the Molecular weight (Mw) of the conjugate or pneumococcal saccharide, respectively. The Mw may be provided in kilodaltons (kDa). As used herein "in the range" and "between" are inclusive of the range's upper and lower limits (for example, "in the range 30 - 300kDa" includes 30 kDa and 300kDa).

The term **"carrier protein"** as used herein refers to a protein covalently attached to an antigen (e.g. saccharide antigen) to create a conjugate (e.g. glycoconjugate or bioconjugate). A carrier protein activates T-cell mediated immunity in relation to the antigen to which it is conjugated. A "carrier protein" is preferably non-toxic and non-reactogenic and obtainable in sufficient amount and purity. A carrier protein is amenable to standard conjugation procedures.

The nature of the carrier protein is critical in defining the magnitude and quality of vaccine-induced immune responses, and the duration of protection. Different aspects, such as competition with anti-carrier antibodies elicited by related vaccination against tetanus and diphtheria, an overload of carrier protein or carrier-mediated epitope suppression, may influence both T- and B- cell responses to glycoconjugate vaccines. Additionally, differences in extent and persistence of protective antibodies triggered by primary vaccination with bacterial polysaccharides conjugated to various carrier proteins have been observed in infants.

Carrier proteins known in the prior art include inactivated bacterial toxins such as diphtheria toxoid, tetanus toxoid (TT), pertussis toxoid (PT), cholera toxoid (CT), E. *coli* LT, *E. coli* ST, and exotoxin A from *Pseudomonas aeruginosa.* Bacterial outer membrane proteins such as outer membrane complex c (OMPC), porins, transferrin binding proteins, detoxified pneumolysin, pneumococcal surface protein A (PspA), pneumococcal adhesin protein (PsaA), C5a peptidase from Group A or Group B streptococcus, *Haemophilus influenzae* protein D (PD), or a member of the polyhistidine triad family (Pht) proteins. Other proteins, such as ovalbumin, keyhole limpet hemocyanin (KLH), bovine serum albumin (BSA) or purified protein derivative of tuberculin (PPD) can also be used as carrier proteins. Fragments or fusion proteins of the aforementioned proteins can also be used as carrier protein.

The diphtheria toxoid includes CRM₂₂₈, CRM₁₉₇, CRM₁₇₃, CRM₄₅. In particular, the diphtheria toxoid CRM₁₉₇ which is a non-toxic variant (i.e. toxoid) of diphteria toxin isolated from cultures of *Corynebacterium diphteria strain* C7 (β197) grown on casamino acids and yeast extract-based medium, may also be used as carrier protein. CRM₁₉₇ is purified through ultra-filtration, ammonium sulfate precipitation, and ion-exchange chromatography. Alternatively, CRM₁₉₇ is prepared recombinantly in accordance with U.S. Patent No. 5,614,382.

As used herein, the term **"immunogenic fragment"** is a portion of an antigen smaller than the whole that is capable of eliciting a humoral and/or cellular immune response in a host animal, e.g. human, specific for that fragment. Fragments of a protein can be produced using techniques known in the art, e.g. recombinantly, by proteolytic digestion, or by chemical synthesis. Internal or terminal fragments of a polypeptide can be generated by removing one or more nucleotides from one end (for a terminal fragment) or both ends (for an internal fragment) of a nucleic acid which encodes the polypeptide. Typically, fragments comprise at least 10, 20, 30, 40 or 50 contiguous amino acids of the full length sequence. Fragments may be readily modified by adding or removing 1, 2, 3, 4, 5, 6, 7, 8, 9, 10, 20, 30, 40 or 50 amino acids from either or both of the N and C termini.

As used herein the term protein or fragment thereof also include **"variant"** of a protein or of a fragment thereof, and refers to a protein or fragment that shares a certain amino acid sequence identity with the reference protein or fragment upon alignment by a method known in the art. A variant of a protein or of a fragment thereof can include a substitution, insertion, deletion, frameshift or rearrangement in another protein. In some embodiments variants share at least 70%, 80%, 85%, 90%, 95% or 99% sequence identity with the native protein or with the fragment thereof.

Recitation of any protein provided herein encompasses a functional variant of the protein. The term "functional variant" of a protein refers to a variant of the protein that retains the ability to be integrated in or specifically targeted to the giant unilamellar vesicle.

The term **"mutated"** is used herein to mean a molecule which has undergone deletion, addition or substitution of one or more amino acids using known techniques for site directed mutagenesis or any other conventional method.

As used herein, the term **"deletion"** is the removal of one or more amino acid residues from the protein sequence. Typically, no more than about from 1 to 6 residues (e.g. 1 to 4 residues) are deleted at any one site within the protein molecule.

As used herein, the term **"addition"** or **"insertion"** is the addition of one or more non-native amino acid residues in the protein sequence. Typically, no more than about from 1 to 10 residues, (e.g. 1 to 7 residues, 1 to 6 residues, or 1 to 4 residues) are inserted at any one site within the protein molecule

As used herein, the term "**conservative amino acid substitution"** involves substitution of a native amino acid residue with a non-native residue such that there is little or no effect on the size, polarity, charge, hydrophobicity, or hydrophilicity of the amino acid residue at that position, and without resulting in decreased immunogenicity. For example, these may be substitutions within the following groups: valine, glycine; glycine, alanine; valine, isoleucine, leucine; aspartic acid, glutamic acid; asparagine, glutamine; serine, threonine; lysine, arginine; and phenylalanine, tyrosine. Conservative amino acid modifications to the sequence of a polypeptide (and the corresponding modifications to the encoding nucleotides) may produce polypeptides having functional and chemical characteristics similar to those of a parental polypeptide.

The term **"conserved protein"** refer to proteins having identical or similar sequences in nucleic acids (DNA and RNA) or proteins across species (orthologous sequences), or within a genome (paralogous sequences). Conservation indicates that a sequence has been maintained by natural selection.

### Virulence factors of S. pneumoniae

In an embodiment of the present invention, the carrier protein is detoxified pneumolysin (dPly). Pneumolysin, Uniprot ID: Q04IN8, is a 53 kDa protein that belongs to the family of cholesterol-dependent cytolysin. Pneumolysin is not actively secreted from the bacterium as it lacks a typical signal secretion leader sequence but escapes from the cell by either autolysis or the action of lytic antibiotics. Pneumolysin forms pores in the cell membrane by oligomerization and conformational change in the structure. The pores formed can be up to 350 Å in diameter with each pore containing up to 50 pneumolysin monomers. The formation of pores results in a cell membrane disintegration which helps the bacteria to spread in the body as well as increase the host cell death and disease manifestation. Pneumolysin is a relatively conserved protein across all serotypes of *S*. *pneumoniae.* However, at least 16 different naturally-occurring variants of pneumolysin have been identified. Pneumolysin is a multifunctional toxin with distinct activities such as complement activation, lysis of red blood cells (haemolysis) causing haemoglobin release, production of immune regulatory molecules, including cytokines as TNFα, IL-1, and IL-6, influencing neutrophil activity and neutrophil extracellular traps formation; favour of bacteria spread in the blood, influence of cell-signalling, cytoskeletal rearrangement and DNA damage induction; colonization of the host; causing endothelial hyper-permeability (pulmonary permeability edema), a major complication of pneumonia.

The evidence from animal infection studies points clearly to an integral role of pneumolysin in invasive pneumococcal diseases. Neutralization of the toxin seems to be a potentially valuable approach to treat pneumococcal diseases as well as an exciting vaccine candidate.

Detoxified pneumolysin (dPly) refers to a pneumolysin variant or mutant exhibiting significantly impaired lytic activity on human cells, such as epithelial cells, so that it is not toxic anymore for human or other animals.

Detoxification of pneumolysin can be conducted by chemical means, e.g., subject to formalin or glutaraldehyde treatment or a combination of both. Such methods are known in the art for various toxins. Alternatively, pneumolysin can be genetically detoxified. Thus, the invention encompasses variants of pneumococcal proteins which may be, for example, mutated proteins. For example, a mutant or mutated pneumolysin protein may be altered so that it is biologically inactive, i.e. not toxic, whilst still maintaining its immunogenic epitopes.

Mutant detoxified pneumolysin proteins suitable for the embodiments of the present invention includes PlyA370G (Ala370 → Gly), PlyA370E (Ala370 → Glu), PlyA406G (Ala406 → Gly), PlyA406E (Ala406 → Glu), PlyW433G (Trp433 → Gly), PlyW433E (Trp433 → Glu), PlyW433F (Trp433 → Phe), PlyL460G (Leu460 → Gly), and PlyL460E (Leu460 → Glu) (Taylor et al., PlosOne 2013), PlyC428G (Cys428 → Gly), PlyC428S (Cys428 →Ser), PlyE434D (Glu434 → Asp), Trp435 → Phe (WO1990006951A1).

As used herein, it is understood that the term **"Ply"** encompasses **mutated pneumolysin** and **detoxified pneumolysin (dPly),** which are suitable for medical use, as they are non toxic.

### Pneumococcal surface protein A - PspA

PspA is described to be an important pneumococcal virulence factor for inhibiting complement deposition and for protecting pneumococci from killing by apolactoferrin. This cross-reactive protein is widely known to be immunogenic and protective and is present in all pneumococcal strains.
PspA is composed of four different distinct regions (1) the C-terminal anchoring the protein to the pneumococcal surface, (2) a stretch of ten highly conserved 20-aminoacid repeats, (3) a proline-rich region which acts as a tether and allows greater flexibility and movement of the amino-terminus, and (4) a highly charged amino-terminus. Based on amino acid sequence ahead of the proline-rich region, PspA is classified into three families and six clades (Family 1, clades 1 and 2; Family 2, clades 3, 4 and 5; and the rarely isolated Family 3, clade 6). The interaction of PspA with the iron-saturated lactoferrin helps bacteria to adhere on the surface of the host. A highly polar electrostatic charge of PspA results in stabilization of the capsular charge and the predominant part of protein prevents C3-mediated binding of the host complement to pneumococci by competing with the C-reactive protein.
The multifunctional immune evasive properties of PspA are essential for pneumococcal nasopharyngeal colonization and invasion of *S*. *pneumoniae.* PspA is immunogenic and induces both the humoral (antibody production) and cellular (activation of phagocytes, cytotoxic T-lymphocytes, and the release of various cytokines) immune response.

A fragment or immunogenic fragment of pneumococcal surface protein A suitable for the present invention comprises the sequence between amino acids 32 - 318 of the protein sequence ID AAA27018.1.

Immunogenic fragments of pneumococcal surface protein A comprise immunogenic fragments of at least 50, 75, 100, 125, 150, 175, 200, 250, or 286 contiguous amino acids of sequence between amino acids 32 - 318 of the protein sequence ID AAA27018.1. In one embodiment the fragment is less than 50, 75, 100, 125, 150, 175, 200, 250, or 286 contiguous amino acids of sequence between amino acids 32 - 318 of the protein sequence ID AAA27018.1. The immunogenic fragments may elicit antibodies which can bind to the sequence between amino acids 32 - 318 of the protein sequence ID AAA27018.1. The immunogenic fragment may comprise a B and/or T cell epitope of the sequence between amino acids 32 - 318 of the protein sequence ID AAA27018.1.

Immunogenic fragments of pneumococcal surface protein A comprise immunogenic fragments having at least 70%, 80%, 85%, 90%, 95% or 99% sequence identity with the sequence between amino acids 32 - 318 of the protein sequence ID AAA27018.1.

As used herein, the term **"conjugation"** is the coupling of carrier protein to oligosaccharide. In the present invention, for example, the oligosaccharide is coupled with the carrier protein by amide coupling reaction and/or reductive amination.

The term **"linker"** as used herein encompasses molecular fragments capable of connecting the reducing-end monosaccharide of a saccharide with an immunogenic carrier or a solid support, optionally by binding to at least one interconnecting molecule. Thus, the function of the linker *per se* or together with the interconnecting molecule is to establish, keep and/or bridge a special distance between the reducing-end monosaccharide and an immunogenic carrier or a solid support. More specifically, one extremity of the linker is connected to the exocyclic oxygen atom at the anomeric center of the reducing-end monosaccharide and the other extremity is connected *via* the nitrogen atom with the interconnecting molecule, or directly with the immunogenic carrier or the solid support.

The **linker** or spacer group herein disclosed may be any moiety that enables to couple the oligosaccharide to a carrier molecule, i.e. a carrier protein or a glycosphingolipid. A large variety of such linker groups are known in the art and a suitable linker group can be selected in dependence from the respective carrier molecule. For example, the linker may be an aliphatic or aromatic residue, e.g. an alkyl(en) group or phenyl(en) group, comprising a reactive functional group, such as an amino group, preferably a primary amino group, (activated) carboxy group, aldehyde, azide, alkenyl or alkynyl group. In specific embodiments the linker may comprise a polyether or polyester chain. In particular, the linker is selected from the group comprising primary alkylamines, alkyl or aralkyl residues with a terminal aldehyde, azide, alkyne or alkene group or (activated) carboxy group, and alkylaryl and aryl residues, e.g. phenyl residues, comprising a reactive amine, an aldehyde or an azide group, or (activated) carboxy group.

The term **"adjuvant"** as used herein refers to an immunological adjuvant i.e. a material used in a vaccine composition that modifies or augments the effects of said vaccine by enhancing the immune response to a given antigen contained in the vaccine without being antigenically related to it. For the persons skilled in the art, classically recognized examples of immunological adjuvants include, but are not restricted to
(1) aluminum salts (alum), such as aluminum hydroxide, aluminum phosphate, aluminum sulfate, etc.;
(2) oil-in-water emulsion formulations (with or without other specific immunostimulating agents such as muramyl peptides (defined below) or bacterial cell wall components), such as, for example:
   (a) MF59™, containing 5% Squalene, 0.5% Tween 80, and 0.5% Span 85. MF59™ mostly acts by enhancing recruitment of antigen-presenting cells,
   (b) Syntex adjuvant formulation (SAF) is an effective adjuvant composed of a muramyl dipeptide derivative (threonyl-MDP) in an oil-in-water (o/w) emulsion vehicle. It contains 10% Squalene, 0.4% Tween 80, 5% Pluronic® L121 polymer, and Threonyl-MDP (N-acetyl muramyl-L-threonyl-D-isoglutamine) either microfluidized into a submicron emulsion or vortexed to generate a larger particle size emulsion, and
   (c) Ribi™ adjuvant system (RAS), (Corixa, Hamilton, MT) containing 2% Squalene, 0.2% Tween 80;
(3) saponins, which are a heterologous group of sterol glycosides and triterpenoid glycosides that are found in the bark, leaves, stems, roots and even flowers of a wide range of plant species. Saponins from the bark of the *Quillaia saponaria,* Molina tree have been widely studied as adjuvants. Saponins can also be commercially obtained from *Smilax ornata* (sarsaprilla), *Gypsophilla paniculata* (brides veil), and *Saponaria oficianalis* (soap root). Saponin adjuvant formulations include purified formulations, such as QS21, as well as lipid formulations, such as ISCOMs. Saponin compositions have been purified using HPLC and RP-HPLC. Saponin formulations may also comprise a sterol, such as cholesterol. Combinations of saponins and cholesterols can be used to form unique particles called immunostimulating complexes (ISCOMs). ISCOMs generally include a phospholipid such as phosphatidylethanolamine or phosphatidylcholine. Any known saponin can be used in ISCOMs. Preferably, the ISCOM includes one or more of QuilA, QHA & QHC;
(4) bacterial lipopolysaccharides, synthetic lipid A analogs such as aminoalkyl glucosamine phosphate compounds (AGP), or derivatives or analogs thereof, which are available from Corixa; one such AGP is 2-[(R)-3-tetradecanoyl-oxytetradecanoylamino]ethyl 2-deoxy-4-O-phosphono-3-O-[(R)-3-tetradecanoyloxytetradecanoyl]-2-[(R)-3-tetradecanoyloxytetradecanoylamino-(β-D-glucopyranoside, which is also known as 529 (formerly known as RC529), which is formulated as an aqueous form or as a stable emulsion, synthetic polynucleotides such as oligonucleotides containing CpG motifs;
(5) cytokines, such as interleukins (e.g., IL-1 , IL-2, IL-4, IL-5, IL-6, IL-7, IL-12, IL-15, IL-18, etc.), interferons (e.g., gamma interferon), granulocyte macrophage colony stimulating factor (GM-CSF), macrophage colony stimulating factor (M-CSF), tumor necrosis factor (TNF), costimulatory molecules B7-1 and B7-2, etc.;
(6) detoxified mutants of a bacterial ADP-ribosylating toxin such as a cholera toxin (CT) either in a wild-type or mutant form, for example, where the glutamic acid at amino acid position 29 is replaced by another amino acid, preferably a histidine, a pertussis toxin (PT), or an *E*. *coli* heat-labile toxin (LT), particularly LT-K63, LT-R72, CT-S109, PT-K9/G129; and
(7) other substances that act as immunostimulating agents to enhance the effectiveness of the composition. Muramyl peptides include, but are not limited to, N-acetyl-muramyl-L-threonyl-D-isoglutamine (thr-MDP), N-acetyl-normuramyl-L-alanine-2-(1'-2'dipalmitoyl-sn-glycero-3-hydroxyphosphoryloxy)-ethylamine (MTP-PE)
(8) microparticles formed from materials that are biodegradable and non-toxic. Such non-toxic and biodegradable materials include, but are not restricted to poly(α-hydroxy acid), polyhydroxybutyric acid, polyorthoester, polyanhydride, polycaprolactone;
(9) CD1d ligands, such as an α-glycosylceramide, phytosphingosine-containing α-glycosylceramides, OCH, KRN7000 [(2*S*,3*S*,4*R*)-1-*O*-(α-D-galactopyranosyl)-2-(*N*-hexacosanoylamino)-1,3,4-octadecanetriol], CRONY- 101, 3"-sulfo-galactosyl-ceramide;
(10) immunostimulatory oligonucleotides, such as CpG motif (a dinucleotide sequence containing an unmethylated cytosine residue linked by a phosphodiester bond to a guanosine residue), or Cpl motif (a dinucleotide sequence containing cytosine linked to inosine), or a double-stranded RNA, or an oligonucleotide containing a palindromic sequence, or an oligonucleotide containing a poly(dG) sequence. Immunostimulatory oligonucleotides can include nucleotide modifications or analogs such as phosphorothioate modifications and can be double-stranded or, except for RNA, single-stranded;
(11) compounds containing lipids linked to a phosphate-containing acyclic backbone, such as the TLR4 antagonist E5564;
(12) oil emulsions, e.g. Freund's adjuvant.

Theoretically, each molecule or substance that is able to favor or amplify a particular situation in the cascade of immunological events, ultimately leading to a more pronounced immunological response, can be defined as an **adjuvant.**

In principle, through the use of adjuvants in vaccine formulations, one can
- direct and optimize immune responses that are appropriate or desirable for the vaccine;
- enable mucosal delivery of vaccines, i.e. administration that results in contact of the vaccine with a mucosal surface such as buccal or gastric or lung epithelium and the associated lymphoid tissue;
- promote cell-mediated immune responses;
- enhance the immunogenicity of weaker immunogens, such as highly purified or recombinant antigens;
- reduce the amount of antigen or the frequency of immunization required to provide protective immunity; and
- improve the efficacy of vaccines in individuals with reduced or weakened immune responses, such as newborns, elderly people, and immunocompromised vaccine recipients.

Although little is known about their mode of action, it is currently believed that **adjuvants** augment immune responses by one of the following mechanisms:
- increasing the biological or immunologic half-life of antigens;
- improving antigen delivery to antigen-presenting cells (APCs), as well as antigen processing and presentation by the APCs, e.g., by enabling antigen to cross endosomal membranes into the cytosol after ingestion of antigen-adjuvant complexes by APC;
- mimicking danger inducing signals from stressed or damaged cells, which serve to initiate an immune response;
- inducing the production of immunomodulatory cytokines;
- biasing the immune response towards a specific subset of the immune system; and
- blocking the rapid dispersal of the antigen challenge.

The present invention is directed to a vaccine comprising
a) at least one glycoconjugate of a synthetic *Streptococcus pneumoniae* oligosaccharide and a carrier protein;
b) at least one adjuvant, wherein the at least one adjuvant is selected from agonists of toll-like receptors 1, 2, 4, 6, 7, 8, or 9,
   wherein the at least one glycoconjugate and the at least one adjuvant are encapsulated in biocompatible and biodegradable polymer particles.

Preferably, the carrier protein is selected from diphtheria toxoid, detoxified pneumolysin, or an immunogenic fragment of detoxified pneumolysin, pneumococcal surface protein A (PspA), or an immunogenic fragment of pneumococcal surface protein A.

The diphtheria toxoid includes CRM₂₂₈, CRM₁₉₇, CRM₁₇₃, CRM₄₅. In particular, CRM₁₉₇ is preferred as the carrier protein.

Thus, preferred, the carrier protein is selected from diphtheria toxoid, CRM₁₉₇, detoxified pneumolysin or an immunogenic fragment thereof, pneumococcal surface protein A, or an immunogenic fragment thereof, tetanus toxoid, diphtheria toxoid, preferably, the carrier protein is CRM₁₉₇.

Thus, the present invention refers to a vaccine comprising
a) at least one glycoconjugate of a synthetic *Streptococcus pneumoniae* oligosaccharide and a carrier protein,
   wherein the carrier protein is selected from diphtheria toxoid, detoxified pneumolysin, an immunogenic fragment of detoxified pneumolysin, pneumococcal surface protein A (PspA), an immunogenic fragment of pneumococcal surface protein A, and tetanus toxoid (TT);
b) at least one adjuvant, wherein the at least one adjuvant is selected from agonists of toll-like receptors 1, 2, 4, 6, 7, 8, or 9,
   wherein the at least one glycoconjugate and the at least one adjuvant are encapsulated in biocompatible and biodegradable polymer particles.

In one embodiment, the present invention is directed to a vaccine comprising
a) at least one glycoconjugate of a synthetic *Streptococcus pneumoniae* oligosaccharide and a carrier protein,
   wherein the carrier protein is selected from CRM₁₉₇, detoxified pneumolysin, or an immunogenic fragment of detoxified pneumolysin, pneumococcal surface protein A (PspA), or an immunogenic fragment of pneumococcal surface protein A, and tetanus toxoid (TT);
b) at least one adjuvant, wherein the at least one adjuvant is selected from agonists of toll-like receptors 1, 2, 4, 6, 7, 8, or 9,
   wherein the at least one glycoconjugate and the at least one adjuvant are encapsulated in biocompatible and biodegradable polymer particles.

In one preferred embodiment, the present invention is directed to a vaccine comprising
a) at least one glycoconjugate of a synthetic *Streptococcus pneumoniae* oligosaccharide and a carrier protein, the carrier protein is CRM₁₉₇;
b) at least one adjuvant, wherein the at least one adjuvant is selected from agonists of toll-like receptors 1, 2, 4, 6, 7, 8, or 9,
   wherein the at least one glycoconjugate and the at least one adjuvant are encapsulated in biocompatible and biodegradable polymer particles.

In the present invention, the vaccine comprises agonists of toll-like receptors (TLRs) 1, 2, 4, 6, 7, 8, or 9, is used as the at least one adjuvant.

Among the known toll-like receptors (TLRs) in humans, TLR1, 2, 4, 5, and 6 recognize bacterial components, TLR3, 7, and 8 recognize viral RNA, and TLR9 detect unmethylated DNA.

TLR1 agonists comprise synthetic bacterial lipoprotein. Agonists of TLR2 or TLR4 comprise non-protein microbial components such as lipopolysaccharides, oligonucleotides, and lipopeptides. Preferably, TLR4 agonists comprise monophosphoryl lipid A (MPLA) and CRX-524. MPLA may be combined with alum.

TLR2 in combination with TLR1 and TLR6 is primarily involved in the recognition of gram-positive bacteria and fungal species. For example, triacyl-lipoproteins activate TLR-2 in combination with TLR-1, whereas diacyl-lipoproteins are recognized by TLR-2 in combination with TLR-6.

Among the different TLRs, TLR7 and TLR8, which recognize uridine- and/or guanosine-rich viral single stranded RNA (ssRNA), are broadly expressed on dendritic cells (DCs) and other antigen-presenting cells, making TLR7/8 agonists especially valuable for the development of vaccine adjuvants. Human TLR7 is mainly expressed on plasmacytoid DC and B cells and its activation induces IFNα via the IRF7 pathway and proinflammatory cytokines via the NFκB pathway. Human TLR8 is mainly expressed on monocytes, macrophages, neutrophils, and conventional DCs, including a small population of BDCA3⁺ cells shown to be very effective at cross-presenting exogenous antigens to CD8 T cells, and its activation induces proinflammatory cytokines such as TNFα and IL-12p70. While some TLR7/8 agonists are effective vaccine adjuvants *in vivo,* other vaccine studies have shown a lack of adjuvant activity with some small-molecule TLR7/8 agonists. Additionally, conventional vaccination routes (subcutaneous, intramuscular, and intranasal), and oral and topical preparations of small-molecule TLR7/8 agonists can lead to serious side effects due to systemic cytokine distribution, and some clinical trials have been suspended over safety concerns. Consequently, the development of safer and more effective TLR7/8 agonists as vaccine adjuvants is needed.
Preferably, TLR7/8 agonists comprise 1*H*-imidazo[4,5-c]quinoline compounds, 8-oxoadenine compounds and **isolated bacterial RNA.** Preferably, 1*H*-imidazo[4,5-c]quinoline compounds are resiquimod, and imiquimod, and 8-oxoadenine compounds are SM360320 and GSK2245035.

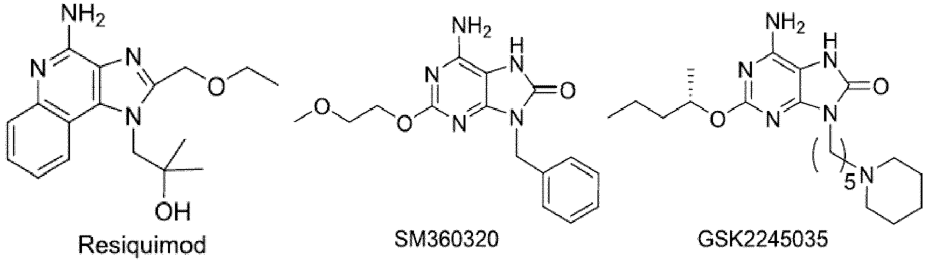

Isolated or purified **bacterial RNA** can be used as vaccine adjuvant to improve dead vaccine efficiency and adjuvant specificity.

The adjuvant of the present invention comprises an effective amount of bacterial RNA, i.e., an amount of bacterial RNA sufficient to stimulate the anti-target immune response to a desired level, such that protective immunity is generated in in a human or an animal host.

As used herein, **"bacterial RNA"** refers to RNA isolated from bacteria, and to RNA having structural features characteristics of RNA obtained from bacteria. In the latter case, the RNA may or may not have significant sequence homology to naturally occurring bacterial RNA, despite having the same structural features. The immune-enhancing property of the bacterial RNA is not sequence-specific. That is, a bacterial RNA would have immune-enhancing effects, regardless of its particular nucleotide composition, as long as the requisite structural features are present.

The term **"isolated"** as used herein with respect to nucleic acids, such as DNA or RNA, refers to molecules separated from other DNAs or RNAs, respectively that are present in the natural source of the macromolecule as well as polypeptides. The term **"isolated nucleic acid"** is meant to include nucleic acid fragments which are not naturally occurring as fragments and would not be found in the natural state.

In some embodiments, the bacterial RNA has a length ranging, for example, from about 50 nucleotides to about 2000 nucleotides, e.g., from about 800 nucleotides to about 2000 nucleotides. In some embodiments, the RNA has a length ranging from about 2000 nucleotides to more than 8000 nucleotides

In some embodiments, the bacterial RNA is modified to increase stability. Exemplary stabilized RNA molecules include, inter alia, RNA molecules having protected 3' and/or 5' ends (as disclosed in U.S. Patent Application No. 2008/0171712).

The bacterial RNA useful in this invention can be isolated from, e.g., bacterial cells by well known methods. For example, bacteria cells are homogenized or disrupted using a combination of denaturants and RNase inhibitors, including, e.g., TRIzol® (Life Technologies). This step is followed by the addition of chloroform to the lysate, and the mixture is separated into three phases by centrifugation. The RNA is then precipitated from the aqueous phase with isopropanol. Alternatively, whole RNA can be extracted using silica-gel column based approaches. Messenger RNA can be purified by negative selection, for example, by removal of rRNA using immobilized rRNA-specific probes (e.g., immobilized on magnetic bead), followed by chromatography to exclude non-rRNA species of smaller size (e.g., tRNA). RNA can be stabilized by various commercially available stabilizers, e.g., RNAlater® (Life Technologies), an aqueous storage reagent that stabilizes and protects RNA, and various widely available RNase inhibitors.

Cellular sources for the bacterial RNA can be, for example, *Streptococcus pneumoniae* or other gram-positive bacteria, *E. coli, Salmonella, Shigella, Pseudomonas, Acinetobacter,* or other gram-negative bacteria. In some embodiments, the bacterial RNA in the adjuvant is total RNA extracted from one or more bacterial sources (e.g., more than one type of bacteria). In some embodiments, the bacterial RNA in the adjuvant is RNA isolated from its bacterial source(s). In other embodiments, the bacterial RNA in the adjuvant is transcribed or synthesized *in vitro.* RNA used in the adjuvant can be a mix of multiple RNA species, each with a different nucleotide sequence, or contains a single species of RNA molecules, each having or originating from the same nucleotide sequence.

The adjuvant may also contain agents that help stabilize the immunogen and/or the RNA component, e.g., RNAse inhibitors.

In particular, RNA does not involve the risk of being stably integrated into the genome of the transfected cell. In addition, RNA is much easier to degrade *in vivo.* Probably due to the relatively short half-life of RNA in the bloodstream compared to DNA, no anti-RNA antibodies have been detected so far.

The term **"isolated"** is also used herein to refer to polynucleotides, polypeptides and proteins that are isolated from other cellular proteins and is meant to encompass both purified and recombinant polypeptides. In other embodiments, the term **"isolated"** means separated from constituents, cellular and otherwise, in which the cell, tissue, polynucleotide, peptide, polypeptide, protein, antibody or fragment(s) thereof, which are normally associated in nature.

TLR9 agonists comprise cytosine-phosphate-guanosine(CpG) oligodeoxynucleotides(ODNs) such as CPG7909.

Preferably, the at least one adjuvant is selected from agonists of toll-like receptors (TLRs) 1, 2, 4, 6, 7, 8, and 9. More preferably, the at least one adjuvant is selected from agonists of toll-like receptors (TLRs) 1, 2, 4, 6, 7, 8, and 9.

More preferably, the at least one adjuvant is selected from monophosphoryl lipid A (MPLA), CRX-524, 1*H*-imidazo[4,5-c]quinoline compounds, 8-oxoadenine compounds, a mixture of monophosphoryl lipid A and one of the 1*H*-imidazo[4,5-c]quinoline compounds, isolated bacterial RNA and cytosine-phosphate-guanosine(CpG) oligodeoxynucleotides.

Still more preferably, the at least one adjuvant is selected from monophosphoryl lipid A (MPLA), CRX-524, resiquimod, imiquimod, SM360320, GSK2245035, a mixture of monophosphoryl lipid A and resiquimod, isolated bacterial RNA and CPG7909.

Most preferably, the at least one adjuvant is selected from monophosphoryl lipid A, resiquimod, a mixture of monophosphoryl lipid A and resiquimod, and isolated bacterial RNA.

Further, in the vaccine of the present invention, a ratio of the at least one glycoconjugate and the at least one adjuvant is comprised between 1 : 1 and 1 : 100 (w/w).

Thus, the present invention preferably refers to a vaccine comprising
a) at least one glycoconjugate of a synthetic *Streptococcus pneumoniae* oligosaccharide and a carrier protein,
b) at least one adjuvant, wherein the at least one adjuvant is selected from monophosphoryl lipid A (MPLA), CRX-524, 1*H*-imidazo[4,5-c]quinoline compounds, 8-oxoadenine compounds, a mixture of monophosphoryl lipid A and one 1*H*-imidazo[4,5-c]quinoline compound, isolated bacterial RNA and cytosine-phosphate-guanosine(CpG) oligodeoxynucleotides;
   wherein the at least one glycoconjugate and the at least one adjuvant are encapsulated in biocompatible and biodegradable polymer particles.

The present invention preferably refers to a vaccine comprising
a) at least one glycoconjugate of a synthetic *Streptococcus pneumoniae* oligosaccharide and a carrier protein,
b) at least one adjuvant selected from monophosphoryl lipid A (MPLA), CRX-524, resiquimod, imiquimod, SM360320, GSK2245035, a mixture of monophosphoryl lipid A and resiquimod, isolated bacterial RNA and CPG7909;
   wherein the at least one glycoconjugate and the at least one adjuvant are encapsulated in biocompatible and biodegradable polymer particles.

Also preferably, the present invention refers to a vaccine comprising
a) at least one glycoconjugate of a synthetic *Streptococcus pneumoniae* oligosaccharide and a carrier protein,
b) at least one adjuvant, wherein the at least one adjuvant is selected from monophosphoryl lipid A (MPLA), resiquimod, a mixture of monophosphoryl lipid A and resiquimod, isolated bacterial RNA;
   wherein the at least one glycoconjugate and the at least one adjuvant are encapsulated in biocompatible and biodegradable polymer particles.

More preferably, the present invention refers to a vaccine comprising
a) at least one glycoconjugate of a synthetic *Streptococcus pneumoniae* oligosaccharide and a carrier protein,
   wherein the carrier protein is selected from diphtheria toxoid, detoxified pneumolysin, an immunogenic fragment of detoxified pneumolysin, pneumococcal surface protein A (PspA), an immunogenic fragment of pneumococcal surface protein A, and tetanus toxoid (TT);
b) at least one adjuvant, wherein the at least one adjuvant is selected from monophosphoryl lipid A (MPLA), CRX-524, resiquimod, imiquimod, SM360320, GSK2245035, a mixture of monophosphoryl lipid A and resiquimod, isolated bacterial RNA, and CPG7909,
   wherein the at least one glycoconjugate and the at least one adjuvant are encapsulated in biocompatible and biodegradable polymer particles.

Still more preferably, the present invention refers to a vaccine comprising
a) at least one glycoconjugate of a synthetic *Streptococcus pneumoniae* oligosaccharide and a carrier protein,
   wherein the carrier protein is selected from CRM₁₉₇, detoxified pneumolysin, or an immunogenic fragment of detoxified pneumolysin, pneumococcal surface protein A (PspA), or an immunogenic fragment of pneumococcal surface protein A, and tetanus toxoid (TT);
b) at least one adjuvant, wherein the at least one adjuvant is selected from monophosphoryl lipid A (MPLA), CRX-524, resiquimod, imiquimod, SM360320, GSK2245035, a mixture of monophosphoryl lipid A and resiquimod, isolated bacterial RNA, and CPG7909,
   wherein the at least one glycoconjugate and the at least one adjuvant are encapsulated in biocompatible and biodegradable polymer particles.

In one embodiment, the present invention is directed to a vaccine comprising
a) at least one glycoconjugate of a synthetic *Streptococcus pneumoniae* oligosaccharide and a carrier protein,
   wherein the carrier protein is selected from CRM₁₉₇, detoxified pneumolysin, or an immunogenic fragment of detoxified pneumolysin, pneumococcal surface protein A (PspA), or an immunogenic fragment of pneumococcal surface protein A, and tetanus toxoid (TT);
b) at least one adjuvant, wherein the at least one adjuvant is selected from monophosphoryl lipid A (MPLA), resiquimod, a mixture of monophosphoryl lipid A and resiquimod, isolated bacterial RNA;
   wherein the at least one glycoconjugate and the at least one adjuvant are encapsulated in biocompatible and biodegradable polymer particles.

In one preferred embodiment, the present invention is directed to a vaccine comprising
a) at least one glycoconjugate of a synthetic *Streptococcus pneumoniae* oligosaccharide and a carrier protein, the carrier protein is CRM₁₉₇;
b) at least one adjuvant, wherein the at least one adjuvant is selected from monophosphoryl lipid A (MPLA), resiquimod, a mixture of monophosphoryl lipid A (MPLA) and resiquimod, isolated bacterial RNA;
   wherein the at least one glycoconjugate and the at least one adjuvant are encapsulated in biocompatible and biodegradable polymer particles.

The biocompatible and biodegradable polymer particles are used as vaccine delivery systems. Appropriate vaccine delivery is a key element to ensure successful vaccination.

By the term **"biocompatible",** the person skilled in the art will understand that the polymer particles, when administered to a subject, will not produce any substantial adverse effect. By the term **"biodegradable",** the person skilled in the art will understand that the polymer particles, when administered to a subject, are broken down (i.e. degraded) by hydrolysis and/or enzymatic processes within the body.

It was found that poly(lactic-co-glycolic acid) (PLGA) particles are useful as vaccine delivery system for the inventive vaccine formulation comprising the glycoconjugates of a synthetic *Streptococcus pneumoniae* oligosaccharide and a carrier protein and enhanced immunogenicity and stability of the vaccine of the present invention.

Moreover, the polymer particles for the invention disclosed herein may be preferably selected from polyglycolic acid (PGA), the polylactic acid (PLA), poly(lactic-co-glycolic acid) (PLGA), polycarprolactone (PCL), chitosan, chitin, gelatin, polycyanoacrylate (PCA) and poly-alkyl-cyanoacrylate (PACA) polymers. However, most preferably, the polymeric particles comprise a polylactic acid (PLA) or PLGA polymer.

Therefore, in the present invention, the biocompatible and biodegradable polymer particles are selected from polylactic acid (PLA) particles, polyglycolic acid (PGA) particles, poly(lactic-co-glycolic acid) (PLGA) particles, PLGA/PEG co-polymer (PLGA-PEG) particles and tri-block PLGA/PEG/PLGA copolymer particles as vaccine delivery system. Poly(lactic-co-glycolic acid) (PLGA) particles are particularly useful as vaccine delivery system for the inventive vaccine formulation.

Biodegradable poly(lactic-co-glycolic acid) (PLGA) is degraded into lactic and glycolic acids by hydrolysis and possibly some enzymatic action. In turn, the lactic acids enter the tricarboxylic acid cycle and are metabolized and eventually eliminated from the body as carbon dioxide and water, while glycolic acid is believed to be excreted either unchanged in the kidney or, like the lactic acids, via the tricarboxylic acid cycle where it is metabolized and eventually eliminated from the body as carbon dioxide and water.
Some particular examples include PLGA polymers with a polylactic acid (PLA) / poly glycolic acid (PGA) ratio of 50 : 50, 65 : 35, 75 : 25 and 85:15. With regard to PLGA polymers, typically, the higher the content of polyglycolic acid in the PLGA, the faster is the rate of degradation (eg PLGA 50:50 degrades faster than 65:35, which in turn degrades faster than 75:25.), as the hydrophilicity of the PLGA is increased with increasing PGA content (thereby resulting in quicker degradation by hydrolysis).
On the other hand, the use of a biodegradable polymer with higher molecular weight will generally exhibit a lower rate of degradation. The polymers used in the present invention, and particularly where a PLGA polymer is used, may have a molecular weight (Mw) in the range of, for example, 5-100 kDa, more preferably 25-75 kDa, and most preferably, 30 - 60 kDa. The ability to select PLG polymers with different Mw and/or PLA/PGA ratios enables "tuning" of the release profile of the active substance from the composition of the present invention.
More preferably, the polymeric particles of the vaccine of the present invention comprise a PLGA 50 : 50 polymer with a Mw of 30 - 60 kDa. More preferably, the polymeric particles of the vaccine of the present invention comprise a PLGA 50:50 polymer with a Mw of 24 - 38 kDa. In other words, the polymeric particles of the vaccine of the present invention comprise PLGA particles with a polylactic acid (PLA) / poly glycolic acid (PGA) ratio of 50 : 50 and a Mw of 30 - 60 kDa, and more preferably a Mw of 24 - 38 kDa.

In addition, PLGA copolymers may also be suitable. For example, di-block poly(lactic-co-glycolic acid) / polyethylene glycol (PLGA/PEG) co-polymers and tri-block poly(lactic-co-glycolic acid) / polyethylene glycol / poly(lactic-co-glycolic acid) (PLGA/PEG/PLGA) copolymers may be suitable and may provide an added benefit of increased shelf stability.

Poly(lactic-co-glycolic acid) (PLGA) are copolymers of hydroxy acid monomers, D-lactic, L-lactic, and glycolic acid can entrap antigens for their efficient delivery to cells. PLGA have been used for encapsulation of antigens due to their homogenous distribution on the matrix. They form biodegradable and biocompatible platforms and have been widely approved by the US Food and Drug Administration (FDA) and European Medicines Agency (EMEA) for humans and veterinary use. The slow release of antigen and adjuvant molecules for several weeks to months prolongs and enhances antibody response to the antigen and overall stimulation of the immune system by cytokine production. For the formulation of particles, mostly amorphous d,I-PLGA is used, which differ in L-Iactic: glycolic acid monomer ratio, molecular mass, and end-group chemistry. These parameters determine the hydrophobicity and degradation kinetics of the materials, equivalent to the microencapsulation efficiency and release rate of drugs and antigen. The hydrophobicity has a high impact on the degradation of the polymer by hydrolysis.

Biodegradable particles such as PLGA and PLA particles promote immunogenicity through (1) stabilization and protection of the antigen from chemical or enzymatic degradation; (2) controlled antigen release which increases antigen exposure to the professional APCs and prolong antigen presentation; (3) facilitating the antigen uptake by dendritic cells by mimicking the size and shape of the pathogen; (4) targeted delivery through additional costimulatory molecules; (5) parallel delivery of multiple components and (6) regulation of the type of immune response (by particles size, charge and costimulatory molecules). In general, the use of particles in the vaccine formulations improved not only the antigen delivery (antigen depot and targeting APCs) but might also act as an adjuvant.

In the present invention, the dimension of particles loaded with antigen and adjuvant was analyzed by dynamic light scattering (DLS) measurement and scanning electron microscopy (SEM). The size distribution by intensity and number shows the average magnitude of the ST3-tetrasaccharide CRM₁₉₇ and adjuvant encapsulated particles between 300 - 500 nm depending on the formulation.

Some studies have shown that particles less than 450 - 600 nm induced the most robust immune response after s.c. injection, i.e. activation of CD8⁺ cells. In regards to IgG production, the 200 nm and 500 nm particles elicited similar antibody titer. Microparticles of 0.5 - 5 µm are embraced via phagocytosis or macropinocytosis, and mostly induce humoral responses.

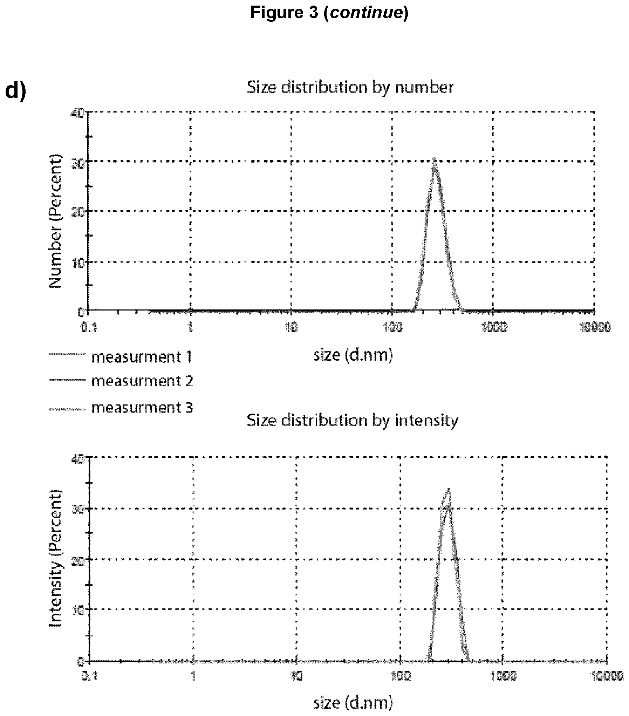

Therefore, for effective vaccine delivery, the biocompatible and biodegradable polymer particles have an average diameter in a range of 100 nm to 10000 nm, preferably of 100 nm to 5000 nm, more preferably of 100 nm to 1000 nm, most preferably in a range of of 150 to 500 nm **(****Figure 3d****).**

Thus, the present invention is directed to a vaccine comprising
a) at least one glycoconjugate of a synthetic *Streptococcus pneumoniae* oligosaccharide and a carrier protein;
b) at least one adjuvant, wherein the at least one adjuvant is selected from agonists of toll-like receptors 1, 2, 4, 6, 7, 8, or 9, wherein the at least one glycoconjugate and the at least one adjuvant are encapsulated in biocompatible and biodegradable polymer particles, and
   the biocompatible and biodegradable polymer particles are selected from polylactic acid (PLA) particles, polyglycolic acid (PGA) particles, poly(lactic-co-glycolic acid) particles, PLGA/PEG co-polymers (PLGA-PEG) particles and tri-block PLGA/PEG/PLGA copolymers particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

Thus, the present invention preferably refers to a vaccine comprising
a) at least one glycoconjugate of a synthetic *Streptococcus pneumoniae* oligosaccharide and a carrier protein,
b) at least one adjuvant, wherein the at least one adjuvant is selected from monophosphoryl lipid A (MPLA), CRX-524, 1*H*-imidazo[4,5-c]quinoline compounds, 8-oxoadenine compounds, a mixture of monophosphoryl lipid A and one of the 1*H*-imidazo[4,5-c]quinoline compounds, isolated bacterial RNA, and cytosine-phosphate-guanosine(CpG) oligodeoxynucleotides;
   wherein the at least one glycoconjugate and the at least one adjuvant are encapsulated in biocompatible and biodegradable polymer particles; and
   the biocompatible and biodegradable polymer particles are selected from polylactic acid (PLA) particles, polyglycolic acid (PGA) particles, poly(lactic-co-glycolic acid) particles, PLGA/PEG co-polymers (PLGA-PEG) particles and tri-block PLGA/PEG/PLGA copolymers particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

The present invention preferably refers to a vaccine comprising
a) at least one glycoconjugate of a synthetic *Streptococcus pneumoniae* oligosaccharide and a carrier protein,
b) at least one adjuvant selected from monophosphoryl lipid A (MPLA), CRX-524, resiquimod, imiquimod, SM360320, GSK2245035, a mixture of monophosphoryl lipid A and resiquimod, isolated bacterial RNA and CPG7909;
   wherein the at least one glycoconjugate and the at least one adjuvant are encapsulated in biocompatible and biodegradable polymer particles; and
   the biocompatible and biodegradable polymer particles are selected from polylactic acid (PLA) particles, polyglycolic acid (PGA) particles, poly(lactic-co-glycolic acid) particles, PLGA/PEG co-polymers (PLGA-PEG) particles and tri-block PLGA/PEG/PLGA copolymers particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

Also preferably, the present invention refers to a vaccine comprising
a) at least one glycoconjugate of a synthetic *Streptococcus pneumoniae* oligosaccharide and a carrier protein,
b) at least one adjuvant, wherein the at least one adjuvant is selected from monophosphoryl lipid A (MPLA), resiquimod, a mixture of monophosphoryl lipid A and resiquimod, isolated bacterial RNA;
   wherein the at least one glycoconjugate and the at least one adjuvant are encapsulated in biocompatible and biodegradable polymer particles; and
   the biocompatible and biodegradable polymer particles are selected from polylactic acid (PLA) particles, polyglycolic acid (PGA) particles, poly(lactic-co-glycolic acid) particles, PLGA/PEG co-polymers (PLGA-PEG) particles and tri-block PLGA/PEG/PLGA copolymers particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

More preferably, the present invention refers to a vaccine comprising
a) at least one glycoconjugate of a synthetic *Streptococcus pneumoniae* oligosaccharide and a carrier protein,
   wherein the carrier protein is selected from diphtheria toxoid, detoxified pneumolysin, an immunogenic fragment of detoxified pneumolysin, pneumococcal surface protein A (PspA), an immunogenic fragment of pneumococcal surface protein A, and tetanus toxoid (TT);
b) at least one adjuvant, wherein the at least one adjuvant is selected from monophosphoryl lipid A (MPLA), CRX-524, resiquimod, imiquimod, SM360320, GSK2245035, a mixture of monophosphoryl lipid A and resiquimod, isolated bacterial RNA and CPG7909,
   wherein the at least one glycoconjugate and the at least one adjuvant are encapsulated in biocompatible and biodegradable polymer particles; and
   the biocompatible and biodegradable polymer particles are selected from polylactic acid (PLA) particles, polyglycolic acid (PGA) particles, poly(lactic-co-glycolic acid) particles, PLGA/PEG co-polymers (PLGA-PEG) particles and tri-block PLGA/PEG/PLGA copolymers particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

Still more preferably, the present invention refers to a vaccine comprising
a) at least one glycoconjugate of a synthetic *Streptococcus pneumoniae* oligosaccharide and a carrier protein,
   wherein the carrier protein is selected from CRM₁₉₇, detoxified pneumolysin, or an immunogenic fragment of detoxified pneumolysin, pneumococcal surface protein A (PspA), or an immunogenic fragment of pneumococcal surface protein A, and tetanus toxoid (TT);
b) at least one adjuvant, wherein the at least one adjuvant is selected from monophosphoryl lipid A (MPLA), CRX-524, resiquimod, imiquimod, SM360320, GSK2245035, a mixture of monophosphoryl lipid A and resiquimod, isolated bacterial RNA and CPG7909,
   wherein the at least one glycoconjugate and the at least one adjuvant are encapsulated in biocompatible and biodegradable polymer particles; and
   the biocompatible and biodegradable polymer particles are selected from polylactic acid (PLA) particles, polyglycolic acid (PGA) particles, poly(lactic-co-glycolic acid) particles, PLGA/PEG co-polymers (PLGA-PEG) particles and tri-block PLGA/PEG/PLGA copolymers particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

In one embodiment, the present invention is directed to a vaccine comprising
a) at least one glycoconjugate of a synthetic *Streptococcus pneumoniae* oligosaccharide and a carrier protein,
   wherein the carrier protein is selected from CRM₁₉₇, detoxified pneumolysin, or an immunogenic fragment of detoxified pneumolysin, pneumococcal surface protein A (PspA), or an immunogenic fragment of pneumococcal surface protein A, and tetanus toxoid (TT);
b) at least one adjuvant, wherein the at least one adjuvant is selected from monophosphoryl lipid A (MPLA), resiquimod, a mixture of monophosphoryl lipid A and resiquimod, isolated bacterial RNA;
   wherein the at least one glycoconjugate and the at least one adjuvant are encapsulated in biocompatible and biodegradable polymer particles; and
   the biocompatible and biodegradable polymer particles are selected from polylactic acid (PLA) particles, polyglycolic acid (PGA) particles, poly(lactic-co-glycolic acid) particles, PLGA/PEG co-polymers (PLGA-PEG) particles and tri-block PLGA/PEG/PLGA copolymers particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

In one preferred embodiment, the present invention is directed to a vaccine comprising
a) at least one glycoconjugate of a synthetic *Streptococcus pneumoniae* oligosaccharide and a carrier protein, the carrier protein is CRM₁₉₇;
b) at least one adjuvant, wherein the at least one adjuvant is selected from monophosphoryl lipid A (MPLA), resiquimod, a mixture of monophosphoryl lipid A (MPLA) and resiquimod, isolated bacterial RNA;
   wherein the at least one glycoconjugate and the at least one adjuvant are encapsulated in biocompatible and biodegradable polymer particles; and
   the biocompatible and biodegradable polymer particles are selected from polylactic acid (PLA) particles, polyglycolic acid (PGA) particles, poly(lactic-co-glycolic acid) particles, PLGA/PEG co-polymers (PLGA-PEG) particles and tri-block PLGA/PEG/PLGA copolymers particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

In one more preferred embodiment, the present invention is directed to a vaccine comprising
a) at least one glycoconjugate of a synthetic *Streptococcus pneumoniae* oligosaccharide and a carrier protein, the carrier protein is CRM₁₉₇;
b) at least one adjuvant, wherein the at least one adjuvant is selected from monophosphoryl lipid A (MPLA), resiquimod, a mixture of monophosphoryl lipid A (MPLA) and resiquimod, isolated bacterial RNA;
   wherein the at least one glycoconjugate and the at least one adjuvant are encapsulated in biocompatible and biodegradable polymer particles; and
   the biocompatible and biodegradable polymer particles are selected from polylactic acid (PLA) particles, poly(lactic-co-glycolic acid) particles.

In one more preferred embodiment, the present invention is directed to a vaccine comprising
a) at least one glycoconjugate of a synthetic *Streptococcus pneumoniae* oligosaccharide and a carrier protein, the carrier protein is CRM₁₉₇;
b) at least one adjuvant, wherein the at least one adjuvant is selected from monophosphoryl lipid A (MPLA), resiquimod, a mixture of monophosphoryl lipid A (MPLA) and resiquimod, isolated bacterial RNA;
   wherein the at least one glycoconjugate and the at least one adjuvant are encapsulated in biocompatible and biodegradable polymer particles;
   the biocompatible and biodegradable polymer particles are **poly(lactic-co-glycolic acid) particles,** and
   optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

The vaccine of the invention comprises 10 or more, 11 or more, 12 or more, 13 or more, 14 or more, 15 or more, 16 or more, 17 or more, 18 or more, 19 or more, 20 or more synthetic saccharide conjugates from different S. *pneumoniae* serotypes. The vaccine of the invention may comprise 10, 11, 12, 13, 14, 15, 16, 17, 18, 19, 20, 21, 22, 23, 24, or 25 synthetic saccharide conjugates from different S. *pneumoniae* serotypes.

### Synthetic oligosaccharides of Streptococcus pneumoniae

In some embodiments, the synthetic *Streptococcus pneumoniae* oligosaccharide is preferably selected from consisting of serotypes 1, 2, 3, 4, 5, and 8. More preferably, the synthetic *Streptococcus pneumoniae* oligosaccharide is of *Streptococcus pneumoniae* serotype 3.

More preferably, the glycoconjugate of the synthetic *Streptococcus pneumoniae* oligosaccharide of serotypes 1 (ST1) has the following formula (**I**), the synthetic *Streptococcus pneumoniae* oligosaccharide of serotype 2 (ST2) has the following formula (**II**), the glycoconjugate of the synthetic *Streptococcus pneumoniae* oligosaccharide of serotypes 3 (ST3) has the following formula (**III**), the glycoconjugate of the synthetic *Streptococcus pneumoniae* oligosaccharide of serotypes 4 (ST4) has the following formula (**IV**), the glycoconjugate of the synthetic *Streptococcus pneumoniae* oligosaccharide of serotypes 5 (ST5) has the following formula (**V**), the glycoconjugate of the synthetic *Streptococcus pneumoniae* oligosaccharide of serotypes 8 (ST8) has the following formula (**VI**).

### Streptococcus pneumoniae serotype 1

*Streptococcus pneumoniae* serotype 1 (ST1) is one of the most prevalent S. *pneumoniae* serotypes. *Streptococcus pneumoniae* serotype 1 capsular polysaccharide is a linear polymer having as a repeating unit:
[→3)-α-2,4,6-trideoxy-4-amino-D-GalNAc-(1→4)-α-D-GalAp-(1→3)-α-D-GalAp-(1→4].

Synthetic oligosaccharide structures derived from [→3)-α-2,4,6-trideoxy-4-amino-D-GalNAc-(1→4)-α-D-GalAp-(1→3)-α-D-GalAp-(1→] trisaccharide repeating unit of *Streptococcus pneumoniae* type 1 capsular polysaccharide were already reported. However, the method developed by Bundle *(*Chem. Eur. J. 2010, 16, 3476.) provides α-methoxy saccharides, which are not suitable for conjugation to an immunogenic carrier protein.

The glycoconjugate of a synthetic oligosaccharide of *Streptococcus pneumoniae serotype 1* and a carrier protein has preferably the following formula (**I**):

**[H-(P)ₙ₃-(N)ₙ₂-(M)ₙ₁-O-L¹-S-W¹]ₘ₁-carrier protein** **(I)**

wherein
**L¹** is a linker;
**M**, **N** and **P** represent independently of each other one of the following sugar fragments: 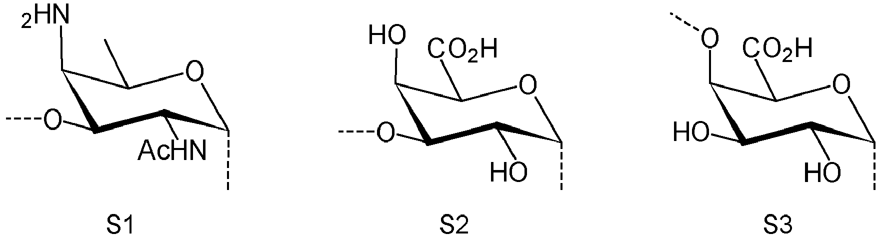 wherein the sugar fragments S1, S2, S3 are connected to each other and to -O-A-SH fragment *via* O-glycosidic bonds, each sugar fragment S1, S2, and S3 is present in the general formula (**I**) not more than once, sugar fragment S1 cannot be simultaneously connected to -O-A-S- and sugar fragment S3, sugar fragment S3 cannot be simultaneously connected to -O-A-S- and sugar fragment S2, and sugar fragment S2 cannot be simultaneously connected to -O-A-S- and sugar fragment S1, and
**W¹** represents 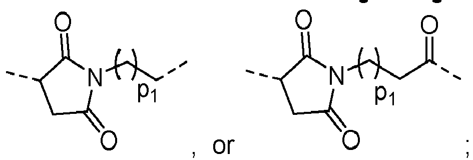
m¹ is an integer between 2 and 18;
n1, n2 and n3 are integers selected from 0 and 1, wherein at least one of the integers n1, n2 and n3 is 1;
p1 is an integer selected from 1 to 5; and
carrier protein is preferably selected from CRM₁₉₇, detoxified pneumolysin, an immunogenic fragment of detoxified pneumolysin, and pneumococcal surface protein A, and an immunogenic fragment of pneumococcal surface protein A, most preferably the carrier protein is CRM₁₉₇.

**L¹ is defined as a linker** and is part of the fragment -O-A-S-. Thus, the linker A is bound to an oxygen atom and to an SH-group, while the oxygen atom and the S-group are bound to different carbon atoms of the linker **L¹.** It is preferred that at least two carbon atoms of the linker are between the oxygen atom and the SH-group, like -O-C-C-S-.

The linker **L¹** preferably contains between 2 and 20 carbon atoms (including the carbon atoms of optional side chains), more preferably between 2 and 18, more preferably between 2 and 16, more preferably between 2 and 14, more preferably between 2 and 12, and still more preferably between 2 and 10 carbon atoms.

The shortest atom chain between the oxygen (i.e. the oxygen of **-O-L¹-**S-) and the S-group consists preferably of 2 to 14 atoms, more preferably of 2 to 12 atoms, more preferably of 2 to 10 atoms, more preferably of 2 to 8 atoms. In case the shortest chain (which is the shortest possible connection between the oxygen and the SH-group) consists of 2 to 5 atoms, these are preferably carbon atoms. In case the shortest chain consists of 4 to 8 atoms, the chain may contain 1, 2, 3, or 4 heteroatoms selected from O, N and S. In case the shortest chain consists of 9 to 14 atoms, the chain may contain 1, 2, 3, 4, 5, or 6 heteroatoms selected from O, N and S. It is preferred that the shortest chain contains 0, 1, or 2 sulphur atoms and/or 0, 1, or 2 nitrogen atoms and/or 0, 1, 2, or 3 oxygen atoms. In case more than 4 oxygen atoms are present, preferably no other heteroatoms are present.

It is also preferred that the linker **L¹,** or the shortest chain is fully or partially fluorinated. The linker **L¹** may contain a 4-membered or a 5-membered or a 6-membered saturated carbocycle or a 6-membered partly unsaturated (and not aromatic) carbocycle or a 4-membered or a 5-membered or a 6-membered saturated oxygen heterocycle or a 4-membered or a 5-membered or a 6-membered saturated nitrogen heterocycle.

The linker **L¹** may also contain amide (-NH-CO-, -CO-NH-) and/or urea (-NH-CO-NH-) residues and preferably only one amide or urea residue. The linker may also contain substituents and preferably one substituent such as R¹ or two substituents such as R¹ and R², which have the meanings as defined herein and which are preferably selected from -OCH₃, -OC₂H₅, -CH₃, -C₂H₅, -CH₂F, -CF₂H, -CF₃, -C(O)-NH₂, -NHAc, -NH(CH₃), -NH(C₂H₅), -N(CH₃)₂, -N(C₂H₅)₂, -NH-C(O)-CH₃, and -C(O)-CH₃.

In case the linker is fluorinated, more than two substituents -F are preferred.
In case an oxygen heterocycle is present each carbon atom of the oxygen heterocycle may be substituted by a hydroxy group (-OH). Thus, a 5-membered oxygen heterocycle may contain one or two hydroxy groups and a 6-membered oxygen heterocycle may contain one or two or preferably 3 hydroxy groups.

The linker **L¹,** also defined herein as -A^{a}-A^{b}-A^{c}-A^{d}- or -A^{a}-A^{b}-A^{d}- contains preferably 1, 2, 3 or 4 and more preferably 1, 2, or 3 of the following fragments: -(CH₂)ₒ₁-, -(CR¹R²)ₒ₁-, -(CH₂)ₒ₃-(CH₂-CH₂-O)ₒ₂-(CH₂)ₒ₁-, -(CH₂)ₒ₁-S-(CH₂)ₒ₄-, -(CH₂)ₒ₁-O-(CH₂)ₒ₄-, -(CH₂)ₒ₁-NH-(CH₂)ₒ₄-, -(CH₂)ₒ₁-NAc-(CH₂)ₒ₄-, -(CH₂)ₒ₁-C(O)-(CH₂)ₒ₄-, -(CH₂)ₚ₁-, -(CR⁷R⁸)ₚ₁-, -(CH₂-CH₂-O)ₚ₁-, -O-, -S-, -NH-, -C(O)-, -NH-C(O)-NH-, -NH-C(O)-(CH₂)ₚ₂-, -C(O)-NH-(CH₂)ₚ₂-, -NH-C(O)-C₂H₄-C(O)-NH-, -C(O)-NH-, -C(O)-NH-(CH₂-CH₂-O)ₚ₁-C₂H₄-, -C(O)-NH-(CH₂-CH₂-O)ₚ₁-, -(CH₂)_{q1}-, -(CR¹⁶R¹⁷)_{q1}-, -(CH₂)_{q1}-NH-C(O)-, -(CH₂)_{q1}-C(O)-NH-, 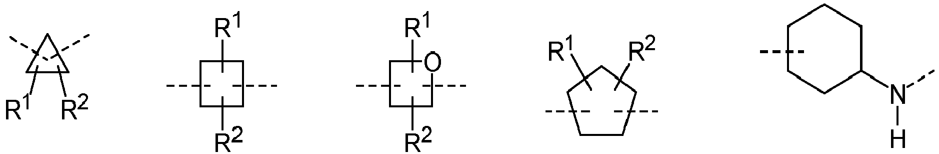 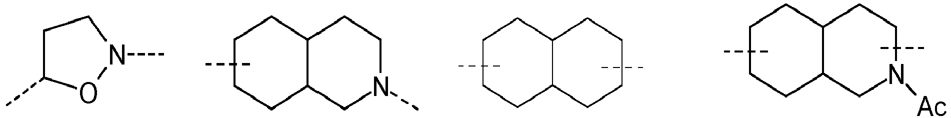 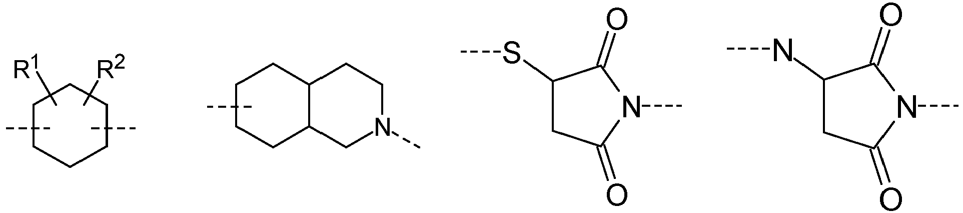 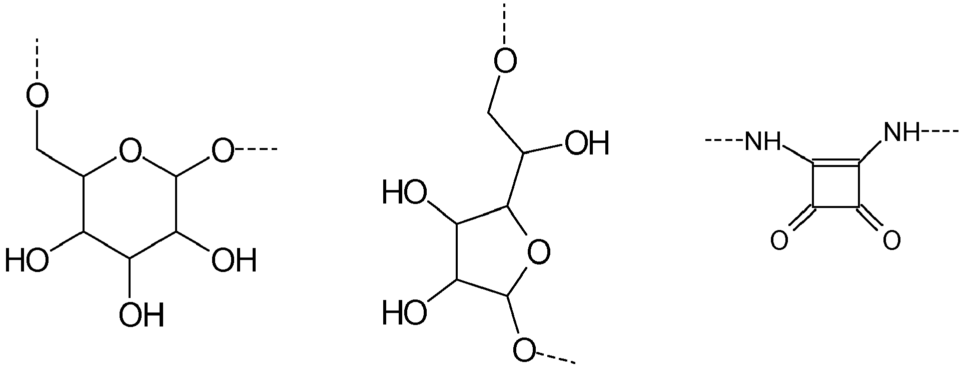 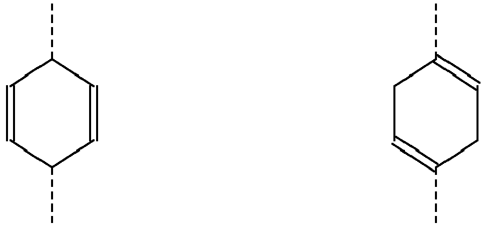
wherein not two heteroatoms of the above-mentioned residues are linked together, such as a group -S- is not linked to a group -NH-C(O)-NH-;
p2, o2, o3 are integers selected independently of each other from: 0, 1, 2, 3, 4, 5, 6, 7, 8, 9, 10;
p1, q1, o1, o4 are integers selected independently of each other from: 1, 2, 3, 4, 5, 6, 7, 8, 9, 10;
and
R¹, R², R⁷, R⁸, R¹⁶, and R¹⁷ represent independently of each other -H, -OCH₃, -OC₂H₅, -CH₃, -C₂H₅, -C₃H₇, -CH(CH₃)₂, -F, -CH₂F, -CF₂H, -CF₃, -C(O)-NH₂, -SCH₃, -SC₂H₅, -NHAc, -NH(CH₃), -NH(C₂H₅), -N(CH₃)₂, -N(C₂H₅)₂, -NH-C(O)-CH₃, and -C(O)-CH₃.

The linker -A- according to the present invention represents:
-A^{a}-A^{b}-A^{c}-A^{d}- or -A^{a}-A^{b}-A^{d}- or -A^{a}-A^{d}- or -A^{a}-;
wherein
A^{a} represents -(CH₂)ₒ₁-, -(CR¹R²)ₒ₁-, -(CR¹R²)ₒ₁-(CR³R⁴)ₒ₂-, -(CR¹R²)ₒ₁-(CR³R⁴)ₒ₂-(CR⁵R⁶)ₒ₃, -(CR¹R²)ₒ₃-(CH₂-CH₂-O)ₒ₂-(CR³R⁴)ₒ₁-, -(CH₂-CH₂-O)ₒ₂-(CR¹R²)ₒ₃-(CR³R⁴)ₒ₁-, -(CR¹R²)ₒ₁-(CR³R⁴)ₒ₂-S-(CR⁵R⁶)ₒ₄-, -(CR¹R²)ₒ₁-(CR³R⁴)ₒ₂-O-(CR⁵R⁶)ₒ₄-, -(CR¹R²)ₒ₁-(CR³R⁴)ₒ₂-NH-(CR⁵R⁶)ₒ₄-, -(CR¹R²)ₒ₁-S-(CR³R⁴)ₒ₄-, -(CR¹R²)ₒ₁-S-(CR⁵R⁶)ₒ₃-(CR³R⁴)ₒ₄--(CR¹R²)ₒ₁-O-(CR³R⁴)ₒ₄- -(CR¹R²)ₒ₁-O-(CR⁵R⁶)ₒ₃-(CR³R⁴)ₒ₄-, -(CR¹R²)ₒ₁-NH-(CR³R⁴)ₒ₄- -(CR¹R²)ₒ₁-NH-(CR⁵R⁶)ₒ₃-(CR³R⁴)ₒ₄-, -(CR¹R²)ₒ₁-C(O)-(CR³R⁴)ₒ₄- -(CR¹R²)ₒ₁-C(O)-(CR⁵R⁶)ₒ₃-(CR³R⁴)ₒ₄-, 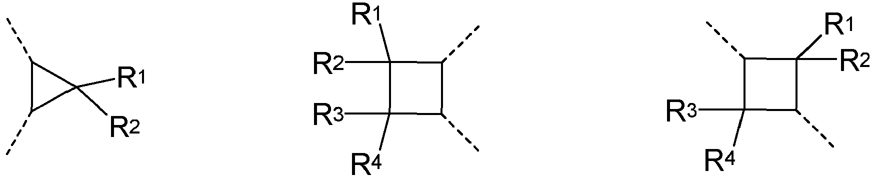 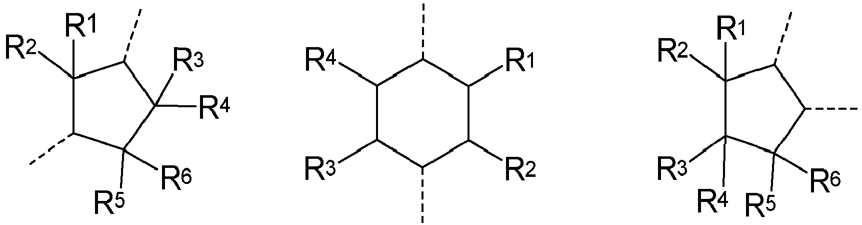 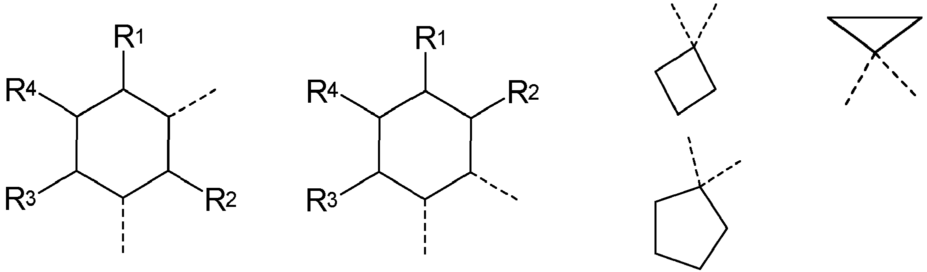 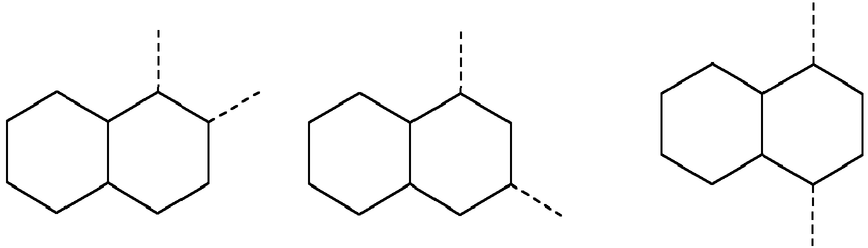 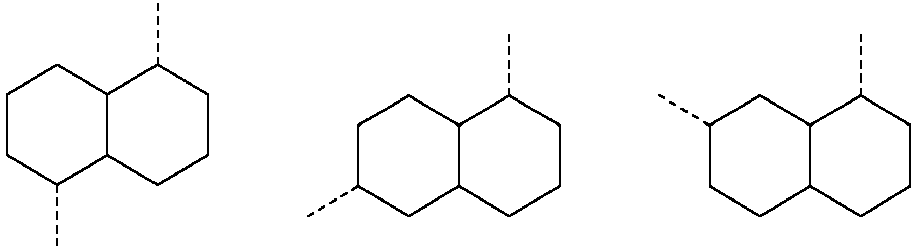 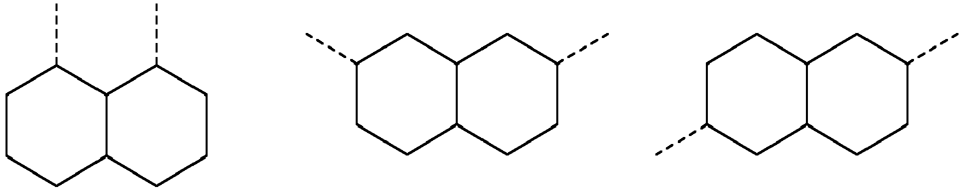 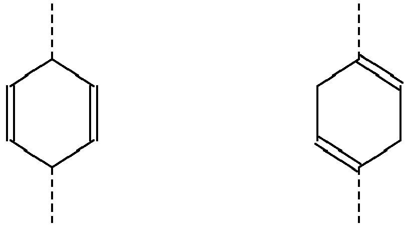
A^{b} represents -(CH₂)ₚ₁-, -(CR⁷R⁸)ₚ₁- -(CH₂-CH₂-O)ₚ₁-, -(CR⁷R⁸)ₚ₁-S-(CR⁹R¹⁰)ₚ₂-, -(CR⁷R⁸)ₚ₁-O-(CR⁹R¹⁰)ₚ₂-, -(CR⁷R⁸)ₚ₁-NH-(CR⁹R¹⁰)ₚ₂-, -O-, -S-, -NH-, -C(O)-, -NH-C(O)-NH-, -NH-C(O)-(CH₂)ₚ₂-, -C(O)-NH-(CH₂)ₚ₂-, -NH-C(O)-C₂H₄-C(O)-NH-, -(CR⁷R⁸)ₚ₁-(CR⁹R¹⁰)ₚ₂-(CH₂-CH₂-O)ₚ₃-, -(CR⁷R⁸)ₚ₁-(CH₂-CH₂-O)ₚ₂-(CR⁹R¹⁰)ₚ₃-, -(CH₂-CH₂-O)ₚ₁-(CR⁷R⁸)ₚ₂-(CR⁹R¹⁰)ₚ₃-, -C(O)-NR¹⁵-, -(CR⁷R⁸)ₚ₁-(CR⁹R¹⁰)ₚ₂-(CR¹¹R¹²)ₚ₃, -C(O)-NH-CH(R¹⁸)-, -C(O)-NH-CH(R¹⁸)-C(O)-NH-CH(R¹⁹)-, -C(O)-NH-(CH₂-CH₂-O)ₚ₁-C₂H₄-, -C(O)-NH-(CH₂-CH₂-O)ₚ₁-, 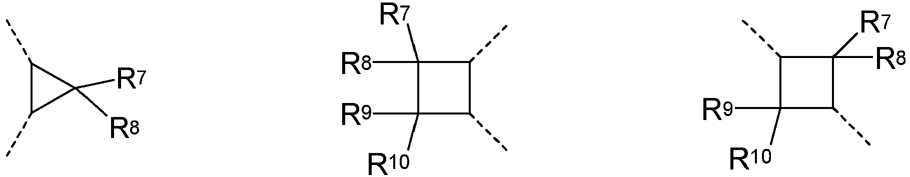 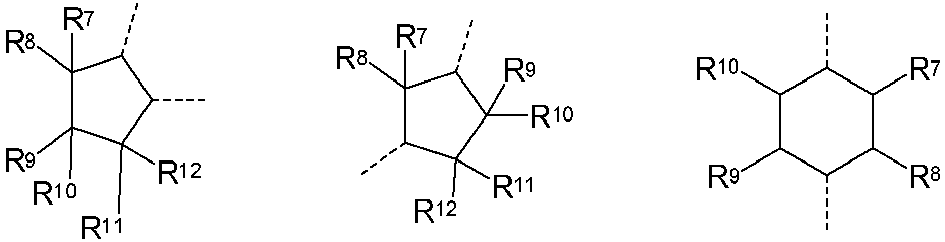 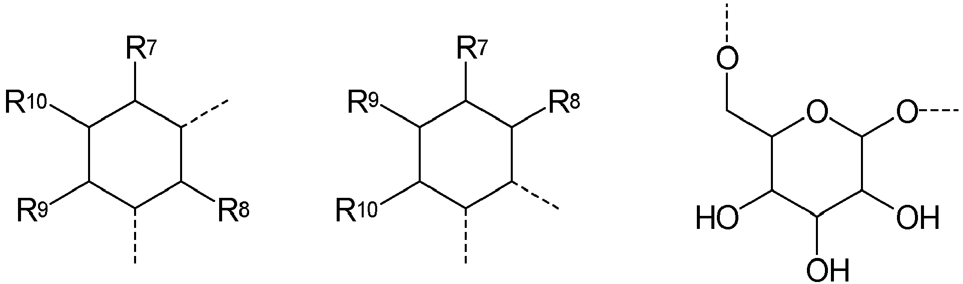 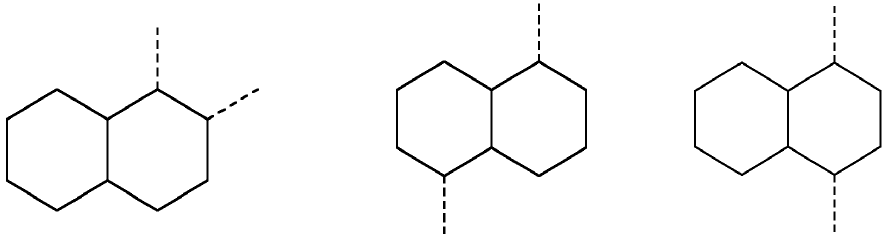 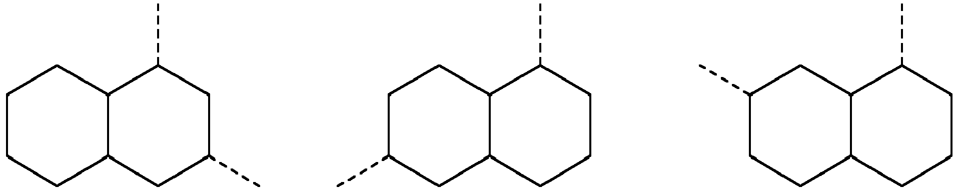 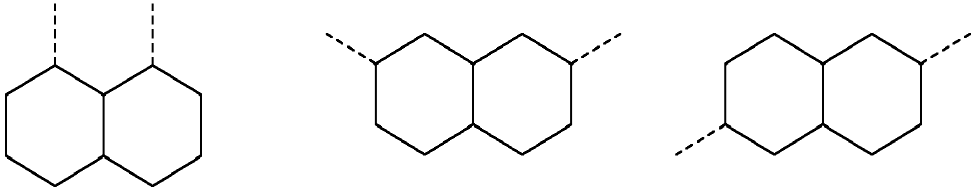 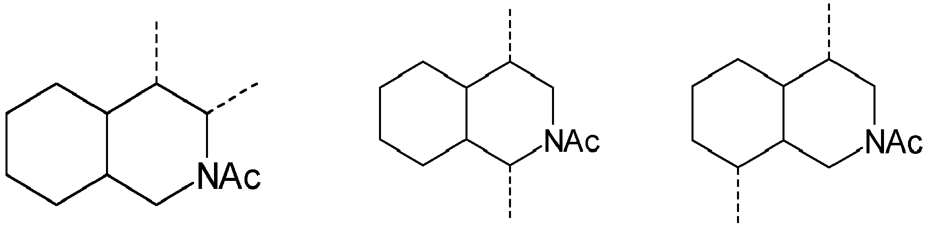 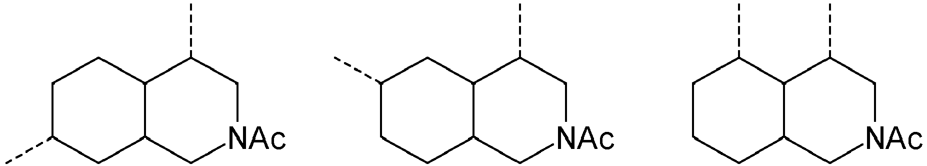 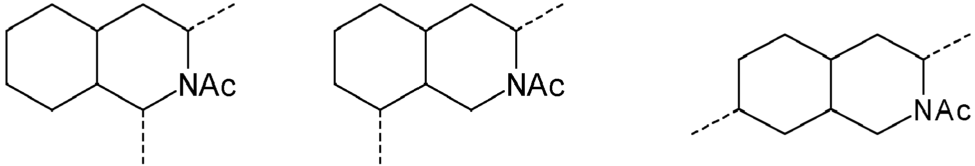 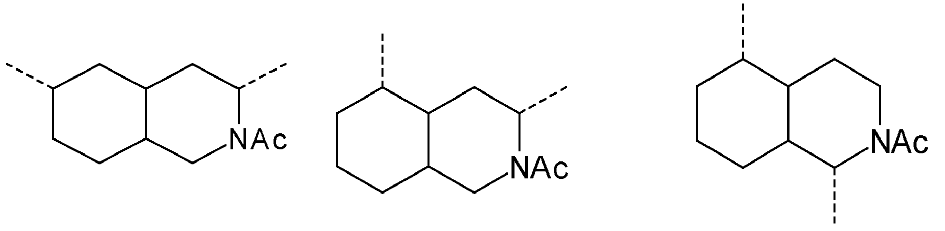 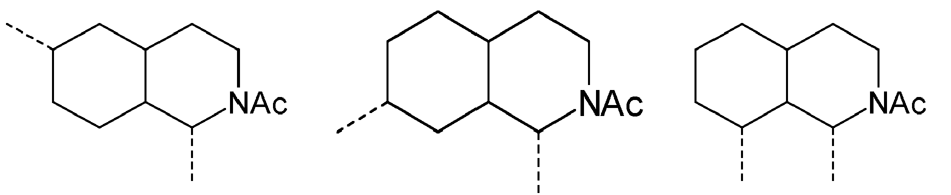 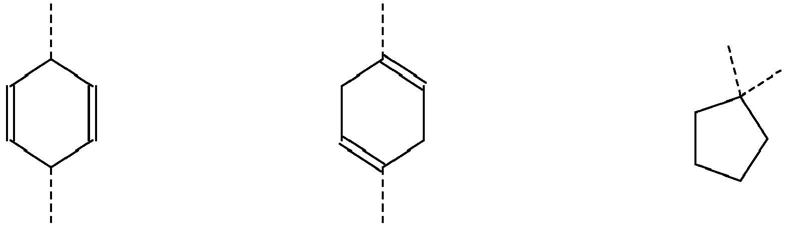 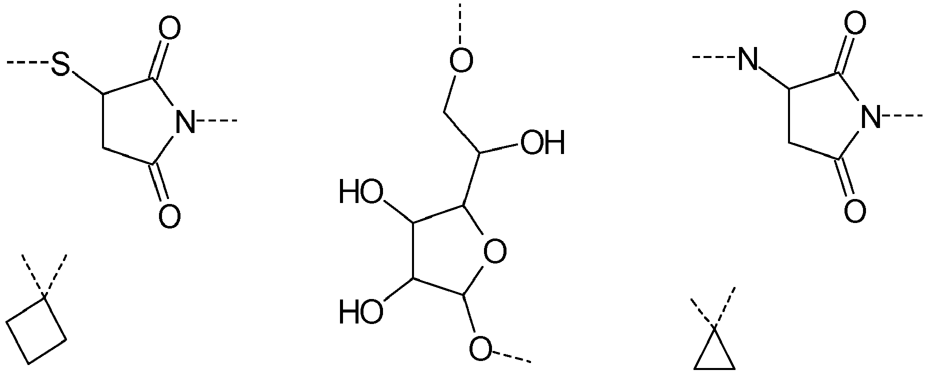 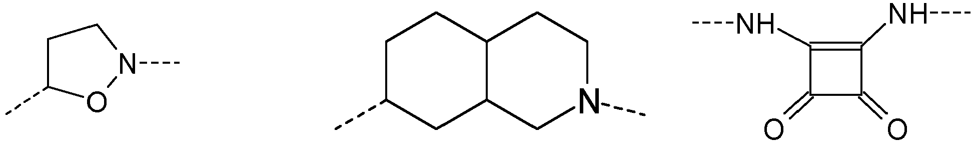 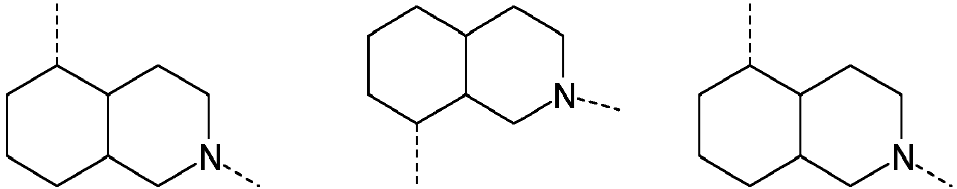 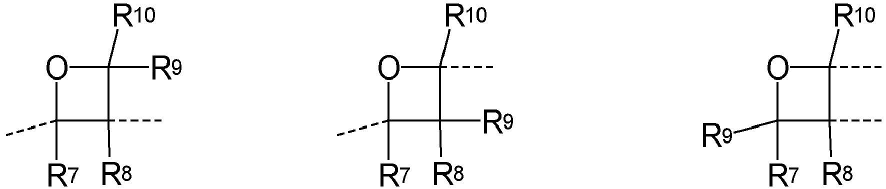
A^{c} represents -(CH₂)_{q1}-, -(CR¹⁶R¹⁷)_{q1}-, -(CH₂)_{q1}-NH-C(O)-, -(CH₂)_{q1}-C(O)-NH-, 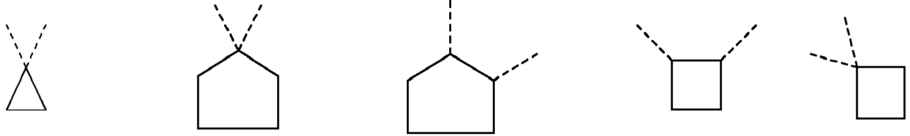 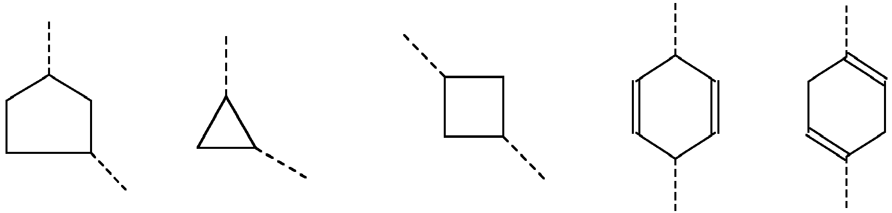 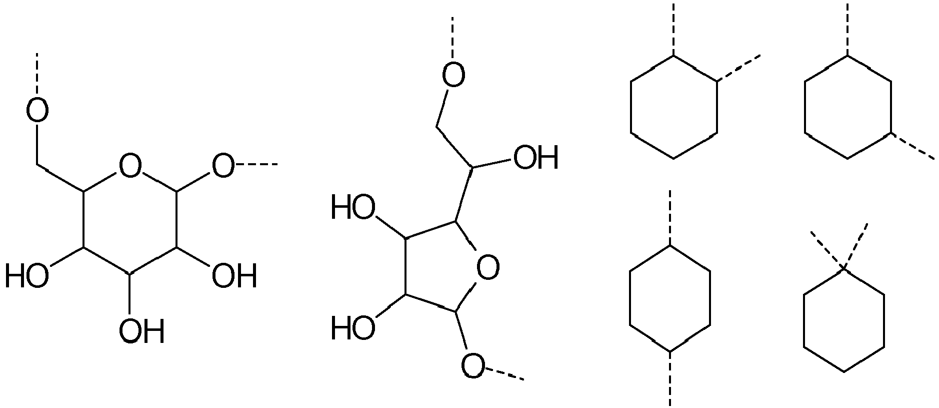
A^{d} represents -(CH₂)ₘ₁-, -(CR¹³R¹⁴)ₘ₁-, -CH₂-S-(CH₂)ₘ₁-, -CH₂-O-(CH₂)ₘ₁-, -CH₂-C(O)-(CH₂)ₘ₁-, -(CH₂-CH₂-O)ₘ₁-(CR¹³R¹⁴)ₘ₂-, -(CH₂)ₘ₁-(CR¹³R¹⁴)ₘ₂-, -(CR¹³R¹⁴)ₘ₂-(CH₂)ₘ₁- -(CR¹³R¹⁴)ₘ₁-(CH₂)ₘ₂-, -(CH₂)ₘ₂-(O-CH₂-CH₂)ₘ₁-, 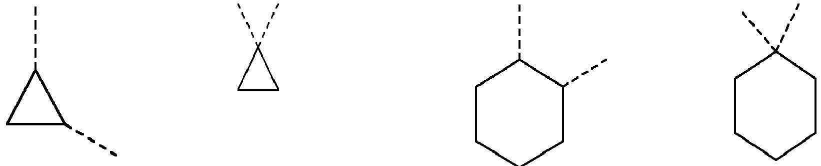 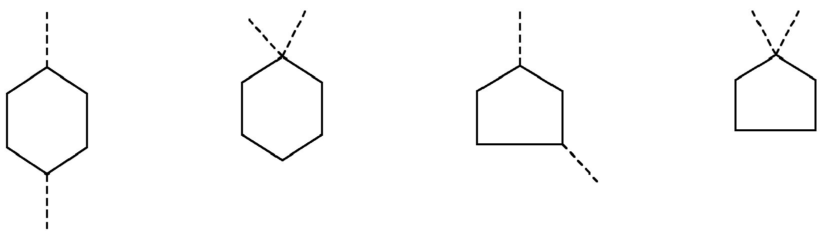 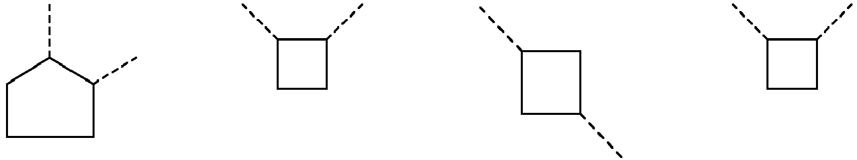 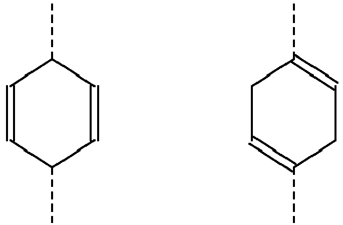
R¹-R¹⁴ , R¹⁶ and R¹⁷ represent independently of each other -H, -OCH₃, -OC₂H₅, -OC₃H₇, -cyclo-C₃H₅, -cyclo-C₄H₇, -cyclo-C₅H₉, -cyclo-C₆H₁₁, -cyclo-C₇H₁₃, -cyclo-C₈H₁₅, -CH₂-cyclo-C₆H₁₁, -C(cyclo-C₆H₁₁)₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -C₇H₁₅, -C₈H₁₇, -C₆H₄-OCH₃, -CH₂-CH₂-OCH₃, -CH₂-OCH₃, -CH₂-C₆H₄-OCH₃, -F, -CH₂F, -CF₂H, -CF₃, -C(O)-NHR¹⁵, -C(O)-NHR²², -C(O)-NHR²³, -C(O)-NHR²⁴, -C(O)-NHR²⁵, -SCH₃, -SC₂H₅, -NR¹⁵R²², -NHR¹⁵, -NHR²², -NHR²³, -NHR²⁴, -NHR²⁵ -NH-C(O)-R¹⁵ -NH-C(O)-R²², -NH-C(O)-R²³, -NH-C(O)-R²⁴, -NH-C(O)-R²⁵, -C(O)-R¹⁵, -C(O)-R²², -C(O)-R²³, -C(O)-R²⁴, -C(O)-R²⁵.
R¹⁵, R²², R²³, R²⁴ and R²⁵ represents : -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃;
R¹⁸, R¹⁹, R²⁰ and R²¹ represent independently of each other -CH₂-OCH₃, -CH₂-SCH₃, -CH₂-SeCH₃, -C₂H₄-OCH₃, -C₂H₄-SCH₃, -C₂H₄-SeCH₃, -H -CH₃, -C₂H₅, -C₃H₇, -CH(CH₃)(OCH₃), -CH(CH₃)₂, -CH₂-CH(CH₃)₂, -CH₂-Ph, -CH₂-CH(CH₃)(C₂H₅), -CH₂-C(O)-NH₂, -C₂H₄-C(O)-NH₂, -CH₂-*p*-C₆H₄-OCH₃;
p2, p3, o2, o3 are integers selected independently of each other from: 0, 1, 2, 3, 4, 5, 6, 7, 8, 9, 10;
p1, q1, o1, o4, m1, m2 are integers selected independently of each other from: 1, 2, 3, 4, 5, 6, 7, 8, 9, 10.

Preferably the linker **L¹** represents the residue -A^{a}-.

-A^{a}- is preferably a linear carbon chain or a saturated carbocycle selected from

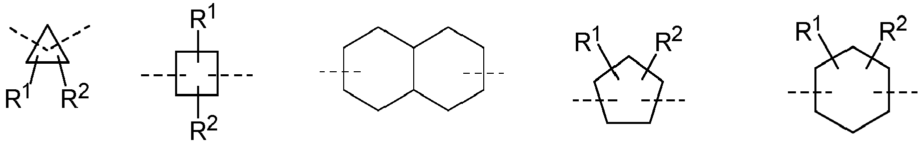

Also preferably the linker **L¹** represents -(CH₂)ₒ₁-A^{b}-A^{c}-A^{d}-, -(CH₂)ₒ₁-A^{b}-A^{d}-, -(CH₂)ₒ₁-A^{d}-, -(CR¹R²)ₒ₁-A^{b}-A^{c}-A^{d}-, -(CR¹R²)ₒ₁-A^{b}-A^{d}-, or -(CR¹R²)ₒ₁-A^{d}-, wherein A^{b}, A^{c} and A^{d} have the meanings as defined herein and R¹ and R² have the meanings as defined herein and preferably represent independently of each other -H, -OCH₃, -OC₂H₅, -CH₃, -C₂H₅, -C₃H₇, -CH(CH₃)₂, -F, -CH₂F, -CF₂H, -CF₃, -C(O)-NH₂, -SCH₃, -SC₂H₅, -NHAc, -NH(CH₃), -NH(C₂H₅), -N(CH₃)₂, -N(C₂H₅)₂, -NH-C(O)-CH₃, and -C(O)-CH₃.

A^{b} represents in general and especially in the afore-mentioned general formula -(CH₂)ₒ₁ -A^{b}-A^{c}-A^{d}-, -(CH₂)ₒ₁-A^{b}-A^{d}-, -(CR¹R²)ₒ₁-A^{b}-A^{c}-A^{d}-, -(CR¹R²)ₒ₁-A^{b}-A^{d}-preferably one of the following residues: -O-, -S-, -NH-, -C(O)-, -NH-C(O)-NH-, -NH-C(O)-(CH₂)ₚ₂-, -C(O)-NH-(CH₂)ₚ₂-, -NH-C(O)-C₂H₄-C(O)-NH-, -C(O)-NR¹⁵-, -C(O)-NH-CH(R¹⁸)-, -C(O)-NH-(CH₂-CH₂-O)ₚ₁-C₂H₄-, -C(O)-NH-(CH₂-CH₂-O)ₚ₁-, wherein the substituents R¹⁵ and R¹⁸ have the meanings as defined herein and preferably -CH₃, -C₂H₅, or -C₃H₇.

A^{c} represents in general and especially in the afore-mentioned general formula -(CH₂)ₒ₁-A^{b}-A^{c}-A^{d}- and -(CR¹R²)ₒ₁-A^{b}-A^{c}-A^{d}- preferably one of the following residues: -(CH₂)_{q1}-,

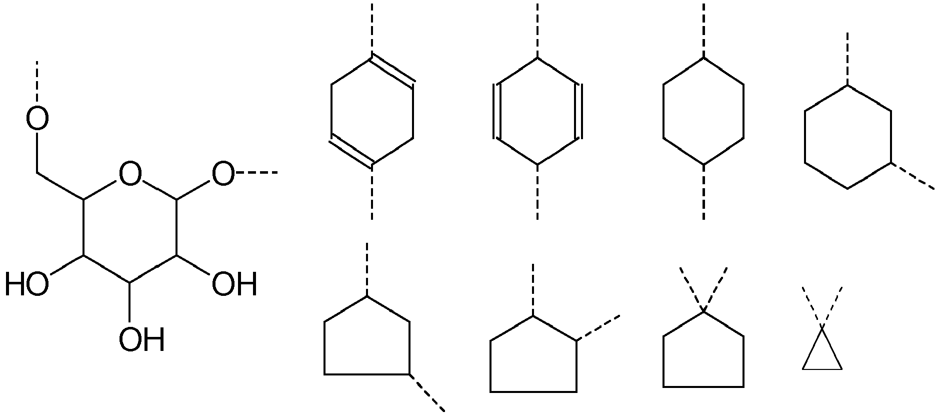

A^{d} represents in general and especially in the afore-mentioned general formula -(CH₂)ₒ₁₋A^{b}-A^{c}-A^{d}-, -(CH₂)ₒ₁-A^{b}-A^{d}-, -(CH₂)ₒ₁-A^{d}-, -(CR¹R²)ₒ₁-A^{b}-A^{c}-A^{d}-, -(CR¹R²)ₒ₁-A^{b}-A^{d}-, and -(CR¹R²)ₒ₁-A^{d}- preferably one of the following residues: -(CH₂)ₘ₁-

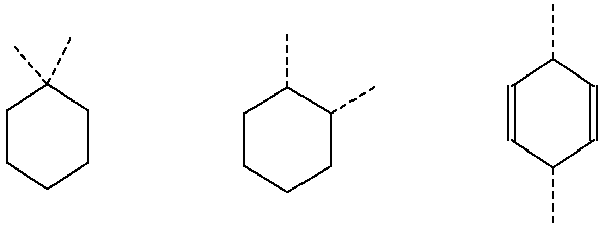

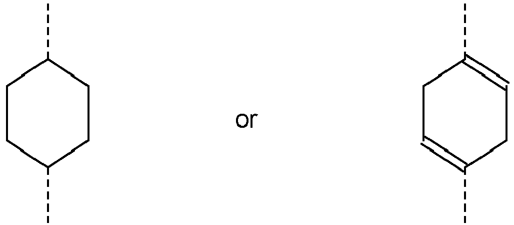

The function of the linker alone or together with the interconnecting molecule is to covalently connect the reducing-end of the saccharides to an immunogenic carrier or to a solid support. An interconnecting molecule according to the present invention refers to a bifunctional molecule containing functional group X and functional group Y, wherein functional group X is capable of reacting with the terminal thiol group on the linker **L¹** and the functional group Y is capable of binding to an immunogenic carrier or to a solid support. Thus, the present invention refers to saccharides of the formula (I), wherein the linker *per se* or together with the interconnecting molecule is capable of establishing, keeping and/or bridging a special distance between the reducing-end monosaccharide and an immunogenic carrier or a solid support.

In a preferred embodiment according to the present invention the linker -A-represents -A^{a}-A^{b}-A^{c}-A^{d}- Preferably, the fragment -A^{a}-A^{b}-A^{c}-A^{d}- is selected from the following fragments:

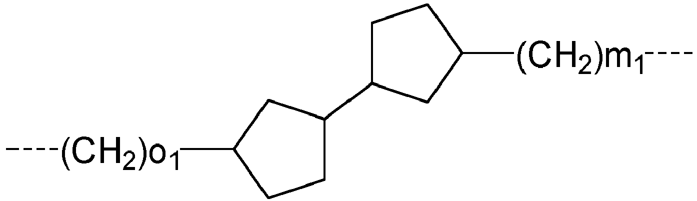

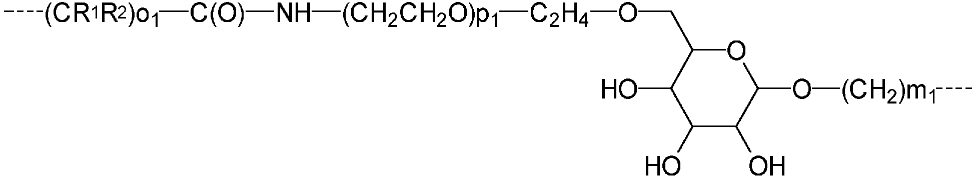

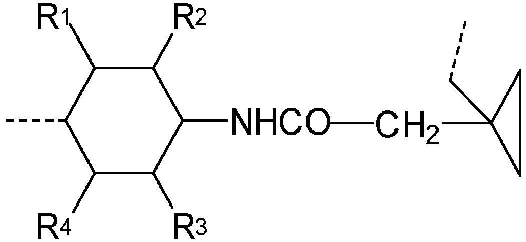

Another preferred embodiment is directed to saccharides of general formula (I), wherein the linker -A- represents -A^{a}-A^{b}-A^{d}-. Preferably, fragment -A^{a}-A^{b}-A^{d}- is selected from:
----(H₂)o₁-S-(CH₂)o₄-NH-C(O)-NH-(CH₂)m₁---*-*
----(CH₂)o₁-NH-C(O)-NH-(CH₂)m₁----
----(CH₂)o₁-CR³R⁴-O-(CH₂)o₄-C(O)-NR¹⁵-CH(C(O)NHR²²)-(CH₂)M1----

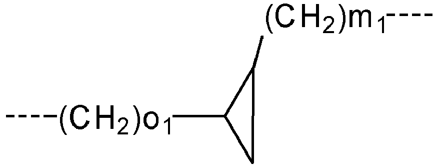

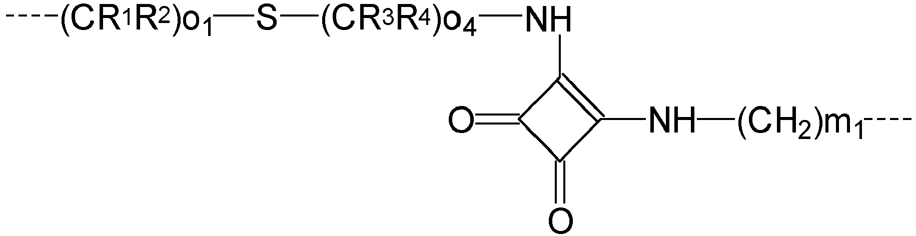

----(CH₂)o₁-(CR³R⁴)o₂-O-(CH₂)o₄-C(O)-NH-(CH₂)m₂-(OCH₂CH₂)m₁----
----(CR¹R²)o₁-(CR³R⁴)o₂-(CR⁵R⁶)o₃-S-(CH₂)m₁((CR¹³R¹⁴)m₂-----

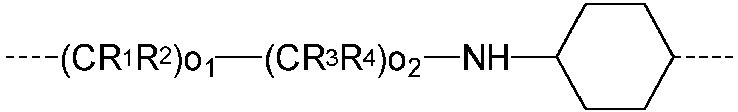

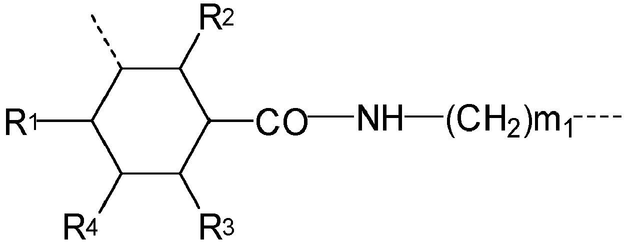

Preferably the linker **L¹** represents -A^{a}-A^{d}-, and more preferably fragment -A^{a}-A^{d}- is selected from
----(CR¹R²)o₃-(C₂H₄O)o₂-(CR³R⁴)o₁-(CH₂)m₁-(CR¹³R¹⁴)m₂----

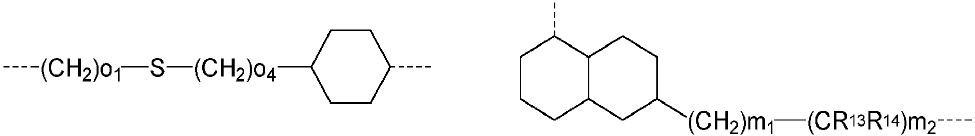

Preferably, the linker -L¹- represents -A^{a}- wherein -A^{a}- is selected from: 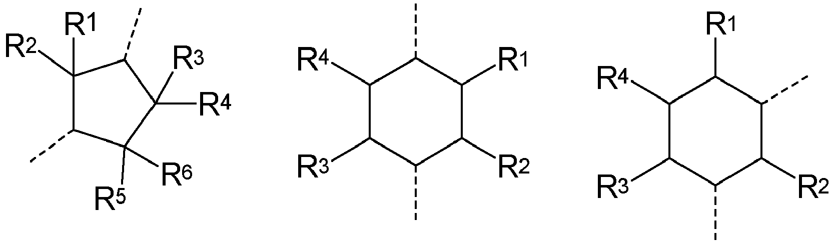 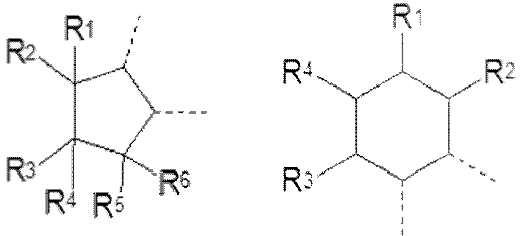 -(CH₂)ₒ₁-, -(CR¹R²)ₒ₁-, -(CR¹R²)ₒ₃-(CH₂-CH₂-O)ₒ₂-(CR³R⁴)ₒ₁-, -(CR¹R²)ₒ₁-(CR³R⁴)ₒ₂-(CR⁵R⁶)ₒ₃, -(CH₂-CH₂-O)ₒ₂-(CR¹R²)ₒ₃-(CR³R⁴)ₒ₁-, -(CH₂)ₒ₁-(CR³R⁴)ₒ₂-S-(CH₂)ₒ₄-, -(CH₂)ₒ₁-(CR³R⁴)ₒ₂-O-(CH₂)ₒ₄-, ⁻(CH₂)ₒ₁-(CR³R⁴)ₒ₂-NH-(CH₂)ₒ₄-, -(CR¹R²)ₒ₁-S-(CR³R⁴)ₒ₄, -(CR¹R²)ₒ₁-O-(CR³R⁴)ₒ₄, -(CR¹R²)ₒ₁-NH-(CR³R⁴)ₒ₄, or -(CR¹R²)ₒ₁-C(O)-(CR³R⁴)ₒ₄.

In a preferred embodiment, substituents R¹-R¹⁴, R¹⁶ and R¹⁷ are selected from:
-H, -OCH₃, -OC₂H₅, -CH₃, -C₂H₅, -C₃H₇, -F, -CH₂F, -CF₂H, -CF₃, -CH(CH₃)₂, -C₄H₉, -CH₂-CH(CH₃)₂, -CH(CH₃)-C₂H₅, -C(CH₃)₃, -CH₂-CH₂-OCH₃, -CH₂-OCH₃, -SCH₃, -SC₂H₅, -NR¹⁵R²², -NHR¹⁵, -NHR²², -NHR²³, -NHR²⁴, -NHR²⁵ -NH-C(O)-R¹⁵ -NH-C(O)-R²², -NH-C(O)-R²³, -NH-C(O)-R²⁴, -NH-C(O)-R²⁵.

Even more preferred are linkers, wherein -A- represents -A^{a}-. Preferably, fragment
-A^{a}- has the meaning: -(CH₂)ₒ₁-, -(CR¹R²)ₒ₁-, -(CH₂)ₒ₁-(CR³R⁴)ₒ₂-(CH₂)ₒ₃, -(CH₂-CH₂-O)ₒ₂-(CH₂)ₒ₁-, -(CH₂-CH₂-O)ₒ₂-(CR¹R²)-(CH₂)ₒ₁-, -(CH₂)ₒ₁-(CR³R⁴)ₒ₂-S-(CH₂)ₒ₄, -(CR¹R²)ₒ₁-S-(CH₂)ₒ₄, -(CH₂)ₒ₁-(CR³R⁴)ₒ₂-O-(CH₂)ₒ₄, -(CR¹R²)ₒ₁-O-(CH₂)ₒ₄, or -(CR¹R²)ₒ₁-C(O)-(CR³R⁴)ₒ₄, 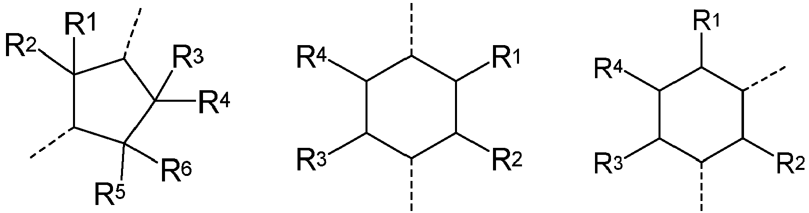 wherein
o1 and o4 are integers selected from 1, 2, 3, 4, 5, 6;
o2 and o3 are integers selected from 0, 1, 2, 3, 4, 5, 6;
and, substituents R¹-R⁶ are selected from:
   -H, -OCH₃, -OC₂H₅, -CH₃, -C₂H₅, -C₃H₇, -F, -CH₂F, -CF₂H, -CF₃, -CH(CH₃)₂, -C₄H₉, -CH₂-CH(CH₃)₂, -CH(CH₃)-C₂H₅, -C(CH₃)₃, -CH₂-CH₂-OCH₃, -CH₂-OCH₃, -SCH₃, -SC₂H₅, -NHR¹⁵ -NHR²²,-NHR²³,
   -NHR²⁴, -NHR²⁵ -NH-C(O)-R¹⁵ -NH-C(O)-R²², -NH-C(O)-R²³, -NH-C(O)-R²⁴, -NH-C(O)-R²⁵.

### Streptococcus pneumoniae serotype 2

A glycoconjugate of a synthetic oligosaccharide of *Streptococcus pneumoniae serotype 2* and a carrier protein has preferably the following general formula (**II**):

**[B*-A_{y+3}-A_{y+2}-A_{y+1}-A_{y}-O-L²-NH-W²]ₘ₂-carrier protein** **(II)**

wherein
y is an integer selected from 1, 2, 3 and 4;
A₁ = A₅ = 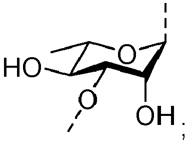
A₂ = A₆ = 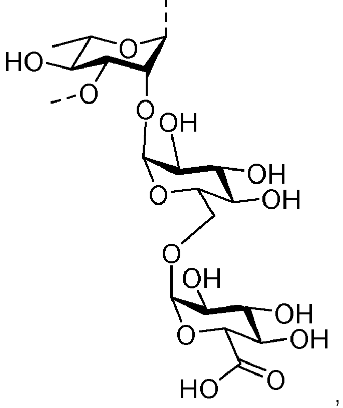
A₃ = A₇ = 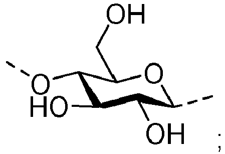 A₄ =

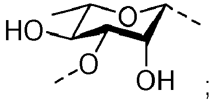

B*- represents: H-, H-A_{y}-, H-A_{y+1}-A_{y}-, H-A_{y+2}-A_{y+1}-A_{y}- or H-A_{y+3}-A_{y+2}-A_{y+1}-A_{y}-;
L² represents a linker;
m² is an integer between 2 and 18;
-W²- is selected from: 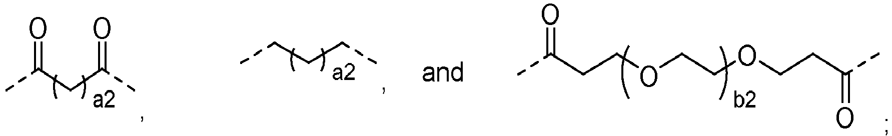
   a2 represents an integer from 1 to 10;
   b2 represents an integer from 1 to 4; and
   carrier protein is preferably selected from CRM₁₉₇, detoxified pneumolysin, an immunogenic fragment of detoxified pneumolysin, and pneumococcal surface protein A, and an immunogenic fragment of pneumococcal surface protein A, most preferably the carrier protein is CRM₁₉₇.
-L²- is defined as a linker and is part of the fragment -O-L²-NH-. Thus, the linker
-L²- is bound to an oxygen atom and to the nitrogen atom of the -NH-W²-fragment. It is preferred that at least two carbon atoms of the linker are between the oxygen atom and the -NH-W²- fragment, like -O-C-C-NH-W²-. The linker
-L²- can be an aliphatic chain, wherein the aliphatic chain can optionally include an aromatic chain inserted in it, or a number of heteroatoms oscillating from 0 to 10.

The linker -L²- preferably contains between 2 and 40 carbon atoms (including the carbon atoms of optional side chains), more preferably between 2 and 30, more preferably between 2 and 20, more preferably between 2 and 14, more preferably between 2 and 12, and still more preferably between 2 and 10 carbon atoms.

The shortest atom chain between the oxygen atom (i.e. the oxygen of -O-L²-NH-) and the -NH-W²- fragment consists preferably of 2 to 14 atoms, more preferably of 2 to 12 atoms, more preferably of 2 to 10 atoms, more preferably of 2 to 8 atoms. In case the shortest chain (which is the shortest possible connection between the oxygen at the anomeric center and the -NH-W²-fragment) consists of 2 to 6 atoms, these are preferably carbon atoms. In case the shortest chain consists of 4 to 8 atoms, the chain may contain 1, 2 or 3 heteroatoms selected from O, N and S. In case the shortest chain consists of 9 to 14 atoms, the chain may contain 1, 2, 3, 4, 5, or 6 heteroatoms selected from O, N and S.

It is also preferred that the linker -L²-, or the shortest chain is fully or partially fluorinated. The linker -L²- may contain a 3-membered or a 4-membered or a 5-membered or a 6-membered saturated carbocycle or a 5-membered partly unsaturated (and not aromatic) carbocycle or a 4-membered or a 5-membered or a 6-membered saturated oxygen heterocycle or a 4-membered or a 5-membered or a 6-membered saturated nitrogen heterocycle or a 6-membered aromatic carbocycle.

The linker -L²- may also contain amide (-NH-CO-, -CO-NH-) and/or urea (-NH-CO-NH-) residues and preferably only one amide or urea residue. The linker may also contain substituents and preferably two substituents, such as R^{10*} and R^{11*}, or four substituents such as R^{10*}, R^{11*}, R^{15*} and R^{14*}, which have the meanings as defined herein and which are preferably selected from: -F, -CI, -CH₃, -C₂H₅, -C₃H₇, -C₅H₉, -C₆H₁₃, -OCH₃, -OC₂H₅, -CH₂F, -CHF₂, -CF₃, -C(O)-NH₂, -SCH₃, -SC₂H₅, -NHC(O)CH₃, -N(CH₃)₂, and -N(C₂H₅)₂.

In case the linker -L²- is fluorinated, more than two substituents -F are preferred.

Linker -L²- is preferably selected from: -CH₂-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -CF₂-, -(CF₂)₂-, -(CF₂)₃-, -(CF₂)₄-, -(CF₂)₅-, -(CF₂)₆-, -(CF₂)₇-, -(CF₂)₈-, -(CF₂)₉-, -(CF₂)₁₀-, -(CH₂)₂-O-(CH₂)₂-, -CH₂-O-(CH₂)₃-, -(CH₂)₃-O-CH₂-,
-CH₂-O-(CH₂)₂-, -(CH₂)₂-O-CH₂-, -(CH₂)₃-O-(CH₂)₂-, -(CH₂)₂-O-(CH₂)₃--(CH₂)₄-O-CH₂-, -CH₂-O-(CH₂)₄-, -L^{a*}-, -L^{a*}-L^{e*}-, -L^{a*}-L^{b*}-L^{e*}-, _L^{a*}-L^{b*}-L^{d*}-L^{c*}-L^{e*}-, -L^{a*}-L^{d*}-L^{e*}-;
wherein
-L^{a*}- is selected from: -(CH₂)_{o*}-, -(CF₂)_{o*}-, -(CH₂-CH₂-O)_{o*}-C₂H₄-, -(CH₂-CH₂-O)_{o*-}-CH₂-, -(CR^{10*}R^{11*})_{o*}-, 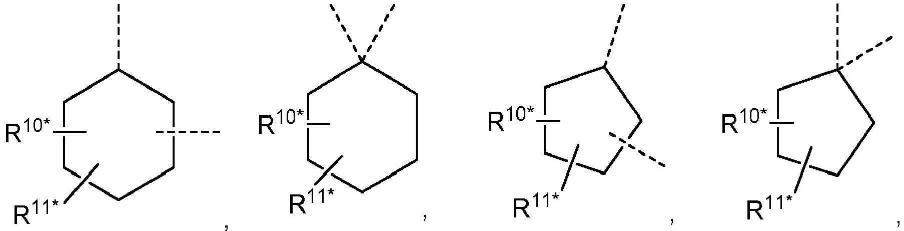 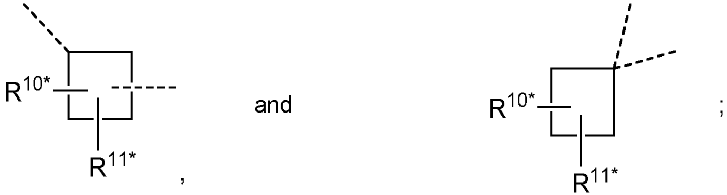 -L^{b*}- and -L^{c*}- are independently of each other selected from: -O-, -NH-C(O)-NH-, -NH-C(S)-NH- -NH-C(O)-, -C(O)-NH-, -NH-C(O)-O-, -NR^{9*}-, -NR^{18*}-, -SO₂-, 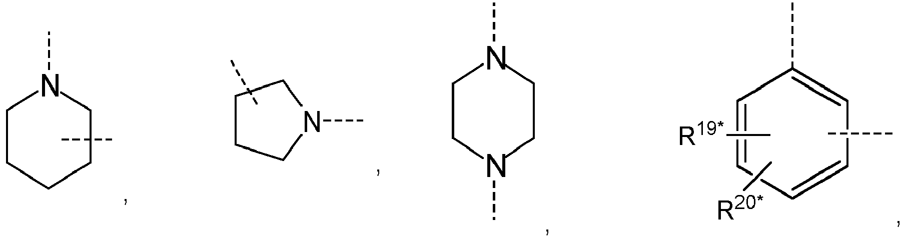 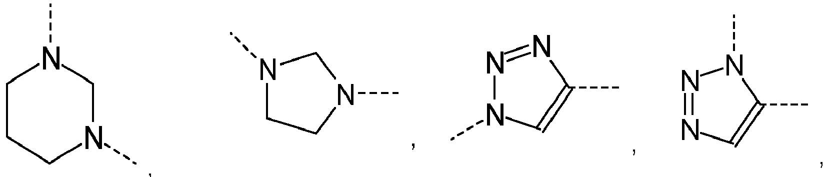 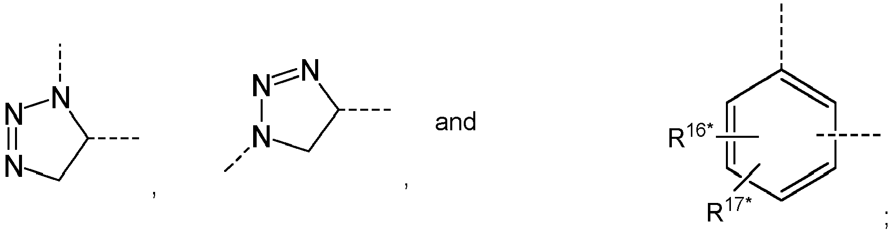 -L^{d*} represents -(CH₂)_{q*}-, -(CF₂)_{q*}-, -(CR^{12*}R^{13*})_{q*}-, -(CH₂-CH₂-O)_{q*}-C₂H₄-, -(CH₂-CH₂-O)_{q*}-CH₂-, 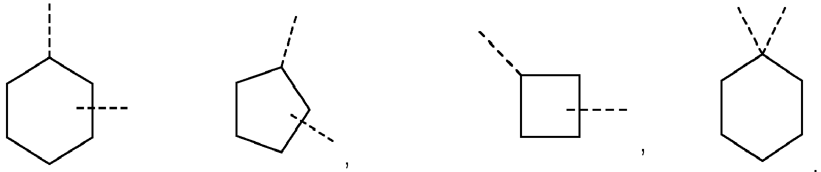 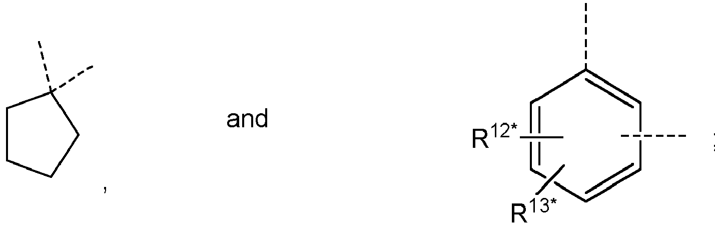 -L^{e*}- is selected from: -(CH₂)_{p1*}- -(CF₂)_{p1*}- -C₂H₄-(O-CH₂-CH₂)_{p1*}-, -CH₂-(O-CH₂-CH₂)_{p1*}-, -(CH₂)_{p1*}-O-(CH₂)_{p2*}-, -(CR^{14*}R^{15*})_{p1*}-, -(CR^{14*}R^{15*})_{p1*}-O-(CR^{21*}R^{22*})_{p2*}- 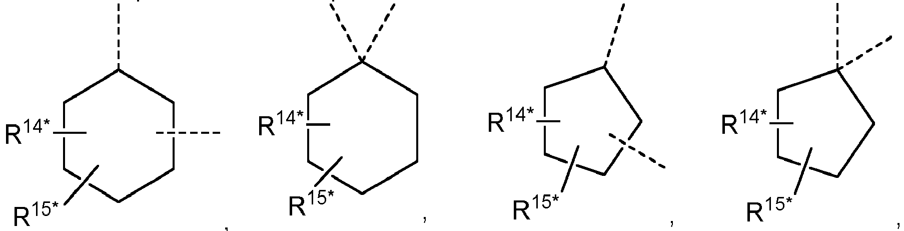 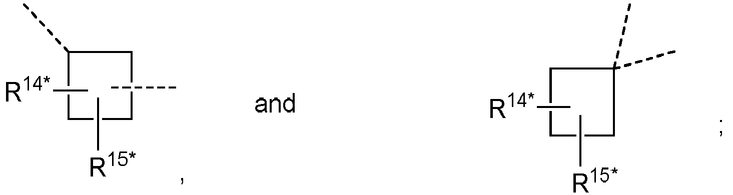 R^{9*} and R^{18*} are independently of each other selected from: -CH₃, -C₂H₅, -C₃H₇, and -C(O)CH₃;
R^{10*} , R^{11*}, R^{12*}, R^{13*}, R^{14*}, R^{15*}, R^{16*}, R^{17*}, R^{19*}, R^{20*}, R^{21*} and R^{22*} are independently of each other selected from: -H, -F, -Cl, -CH₃, -C₂H₅, -C₃H₇, -C₅H₉, -C₆H₁₃, -OCH₃, -OC₂H₅, -CH₂F, -CHF₂, -CF₃, -C(O)-NH₂, -SCH₃, -SC₂H₅, -NHC(O)CH₃, -N(CH₃)₂ and -N(C₂H₅)₂;
o*, q*, p1* and p2* are independently of each other an integer selected from 1, 2, 3, 4, 5, 6, 7, 8, 9, and 10.

More preferably, the *Streptococcus pneumoniae* serotype 2 conjugate has preferably the following general formula (**IIa**): 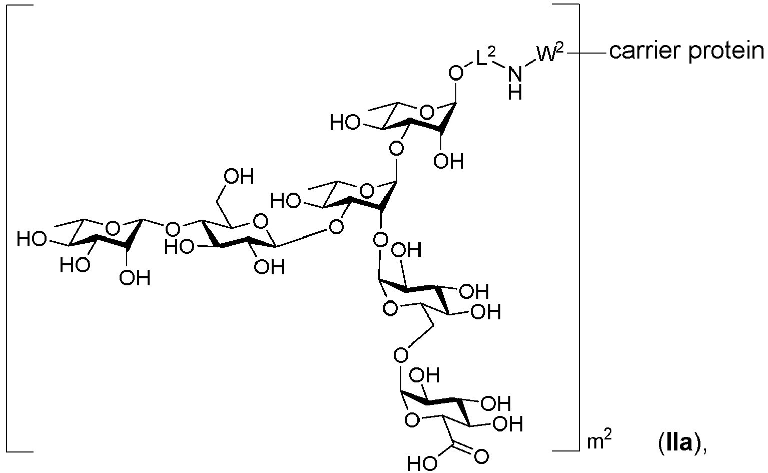
wherein **m²** represents an integer from 6 to 12;
**L²** represents -CH₂CH₂-, or -C₅H₁₀-;
**W²** represents -COCH₂CH₂CH₂CH₂CO-, or -COCH₂CH₂CH₂CH₂CH₂;
carrier protein is preferably selected from CRM₁₉₇, detoxified pneumolysin, an immunogenic fragment of detoxified pneumolysin, and pneumococcal surface protein A, and an immunogenic fragment of pneumococcal surface protein A, most preferably the carrier protein is CRM₁₉₇.

### Streptococcus pneumoniae serotype 3

A glycoconjugate of a synthetic oligosaccharide of *Streptococcus pneumoniae serotype 3* and a carrier protein has the following formula (**III**)

**[H-(C)_{c3}-(D-C)_{c2}-(D)_{c1}-O-L³-NH-W³]ₘ₃- carrier protein** **(III)**

wherein
C represents: 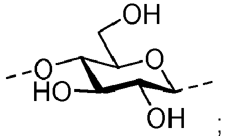
D represents: 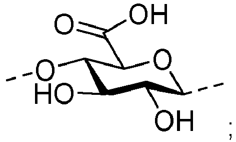
c2 is an integer selected from 1, 2, 3, 4, 5, 6, 7, 8, 9 and 10;
c1 and c3 represent independently of each other an integer 0 or 1;
L³ represents a linker;
m³ is an integer between 2 and 18;
-W³- is selected from: 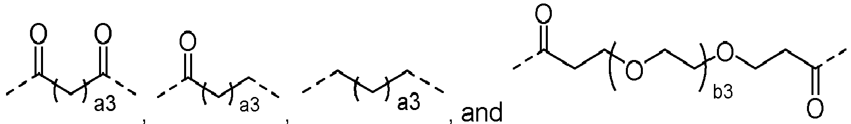
   a3 represents an integer from 1 to 10;
   b3 represents an integer from 1 to 4; and
   carrier protein is preferably selected from CRM₁₉₇, detoxified pneumolysin, an immunogenic fragment of detoxified pneumolysin, and pneumococcal surface protein A, and an immunogenic fragment of pneumococcal surface protein A, most preferably the carrier protein is CRM₁₉₇.

-L³- is defined as a linker and is part of the fragment -O-L³-NH-. Thus, the linker -L³- is bound to an oxygen atom and to the nitrogen atom of the -NH-W³-fragment. It is preferred that at least two carbon atoms of the linker are between the oxygen atom and the -NH-W³- fragment, like -O-C-C-NH-W³-. The linker -L³- can be an aliphatic chain, wherein the aliphatic chain can optionally include an aromatic chain inserted in it, or a number of heteroatoms oscillating from 0 to 10. The linker -L³- preferably contains between 2 and 40 carbon atoms (including the carbon atoms of optional side chains), more preferably between 2 and 30, more preferably between 2 and 20, more preferably between 2 and 14, more preferably between 2 and 12, and still more preferably between 2 and 10 carbon atoms. The shortest atom chain between the oxygen atom (i.e. the oxygen of -O-L³-NH-) and the -NH-W³- fragment consists preferably of 2 to 14 atoms, more preferably of 2 to 12 atoms, more preferably of 2 to 10 atoms, more preferably of 2 to 8 atoms. In case the shortest chain (which is the shortest possible connection between the oxygen at the anomeric center and the -NH-W³-fragment) consists of 2 to 6 atoms, these are preferably carbon atoms. In case the shortest chain consists of 4 to 8 atoms, the chain may contain 1, 2 or 3 heteroatoms selected from O, N and S. In case the shortest chain consists of 9 to 14 atoms, the chain may contain 1, 2, 3, 4, 5, or 6 heteroatoms selected from O, N and S.

It is also preferred that the linker -L³-, or the shortest chain is fully or partially fluorinated. The linker -L³- may contain a 3-membered or a 4-membered or a 5-membered or a 6-membered saturated carbocycle or a 5-membered partly unsaturated (and not aromatic) carbocycle or a 4-membered or a 5-membered or a 6-membered saturated oxygen heterocycle or a 4-membered or a 5-membered or a 6-membered saturated nitrogen heterocycle or a 6-membered aromatic carbocycle.

The linker -L³- may also contain amide (-NH-CO-, -CO-NH-) and/or urea (-NH-CO-NH-) residues and preferably only one amide or urea residue. The linker may also contain substituents and preferably two substituents, such as R^{10**} and R^{11**}, or four substituents such as R^{10**}, R^{11**}, R^{15**} and R^{14**}, which have the meanings as defined herein and which are preferably selected from: -F, -Cl, -CH₃, -C₂H₅, -C₃H₇, -C₅H₉, -C₆H₁₃, -OCH₃, -OC₂H₅, -CH₂F, -CHF₂, -CF₃, -C(O)-NH₂, -SCH₃, -SC₂H₅, -NHC(O)CH₃, -N(CH₃)₂, and -N(C₂Hₛ)₂;

In case the linker -L³- is fluorinated, more than two substituents -F are preferred.

Linker -L³- is preferably selected from: -CH₂-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -CF₂-, -(CF₂)₂-, -(CF₂)₃-, -(CF₂)₄-, -(CF₂)₅-, -(CF₂)₆-, -(CF₂)₇-, -(CF₂)₈-, -(CF₂)₉-, -(CF₂)₁₀-, -(CH₂)₂-O-(CH₂)₂-, -CH₂-O-(CH₂)₃-, -(CH₂)₃-O-CH₂, -CH₂-O-(CH₂)₂-, -(CH₂)₂-O-CH₂- -(CH₂)₃-O-(CH₂)₂-, -(CH₂)₂-O-(CH₂)₃-, -(CH₂)₄-O-CH₂-, -CH₂-O-(CH₂)₄-, -L^{a**}-, -L^{a**}-L^{e**}-, -L^{a**}-L^{b**}-L^{e**}-, -L^{a**}-L^{b**}-L^{d**}-L^{C**}-L^{e**}-, -L^{a**}-L^{d**}-L^{e**}-;
wherein
-L^{a**}- is selected from: -(CH₂)_{o**}-, -(CF₂)_{o**}-, -(CH₂-CH₂-O)_{o**}-C₂H₄-, -(CH₂-CH₂-O)_{o**}-CH₂-, -(CR^{10*}R¹¹)_{o**}-, 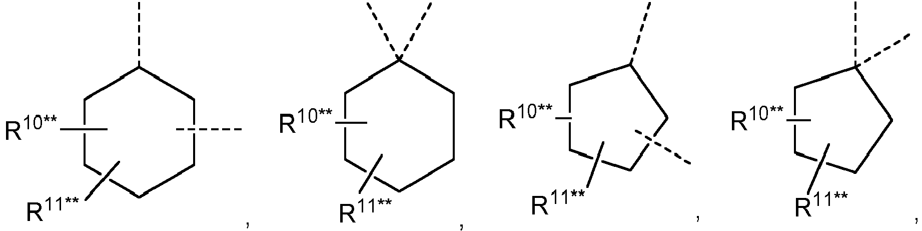 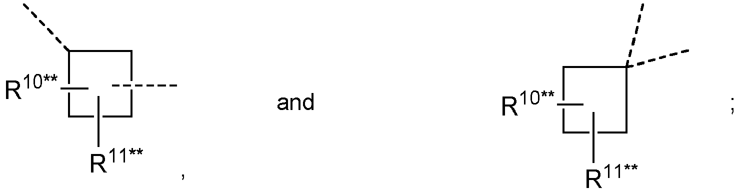 -L^{b**}- and -L^{c**}- are independently of each other selected from: -O-, -NH-C(O)-NH-, -NH-C(S)-NH-, -NH-C(O)-, -C(O)-NH-, -NH-C(O)-O-, -NR^{9**}-, -NR^{18**}-, -SO₂-, 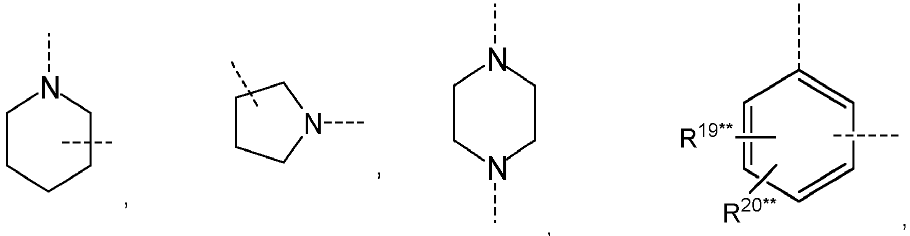 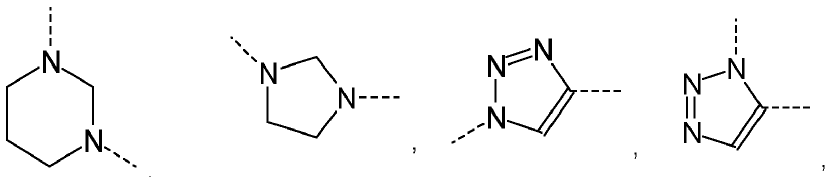 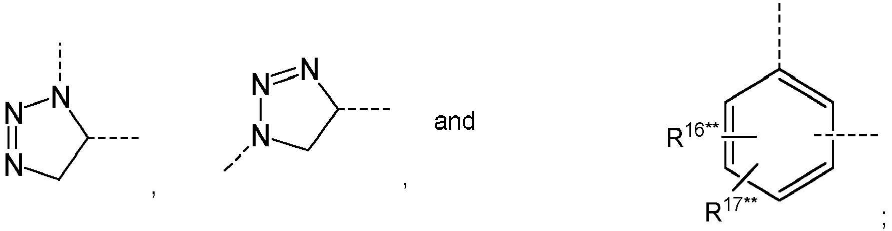 -L^{d**}- represents: -(CH₂)_{q**}-, -(CF₂)_{q**}-, -(CR^{12**}R^{13**})_{q**}-, -(CH₂-CH₂-O)_{q**}-C_{2H4}-, -(CH₂-CH₂-O)_{q**}-CH₂-, 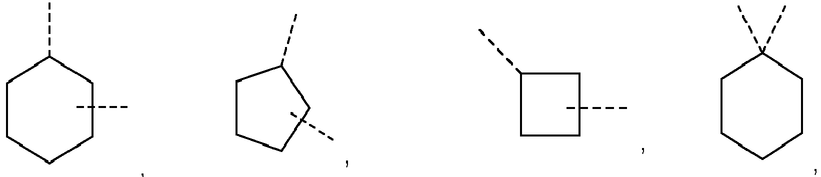 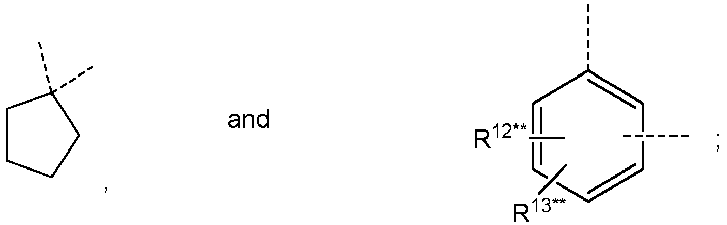 -L^{e**}- is selected from: -(CH₂)_{p1**}-, -(CF₂)_{p1**}-, -C₂H₄-(O-C H₂-CH₂)_{p1**}-, -CH₂-(O-CH₂-CH₂)_{p1**}-, -(CH₂)_{p1**}-O-(CH₂)_{p2**}-, -(CR^{14**}R^{15**})_{p1**}-, -(CR^{14**}R^{15**})_{p1**}-O-(CR^{21**}R ^{22**})_{p2**}-, 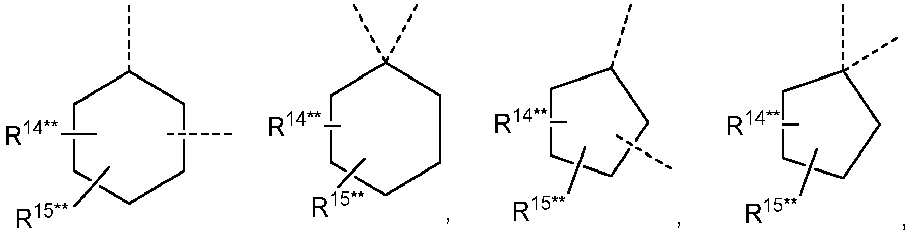 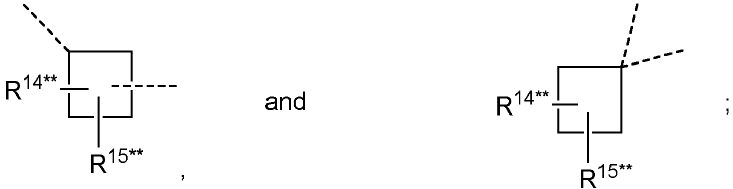 R^{9**} and R^{18**} are independently of each other selected from: -CH₃, -C₂H₅, -C₃H₇, and -C(O)CH₃;
R^{10**}, R^{11**}, R^{12**}, R^{13**}, R^{14**}, R^{15**}, R^{16**}, R^{17**}, R^{19**}, R^{20**}, R^{21**} and R^{22**} are independently of each other selected from: -H, -F, -CI, -CH₃, -C₂H₅, -C₃H₇, -C₅H₉, -C₆H₁₃, -OCH₃, -OC₂H₅, -CH₂F, -CHF₂, -CF₃, -C(O)-NH₂, -SCH₃, -SC₂H₅, -NHC(O)CH₃, -N(CH₃)₂ and -N(C₂H₅)₂;
o**, q**, p1** and p2** are independently of each other an integer selected from 1, 2, 3, 4, 5, 6, 7, 8, 9, and 10.

In the formula **(III),** c1 and c3 represent preferably 0. Thus, *S*. *pneumoniae* serotype 3 conjugate has general formula **(III-A):** 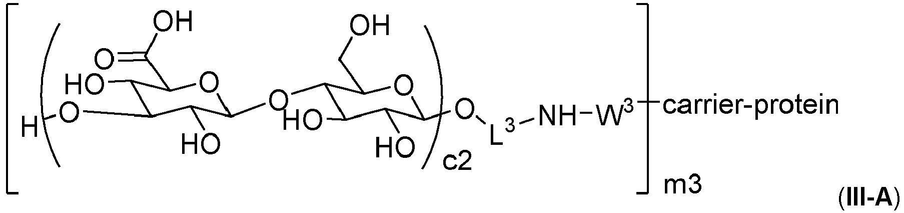 wherein c2, L³, -W³-, m3 and carrier protein have the meaning defined herein.

Preferably the linker -L³- is selected from: -L^{a**}-, -L^{a**}-L^{e**}-, -L^{a**}-L^{b**}-L^{e**}-, -L^{a**}-L^{d**}-L^{e**}-; wherein
-L^{a**}- is selected from: -(CH₂)_{o**}-, -(CH₂-CH₂-O)_{o**}-C₂H₄-, -(CH₂-CH₂-O)_{o**}-CH₂-;
-L^{b**}- represents -O-;
-L^{d**}- is selected from: -(CH₂)_{q**}-, -(CF₂)_{q**}-, -(CH₂-CH₂-O)_{q**}-C₂H₄-, and -(CH₂-CH₂-O)_{q**}-CH₂-;
-L^{e**}- is selected from: -(CH₂)_{p1**}-, -(CF₂)_{p1**}-, -C₂H₄-(O-C H₂-C H₂)_{p1**}-, -CH₂-(O-CH₂-CH₂)ₚ₁**- and -(CH₂)_{p1*}-O-(CH₂)ₚ₂**-;
and o^{**}, q**, p1^{**} and p2^{**} are independently of each other an integer selected from 1, 2, 3, 4, 5, and 6.

More preferably, the linker -L³- represents -(CH₂)_{o**}- and o^{**} is an integer selected from 2, 3, 4, 5, 6, 7 and 8.

It is also preferred that -W³- represents 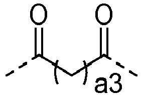 and a3 is an integer selected from 2, 3, 4, 5 and 6.

Most preferably, the glycoconjugate of the synthetic oligosaccharide of S. *pneumoniae* serotype 3 and a carrier protein has the following formula (**IIIa**), 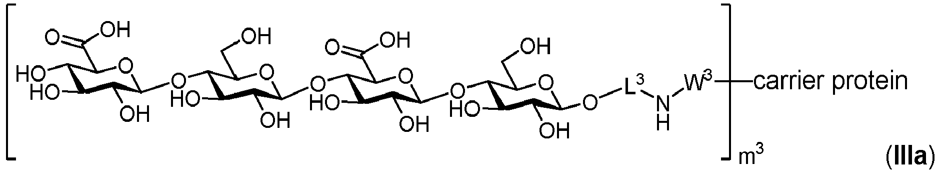
wherein **m³** represents an integer from 3 to 12;
L³ represents -CH₂CH₂-;
**W³** represents -COCH₂CH₂CH₂CH₂CO-, or -COCH₂CH₂CH₂CH₂CH₂;
carrier protein is preferably selected from CRM₁₉₇, detoxified pneumolysin, an immunogenic fragment of detoxified pneumolysin, and pneumococcal surface protein A, and an immunogenic fragment of pneumococcal surface protein A, most preferably the carrier protein is CRM₁₉₇.

### Streptococcus pneumoniae serotype 4

*Streptococcus pneumoniae* serotype 4 capsular polysaccharide is included in all pneumococcal conjugate vaccines. The *Streptococcus pneumoniae* ST4 capsular polysaccharide consists of a tetrasaccharide repeating unit with the sequence β-(1,3)-ManNAc-α-(1,3)-FucNAc-α-(1,3)-GalNAc-α-(1,4)-Gal containing an acid labile *trans*-2, 3 (*S*)-pyruvate on the galactose moiety.

Preferably, a glycoconjugate of a synthetic oligosaccharide of *Streptococcus pneumoniae serotype* 4 and a carrier protein has the following formula **(IV)**

**[H-(D₄-C₄-B₄-A₄)ₙ₄-O-L⁴-NH-W⁴]ₘ₄- carrier protein** **(IV)**

wherein

| | | | |
|---|---|---|---|
| **A₄** represents | 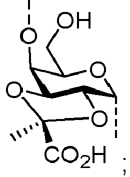 | **B₄** represents | 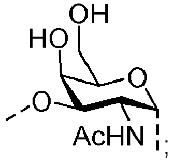 |
| **C₄** represents | 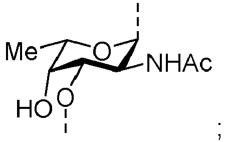 | **D₄** represents | 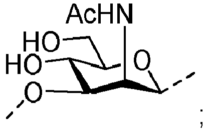 |

n4 is an integer selected from 1, 2, 3, 4, and 5;
L⁴ represents a linker; preferably
-L⁴- is selected from: -L^{a}-, -L^{a}-L^{e}-, -L^{a}-L^{b}-L^{e}- and -L^{a}-L^{d}-L^{e}-;
-L^{a}- is selected from: -(CH₂)ₒ-, -(CH₂-CH₂-O)ₒ-C₂H₄- and
-(CH₂-CH₂-O)ₒ-CH₂;
-L^{b}- represents -O-;
-L^{d}- is selected from: -(CH₂)_{q}-, -(CF₂)_{q}-, -(CH₂-CH₂-O)_{q-}C₂H₄-, and
-(CH₂-CH₂-O)_{q}-CH₂-;
-L^{e}- is selected from: -(CH₂)ₚ₁-, -(CF₂)ₚ₁-, -C₂H₄-(O-CH₂-CH₂)ₚ₁-; -CH₂-(O-CH₂-CH₂)ₚ₁- and -(CH₂)ₚ₁-O-(CH₂)ₚ₂-;
o, q, p1 and p2 are independently of each other an integer selected from 1, 2, 3, 4, 5, and 6;
m⁴ is an integer between 2 and 18;
-W⁴- is selected from: 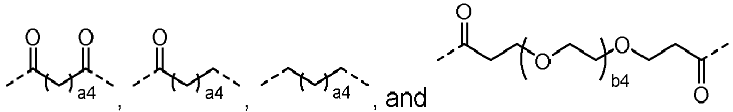
a4 represents an integer from 1 to 10;
b4 represents an integer from 1 to 4; and
carrier protein is preferably selected from CRM₁₉₇, detoxified pneumolysin, an immunogenic fragment of detoxified pneumolysin, and pneumococcal surface protein A, and an immunogenic fragment of pneumococcal surface protein A, most preferably the carrier protein is CRM₁₉₇.

### Streptococcus pneumoniae serotype 5

*S*. *pneumoniae* serotype 5 (ST5) is globally the fifth most prevalent serotype causing Invasive Pneumococcal Disease (IPD) among young children, and the second most prevalent serotype amongst children in the poorest countries of the world, eligible to the support of the Global Alliance for Vaccines and Immunization (GAVI). Furthermore, ST5 was recently identified as the causative agent of an epidemic of severe pneumonia among young Israeli army recruits and community outbreaks of invasive infection in impoverished, urban populations in Canada. A very recent study showed that ST5 is a frequent cause of IPD outbreaks among children and adults in Spain. Although most ST5 subtypes are still susceptible to antibiotics, the emergence and dissemination of antibiotic-resistant ST5 bacteria is of concern.

The ST5 capsular polysaccharide is a component of the most recent PCV10 and PCV13 conjugate vaccines. The ST5 capsular polysaccharide consists of a branched pentasaccharide repeating unit with the sequence [→4)-β-D-Glc*p*-(1→4)-[α-L-Pne*p*NAc-(1→2)-β-D-Glc*p*A-(1→3)]-α-L-Fuc*p*NAc-(1→3)-β-D-Sug*p* (1→], where Sug*p* is 4-keto-D-FucNAc (systematic name: 2-acetamido-2,5-dideoxy-D-*xylo*-hexos-4-ulose) and PneNAc is *N*-acetyl-L-pneumosamine.

Preferably, the glycoconjugate of a synthetic oligosaccharide of *Streptococcus pneumoniae serotype* 5 and a carrier protein has the following formula **(V):**

**[H-D₅-(C₅)_{d2}-(B₅)_{d1}-A₅-O-L⁵-NH-W⁵]ₘ₅-carrier protein** **(V)**

wherein
**A₅** represents: 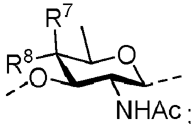
**B₅** represents: 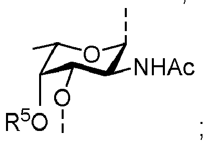
**C₅** represents: 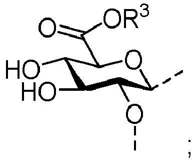
**D₅** represents: 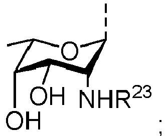
**R⁵** represents: 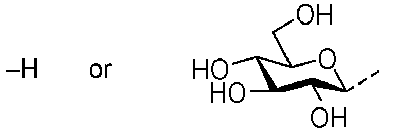
R⁷ and R⁸ are independently of each other selected from -H and -OH and cannot be simultaneously -H;
R⁷ and R⁸ can form together a =O residue; or in other words R⁷ and R⁸ can form together with the carbon atom to which they are attached to a carbonyl group C=O;
R²³ is selected from -H, -C(O)CH₃, -C(O)CF₃ and -C(O)CCl₃;
**L⁵** represents a linker;
d1 and d2 represent independently of each other an integer 0 or 1; d1 and d2 cannot be simultaneously 0; m⁵ is an integer between 2 and 18;
-W⁵- is selected from: 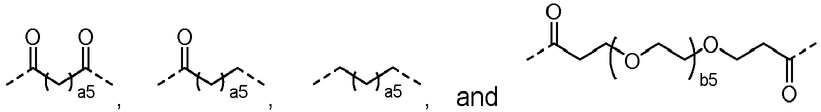
   a5 represents an integer from 1 to 10;
   b5 represents an integer from 1 to 4; and
   carrier protein is preferably selected from CRM₁₉₇, detoxified pneumolysin, an immunogenic fragment of detoxified pneumolysin, and pneumococcal surface protein A, and an immunogenic fragment of pneumococcal surface protein A, most preferably the carrier protein is CRM₁₉₇.
**-L⁵-** is defined as a linker and is part of the fragment -O-L⁵-NH₂. Thus, the linker **-L⁵-** is bound to an oxygen atom and to the nitrogen atom of the NH₂-group. It is preferred that at least two carbon atoms of the linker are between the oxygen atom and the NH₂-group, like -O-C-C-NH₂. The linker **-L⁵-** can be an aliphatic chain, wherein the aliphatic chain can optionally include an aromatic chain inserted in it, or a number of heteroatoms oscillating from 0 to 10.

The linker **L⁵** preferably contains between 2 and 40 carbon atoms (including the carbon atoms of optional side chains), more preferably between 2 and 30, more preferably between 2 and 20, more preferably between 2 and 14, more preferably between 2 and 12, and still more preferably between 2 and 10 carbon atoms.

The shortest atom chain between the oxygen (i.e. the oxygen of -O-**L⁵**-NH₂) and the NH₂-group consists preferably of 2 to 14 atoms, more preferably of 2 to 12 atoms, more preferably of 2 to 10 atoms, more preferably of 2 to 8 atoms. In case the shortest chain (which is the shortest possible connection between the oxygen at the anomeric center and the NH₂-group) consists of 2 to 6 atoms, these are preferably carbon atoms. In case the shortest chain consists of 4 to 8 atoms, the chain may contain 1, or 2 heteroatoms selected from O, N and S. In case the shortest chain consists of 9 to 14 atoms, the chain may contain 1, 2, 3, or 4 heteroatoms selected from O, N and S. It is preferred that the shortest chain contains 0, 1, or 2 sulphur atoms and/or 0, 1, or 2 nitrogen atoms and/or 0, 1, 2, or 3 oxygen atoms.

It is also preferred that the linker -**L⁵**-, or the shortest chain is fully or partially fluorinated. The linker -**L⁵**- may contain a 3-membered or a 4-membered or a 5-membered or a 6-membered saturated carbocycle or a 5-membered partly unsaturated (and not aromatic) carbocycle or a 4-membered or a 5-membered or a 6-membered saturated oxygen heterocycle or a 4-membered or a 5-membered or a 6-membered saturated nitrogen heterocycle or a 6-membered aromatic carbocycle.

The linker -**L⁵**- may also contain amide (-NH-CO-, -CO-NH-) and/or urea (-NH-CO-NH-) residues and preferably only one amide or urea residue. The linker may also contain substituents and preferably one substituent such as R¹⁰ or two substituents such as R¹⁰ and R¹⁴, which have the meanings as defined herein and which are preferably selected from: -F, -CI, -CH₃, -C₂H₅, -C₃H₇, -C₅H₉, -C₆H₁₃, -OCH₃, -OC₂H₅, -CH₂F, -CHF₂, -CF₃, -C(O)-NH₂, -SCH₃, -SC₂H₅, -NHC(O)CH₃, -N(CH₃)₂, and -N(C₂H₅)₂;

In case the linker is fluorinated, more than two substituents -F are preferred.

Preferably the linker -**L⁵**- is selected from: -CH₂-, -(CH₂)₂-, -(CH₂)₃--(CH₂)₄-, -(CH₂)₅-, -(CH₂)₆-, -(CH₂)₇-, -(CH₂)₈-, -(CH₂)₉-, -(CH₂)₁₀-, -CF₂-, -(CF₂)₂-, -(CF₂)₃- -(CF₂)₄-, -(CF₂)₅-, -(CF₂)₆-, -(CF₂)₇--(CF₂)₈-, -(CF₂)₉-, -(CF₂)₁₀-, -(CH₂)₂-O-(CH₂)₂-, -CH₂-O-(CH₂-, -(CH₂)₃-O-CH₂-, -CH₂-O-(CH₂)₂-, -(CH₂)₂-O-CH₂-, -(CH₂)₃-O-(CH₂)₂-, -(CH₂)₂-O-(CH₂)₃-, -(CH₂)₄-O-CH₂-, -CH₂-O-(CH₂)₄-, -L^{a}-, -L^{a}-L^{e}-, -L^{a}-L^{b}-L^{e}-, -L^{a}-L^{b}-L^{d}-L^{c}-L^{e}-, -L^{a}-L^{d}-L^{e}-;
wherein
-L^{a}- is selected from: -(CH₂)ₘ-, -(CF₂)ₘ-, -(CH₂-CH₂-O)ₘ-C₂H₄-, -(CH₂-CH₂-O)m-CH₂-, -(CR¹⁰R¹¹)ₘ-, 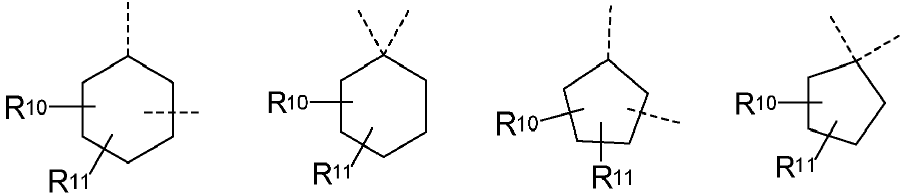 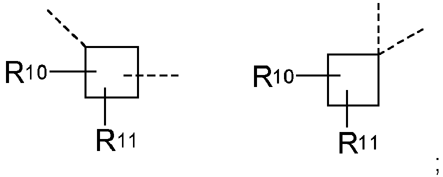 -L^{b}- and -L^{c}- are independently of each other selected from: -O-, -S-, -NH-C(O)-NH-, -NH-C(S)-NH- -NH-C(O)-, -C(O)-NH-, -NH-C(O)-O-, -NR⁹-, -NR¹⁸-, -SO₂-, 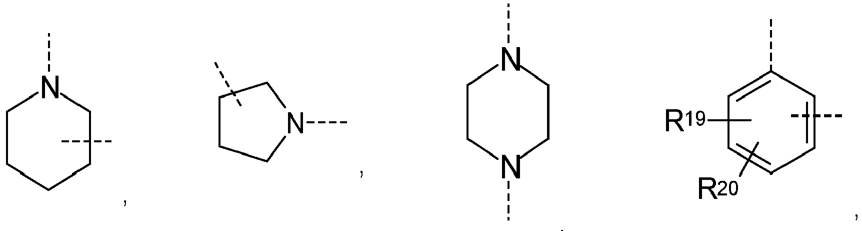 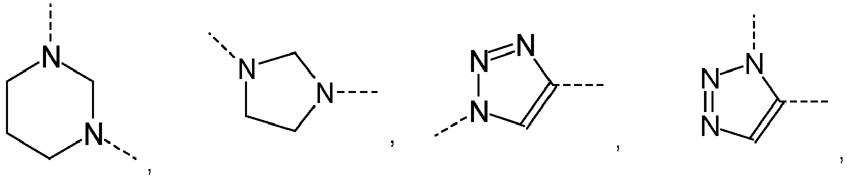 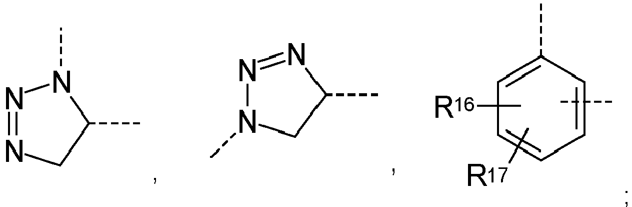 -L^{d}- represents -(CH₂)ₙ-, -(CF₂)ₙ-, -(CR¹²R¹³)ₙ-, -(CH₂-CH₂-O)ₙ-C₂H₄- -(CH₂-CH₂-O)ₙ-CH₂-, 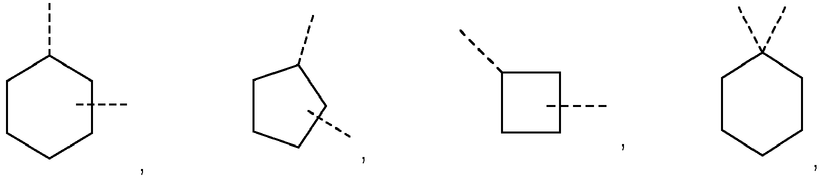 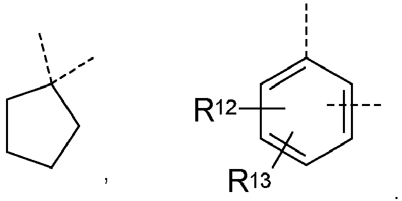 , -L^{e}- is selected from: -(CH₂)ₚ₁-, -(CF₂)ₚ₁-, -C₂H₄-(O-CH₂-CH₂)ₚ₁-, -CH₂-(O-CH₂-CH₂)ₚ₁-, -(CH₂)ₚ₁-O-(CH₂)ₚ₂-, -(CH₂)ₚ₁-S-(CH₂)ₚ₂-, -(CR¹⁴R¹⁵)ₚ₁-, -(CR¹⁴R¹⁵)ₚ₁-O-(CR²¹R²²)ₚ₂-, -(CR¹⁴R¹⁵)ₚ₁-S-(C)ₚ₂-, 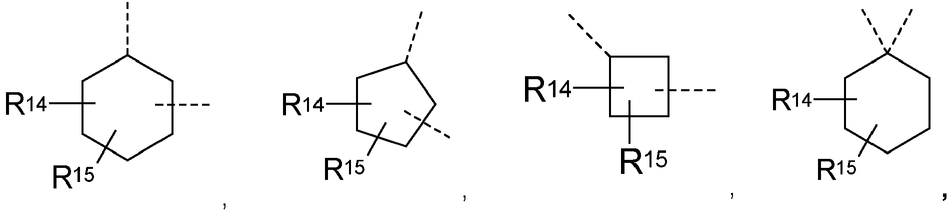 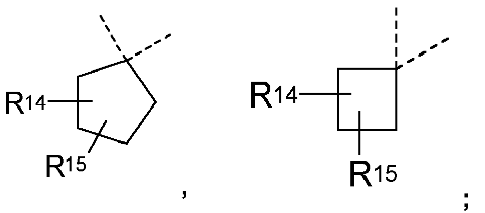 R⁹ and R¹⁸ are independently of each other selected from: -CH₃, -C₂H₅, -C₃H₇, and -C(O)CH₃;
R¹⁰, R¹¹, R¹², R¹³, R¹⁴, R¹⁵, R¹⁶, R¹⁷, R¹⁹, R²⁰, R²¹ and R²² are independently of each other selected from: -H, -F, -CI, -CH₃, -C₂H₅, -C₃H₇, -C₅H₉, -C₆H₁₃, -OCH₃, -OC₂H₅, -CH₂F, -CHF₂, -CF₃, -C(O)-NH₂, -SCH₃, -SC₂H₅, -NHC(O)CH₃, -N(CH₃)₂ and -N(C₂H₅)₂;
m, n, p1 and p2 are independently of each other an integer selected from 1, 2, 3, 4, 5, 6, 7, 8, 9, and 10.

### Streptococcus pneumoniae serotype 8

Preferably, the glycoconjugate of a saccharide from *S*. *pneumoniae* serotype 8 and a carrier protein is a synthetic oligosaccharide from *S*. *pneumoniae* serotype 8 covalently coupled to CRM₁₉₇.

Even more preferably, the conjugate of a saccharide from *S*. *pneumoniae* serotype 8 and a carrier protein has the following general formula **(VI):**

**[V^{*}-Uₓ₊₃-Uₓ₊₂-Uₓ₊₁-Uₓ-O-L⁸-NH-W⁸]ₘ₈-carrier protein** **(VI)**

wherein
x is an integer selected from 1, 2, 3 and 4;
U₁ = U₅ = 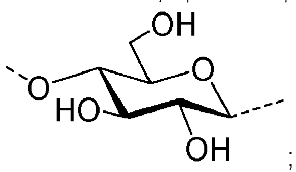
U₂ = U₆ = 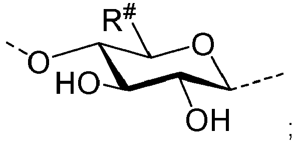
U₃ = U₇ = 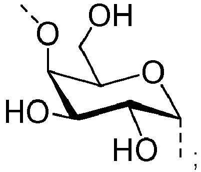
U₄ = 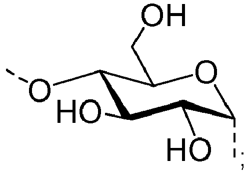
V*- represents H-, H-Uₓ- or H-Uₓ₊₁-Uₓ-;
R^{#} represents -COOH or -CH₂OH;
L⁸ represents a linker;
m8 is comprised between 2 and 18;
-W⁸- is selected from: 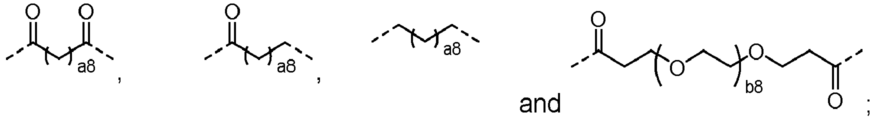
   a1 represents an integer from 1 to 10;
   b1 represents an integer from 1 to 4; and
   carrier protein is preferably selected from CRM₁₉₇, detoxified pneumolysin, an immunogenic fragment of detoxified pneumolysin, and pneumococcal surface protein A, and an immunogenic fragment of pneumococcal surface protein A, most preferably the carrier protein is CRM₁₉₇.
-L⁸- is defined as a linker and is part of the fragment -O-L⁸-NH-. Thus, the linker -L⁸- is bound to an oxygen atom and to the nitrogen atom of the -NH-W¹- fragment. It is preferred that at least two carbon atoms of the linker are between the oxygen atom and the -NH-W¹- fragment, like -O-C-C-NH-W¹-. The linker -L⁸- can be an aliphatic chain, wherein the aliphatic chain can optionally include an aromatic chain inserted in it, or a number of heteroatoms oscillating from 0 to 10. The linker -L⁸- preferably contains between 2 and 40 carbon atoms (including the carbon atoms of optional side chains), more preferably between 2 and 30, more preferably between 2 and 20, more preferably between 2 and 14, more preferably between 2 and 12, and still more preferably between 2 and 10 carbon atoms. The shortest atom chain between the oxygen atom (i.e. the oxygen of -O-L⁸-NH₂) and the -NH-W¹- fragment consists preferably of 2 to 14 atoms, more preferably of 2 to 12 atoms, more preferably of 2 to 10 atoms, more preferably of 2 to 8 atoms. In case the shortest chain (which is the shortest possible connection between the oxygen at the anomeric center and the -NH-W¹- fragment) consists of 2 to 6 atoms, these are preferably carbon atoms. In case the shortest chain consists of 4 to 8 atoms, the chain may contain 1, 2 or 3 heteroatoms selected from O, N and S. In case the shortest chain consists of 9 to 14 atoms, the chain may contain 1, 2, 3, 4, 5, or 6 heteroatoms selected from O, N and S.

It is also preferred that the linker -L⁸- or the shortest chain is fully or partially fluorinated. The linker -L⁸- may contain a 3-membered or a 4-membered or a 5-membered or a 6-membered saturated carbocycle or a 5-membered partly unsaturated (and not aromatic) carbocycle or a 4-membered or a 5-membered or a 6-membered saturated oxygen heterocycle or a 4-membered or a 5-membered or a 6-membered saturated nitrogen heterocycle or a 6-membered aromatic carbocycle.

The linker -L⁸- may also contain amide (-NH-CO-, -CO-NH-) and/or urea (-NH-CO-NH-) residues and preferably only one amide or urea residue. The linker may also contain substituents and preferably two substituents, such as R¹⁰ and R¹¹, or four substituents such as R¹⁰, R¹¹, R¹⁵, and R¹⁴, which have the meanings as defined herein and which are preferably selected from: -F, -CI, -CH₃, -C₂H₅, -C₃H₇, -C₅H₉, -C₆H₁₃, -OCH₃, -OC₂H₅, -CH₂F, -CHF₂, -CF₃, -C(O)-NH₂, -SCH₃, -SC₂H₅, -NHC(O)CH₃, -N(CH₃)₂, and -N(C₂H₅)₂.

In case the linker -L⁸- is fluorinated, more than two substituents -F are preferred.

Linker -L⁸- is preferably selected from: -CH₂-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -CF₂-, -(CF₂)₂-, -(CF₂)₃-, -(CF₂)₄-, -(CF₂)₅-, -(CF₂)₆-, -(CF₂)₇- -(CF₂)₈-, -(CF₂)₉-, -(CF₂)₁₀-, -(CH₂)₂-O-(CH₂)₂-, -CH₂-O-(CH₂)₃- -(CH₂)₃-O-CH₂--CH₂-O-(CH₂)₂-, -(CH₂)₂-O-CH₂-, -(CH₂)₃-O-(CH₂)₂-, -(CH₂)₂-O-(CH₂)₃--(CH₂)₄-O-CH₂-, -CH₂-O-(CH₂)₄-, -L^{a}-, -L^{a}-L^{e}-, -L^{a}-L^{b}-L^{e}-, -L^{a}-L^{b}-L^{d}-L^{c}-L^{e}-, -L^{a}-L^{d}-L^{e}-;
wherein
-L^{a}- is selected from: -(CH₂)ₒ-, -(CF₂)ₒ-, -(CH₂-CH₂-O)ₒ₋C₂H₄-, -(CH₂-CH₂-O)ₒ-CH₂-, -(CR¹⁰R¹¹)ₒ-, 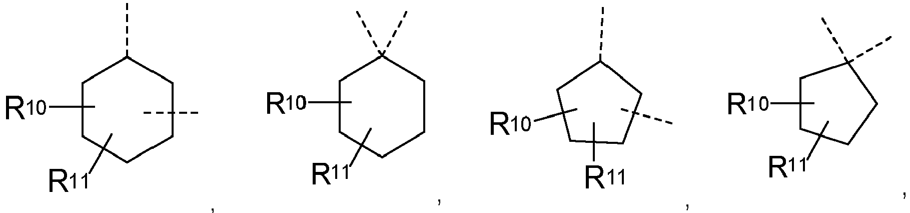 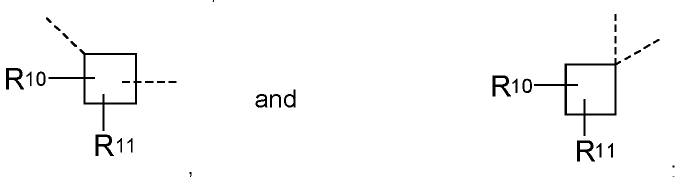 -L^{b}- and -L^{c}- are independently of each other selected from: -O-, -NH-C(O)-NH-, -NH-C(S)-NH- -NH-C(O)-, -C(O)-NH-, -NH-C(O)-O-, -NR⁹-, -NR¹⁸-, -SO₂-, 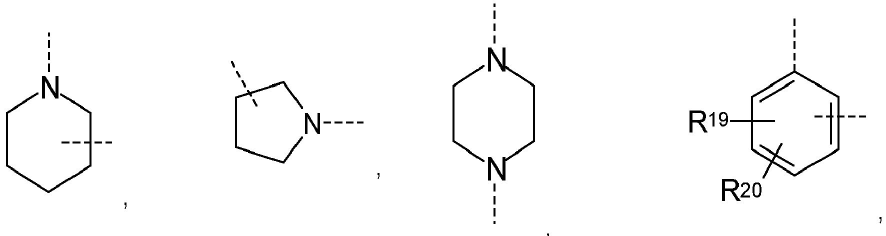 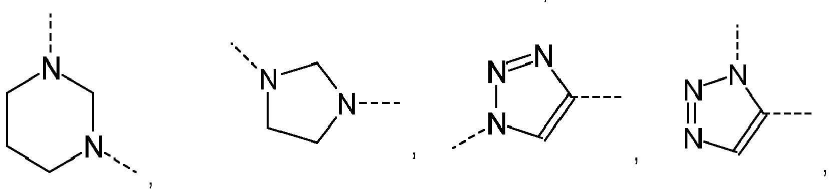 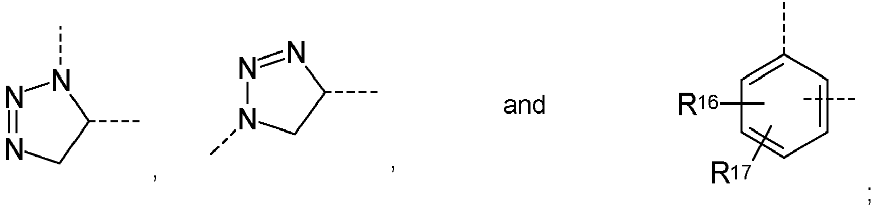 -L^{d}- represents: -(CH₂)_{q}-, -(CF₂)_{q-}, -(CR¹²R¹³)_{q}-, -(CH₂-CH₂-O)_{q}-C₂H₄-, -(CH₂-CH₂-O)_{q}-CH₂-, 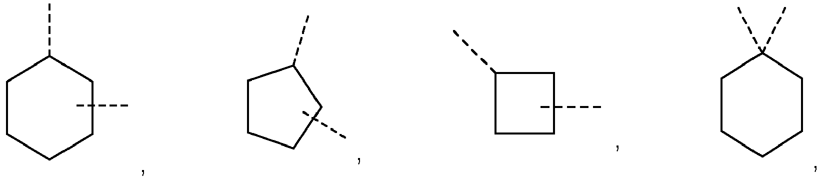 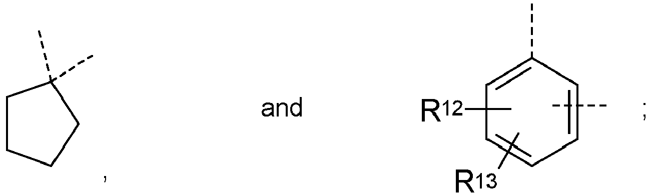 -L^{e}- is selected from: -(CH₂)ₚ₁-, -(CF₂)ₚ₁-, -C₂H₄-(O-CH₂-CH₂)ₚ₁-, -CH₂-(O-CH₂-CH₂)ₚ₁- -(CH₂)ₚ₁-O-(CH₂)ₚ₂-, -(CR¹⁴R¹⁵)ₚ₁-, -(CR¹⁴R¹⁵)ₚ₁-O-(CR²¹R²²)ₚ₂-, 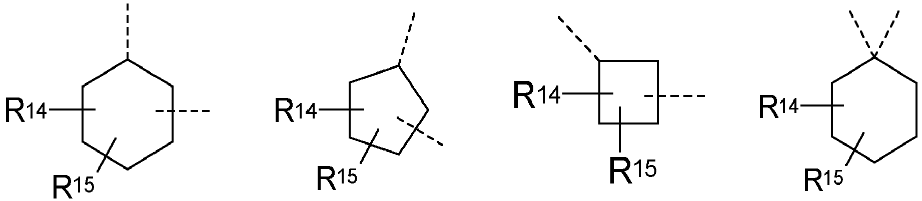 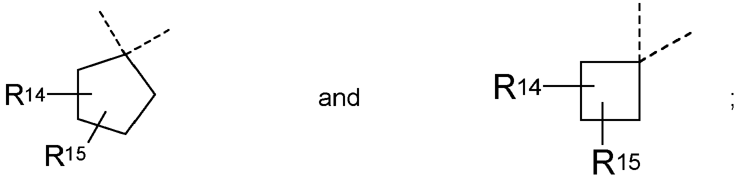 R⁹ and R¹⁸ are independently of each other selected from: -CH₃, -C₂H₅, -C₃H₇, and -C(O)CH₃;
R¹⁰, R¹¹, R¹², R¹³, R¹⁴, R¹⁵, R¹⁶, R¹⁷, R¹⁹, R²⁰, R²¹ and R²² are independently of each other selected from: -H, -F, -Cl, -CH₃, -C₂H₅, -C₃H₇, -C₅H₉, -C₆H₁₃, -OCH₃, -OC₂H₅, -CH₂F, -CHF₂, -CF₃, -C(O)-NH₂, -SCH₃, -SC₂H₅, -NHC(O)CH₃, -N(CH₃)₂ and -N(C₂H₅)₂;
o, q, p1 and p2 are independently of each other an integer selected from 1, 2, 3, 4, 5, 6, 7, 8, 9, and 10.

A spacer as used herein is preferably bifunctional. The spacer is optionally heterobifunctional or homobifunctional, having for example a reactive amino group and a reactive carboxylic acid group, 2 reactive amino groups or two reactive carboxylic acid groups. The spacer has for example between 4 and 20, 4 and 12, 5 and 10 carbon atoms. A possible spacer is adipic acid dihydrazide (ADH). Other spacers include B-propionamido, nitrophenyl-ethylamine, haloalkyl halides, glycosidic linkages, hexane diamine and 6-aminocaproic acid.

Preferably, the vaccine of the present invention comprises at least one glycoconjugate of the synthetic oligosaccharide of *S*. *pneumoniae* serotype 1, 2, 3, 4, 5, and 8 having formula **(I), (II), (III), (IV), (V),** and **(VI),** respectively.

More preferably, the vaccine of the present invention comprises at least one glycoconjugate of the synthetic oligosaccharide of *S*. *pneumoniae* serotype 2 or 3 having any one of the formulae **(II),** or **(III).**

In some embodiments, the vaccine of the present invention comprises at least one glycoconjugate of the synthetic oligosaccharide of *S*. *pneumoniae* serotype 3 having the formula **(III)**

**[H-(C)_{c3}-(D-C)_{c2}-(D)_{c1}-O-L³-NH-W³]ₘ₃-carrier protein** **(III)**

wherein
C represents: 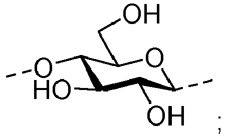
D represents: 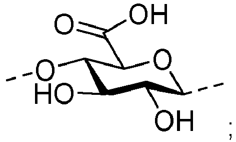
c2 is an integer selected from 1, 2, 3, 4, 5, 6, 7, 8, 9 and 10;
c1 and c3 represent independently of each other an integer 0 or 1;
L³ represents a linker;
m³ is an integer between 2 and 18;
-W³- is selected from: 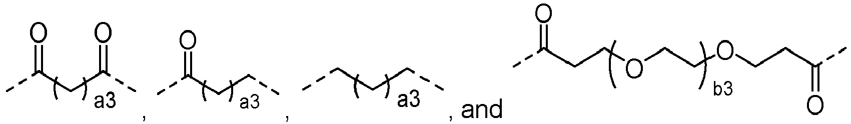
   a3 represents an integer from 1 to 10;
   b3 represents an integer from 1 to 4; and
   carrier protein has the meaning as defined above, the carrier protein is preferably selected from CRM₁₉₇, detoxified pneumolysin, an immunogenic fragment of detoxified pneumolysin, and pneumococcal surface protein A, and an immunogenic fragment of pneumococcal surface protein A, most preferably the carrier protein is CRM₁₉₇.

Therefore, the present invention refers to a vaccine comprising
a) at least one glycoconjugate of a synthetic *Streptococcus pneumoniae* serotype 3 oligosaccharide having the formula **(III);**
b) at least one adjuvant, wherein the at least one adjuvant is selected from agonists of toll-like receptors 1, 2, 4, 6, 7, 8, or 9,
   wherein the at least one glycoconjugate and the at least one adjuvant are encapsulated in biocompatible and biodegradable polymer particles. Preferably, the biocompatible and biodegradable polymer particles are selected from the polylactic acid (PLA) particles, polyglycolic acid (PGA) particles, poly(lactic-co-glycolic acid) (PLGA) particles, PLGA/PEG copolymers (PLGA-PEG) particles and tri-block PLGA/PEG/PLGA copolymers particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

Thus, the present invention preferably refers to a vaccine comprising
a) at least one glycoconjugate of a synthetic *Streptococcus pneumoniae* serotype 3 oligosaccharide having the formula **(III),**
b) at least one adjuvant, wherein the at least one adjuvant is selected from monophosphoryl lipid A (MPLA), CRX-524, 1*H*-imidazo[4,5-c]quinoline compounds, 8-oxoadenine compounds, a mixture of monophosphoryl lipid A and one of the 1*H*-imidazo[4,5-c]quinoline compounds, isolated bacterial RNA and cytosine-phosphate-guanosine(CpG) oligodeoxynucleotides;
   wherein the at least one glycoconjugate and the at least one adjuvant are encapsulated in biocompatible and biodegradable polymer particles. Preferably, the biocompatible and biodegradable polymer particles are selected from the polylactic acid (PLA) particles, polyglycolic acid (PGA) particles, poly(lactic-co-glycolic acid) (PLGA) particles, PLGA/PEG copolymers (PLGA-PEG) particles and tri-block PLGA/PEG/PLGA copolymers particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

The present invention preferably refers to a vaccine comprising
a) at least one glycoconjugate of a synthetic *Streptococcus pneumoniae* serotype 3 oligosaccharide having the formula **(III),**
b) at least one adjuvant selected from monophosphoryl lipid A (MPLA), CRX-524, resiquimod, imiquimod, SM360320, GSK2245035, a mixture of monophosphoryl lipid A and resiquimod, isolated bacterial RNA, and CPG7909;
   wherein the at least one glycoconjugate and the at least one adjuvant are encapsulated in biocompatible and biodegradable polymer particles; and the biocompatible and biodegradable polymer particles are selected from the polylactic acid (PLA) particles, polyglycolic acid (PGA) particles, poly(lactic-co-glycolic acid) (PLGA) particles, PLGA/PEG co-polymers (PLGA-PEG) particles and tri-block PLGA/PEG/PLGA copolymers particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

Also preferably, the present invention refers to a vaccine comprising
a) at least one glycoconjugate of a synthetic *Streptococcus pneumoniae* serotype 3 oligosaccharide having the formula **(III),**
b) at least one adjuvant, wherein the at least one adjuvant is selected from monophosphoryl lipid A (MPLA), resiquimod, a mixture of monophosphoryl lipid A and resiquimod, and isolated bacterial RNA;
   wherein the at least one glycoconjugate and the at least one adjuvant are encapsulated in biocompatible and biodegradable polymer particles; and the biocompatible and biodegradable polymer particles are selected from the polylactic acid (PLA) particles, polyglycolic acid (PGA) particles, poly(lactic-co-glycolic acid) (PLGA) particles, PLGA/PEG co-polymers (PLGA-PEG) particles and tri-block PLGA/PEG/PLGA copolymers particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

Also more preferably, the present invention refers to a vaccine comprising
a) at least one glycoconjugate of a synthetic *Streptococcus pneumoniae* serotype 3 oligosaccharide having the formula **(III),**
b) at least one adjuvant, wherein the at least one adjuvant is selected from monophosphoryl lipid A (MPLA), resiquimod, a mixture of monophosphoryl lipid A and resiquimod, and isolated bacterial RNA;
   wherein the at least one glycoconjugate and the at least one adjuvant are encapsulated in biocompatible and biodegradable polymer particles; and
   the biocompatible and biodegradable polymer particles are poly(lactic-co-glycolic acid) (PLGA) particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

In some embodiments, the vaccine of the present invention comprises the at least one glycoconjugate having the following formula **(IIIa)** 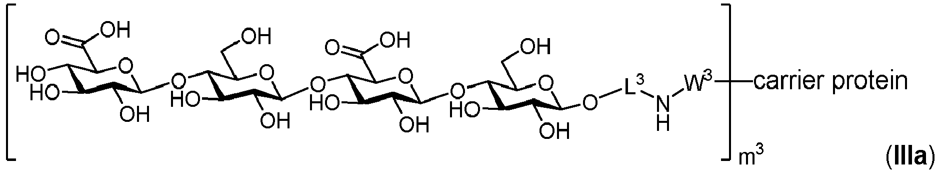
wherein **m³** represents an integer from 3 to 12;
**L³** represents -CH₂CH₂-;
**W³** represents -COCH₂CH₂CH₂CH₂CO-, or -COCH₂CH₂CH₂CH₂CH₂;
carrier protein is preferably selected from CRM₁₉₇, detoxified pneumolysin, an immunogenic fragment of detoxified pneumolysin, and pneumococcal surface protein A, and an immunogenic fragment of pneumococcal surface protein A, most preferably the carrier protein is CRM₁₉₇.

Therefore, the present invention refers to a vaccine comprising
a) at least one glycoconjugate of a synthetic *Streptococcus pneumoniae* serotype 3 oligosaccharide having the formula **(IIIa);**
b) at least one adjuvant, wherein the at least one adjuvant is selected from agonists of toll-like receptors 1, 2, 4, 6, 7, 8, or 9,
   wherein the at least one glycoconjugate and the at least one adjuvant are encapsulated in biocompatible and biodegradable polymer particles. Preferably, the biocompatible and biodegradable polymer particles are selected from the polylactic acid (PLA) particles, polyglycolic acid (PGA) particles, poly(lactic-co-glycolic acid) (PLGA) particles, PLGA/PEG co-polymers (PLGA-PEG) particles and tri-block PLGA/PEG/PLGA copolymers particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

Thus, the present invention preferably refers to a vaccine comprising
a) at least one glycoconjugate of a synthetic *Streptococcus pneumoniae* serotype 3 oligosaccharide having the formula (**IIIa**),
b) at least one adjuvant, wherein the at least one adjuvant is selected from monophosphoryl lipid A (MPLA), CRX-524, 1*H*-imidazo[4,5-c]quinoline compounds, 8-oxoadenine compounds, a mixture of monophosphoryl lipid A and one of the 1*H*-imidazo[4,5-c]quinoline compounds, isolated bacterial RNA and cytosine-phosphate-guanosine(CpG) oligodeoxynucleotides;
   wherein the at least one glycoconjugate and the at least one adjuvant are encapsulated in biocompatible and biodegradable polymer particles. Preferably, the biocompatible and biodegradable polymer particles are selected from the polylactic acid (PLA) particles, polyglycolic acid (PGA) particles, poly(lactic-co-glycolic acid) (PLGA) particles, PLGA/PEG co-polymers (PLGA-PEG) particles and tri-block PLGA/PEG/PLGA copolymers particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

The present invention preferably refers to a vaccine comprising
a) at least one glycoconjugate of a synthetic *Streptococcus pneumoniae* serotype 3 oligosaccharide having the formula (**IIIa**),
b) at least one adjuvant selected from monophosphoryl lipid A (MPLA), CRX-524, resiquimod, imiquimod, SM360320, GSK2245035, a mixture of monophosphoryl lipid A and resiquimod, isolated bacterial RNA, and CPG7909;
   wherein the at least one glycoconjugate and the at least one adjuvant are encapsulated in biocompatible and biodegradable polymer particles; and the biocompatible and biodegradable polymer particles are selected from the polylactic acid (PLA) particles, polyglycolic acid (PGA) particles, poly(lactic-co-glycolic acid) (PLGA) particles, PLGA/PEG co-polymers (PLGA-PEG) particles and tri-block PLGA/PEG/PLGA copolymers particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

More preferably, the present invention refers to a vaccine comprising
a) at least one glycoconjugate of a synthetic *Streptococcus pneumoniae* serotype 3 oligosaccharide having the formula (**IIIa**),
b) at least one adjuvant, wherein the at least one adjuvant is selected from monophosphoryl lipid A (MPLA), resiquimod, a mixture of monophosphoryl lipid A and resiquimod, isolated bacterial RNA;
   wherein the at least one glycoconjugate and the at least one adjuvant are encapsulated in biocompatible and biodegradable polymer particles; and the biocompatible and biodegradable polymer particles are selected from the polylactic acid (PLA) particles, polyglycolic acid (PGA) particles, poly(lactic-co-glycolic acid) (PLGA) particles, PLGA/PEG co-polymers (PLGA-PEG) particles and tri-block PLGA/PEG/PLGA copolymers particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

Still more preferably, the present invention refers to a vaccine comprising
a) at least one glycoconjugate of a synthetic *Streptococcus pneumoniae* serotype 3 oligosaccharide having the formula (**IIIa**),
b) at least one adjuvant, wherein the at least one adjuvant is selected from monophosphoryl lipid A (MPLA), resiquimod, a mixture of monophosphoryl lipid A and resiquimod, isolated bacterial RNA;
   wherein the at least one glycoconjugate and the at least one adjuvant are encapsulated in biocompatible and biodegradable polymer particles; and the biocompatible and biodegradable polymer particles are poly(lactic-co-glycolic acid) particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

Preferably, the present invention refers to a vaccine comprising
a) at least two glycoconjugates of a synthetic *Streptococcus pneumoniae* oligosaccharide having any one of the formula **(II),** or **(III);**
b) at least one adjuvant, wherein the at least one adjuvant is selected from agonists of toll-like receptors 1, 2, 4, 6, 7, 8, or 9,
   wherein the at least one glycoconjugate and the at least one adjuvant are encapsulated in biocompatible and biodegradable polymer particles. Preferably, the biocompatible and biodegradable polymer particles are selected from the polylactic acid (PLA) particles, polyglycolic acid (PGA) particles, poly(lactic-co-glycolic acid) particles, PLGA/PEG co-polymers (PLGA-PEG) particles and tri-block PLGA/PEG/PLGA copolymers particles, and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

The present invention preferably refers to a vaccine comprising
a) at least two glycoconjugates of a synthetic *Streptococcus pneumoniae* oligosaccharide having any one of the formula **(II)** or **(III),**
b) at least one adjuvant, wherein the at least one adjuvant is selected from monophosphoryl lipid A (MPLA), CRX-524, 1*H*-imidazo[4,5-c]quinoline compounds, 8-oxoadenine compounds, a mixture of monophosphoryl lipid A and one of the 1*H*-imidazo[4,5-c]quinoline compounds, isolated bacterial RNA and cytosine-phosphate-guanosine(CpG) oligodeoxynucleotides;
   wherein the at least one glycoconjugate and the at least one adjuvant are encapsulated in biocompatible and biodegradable polymer particles. Preferably, the biocompatible and biodegradable polymer particles are selected from the polylactic acid (PLA) particles, polyglycolic acid (PGA) particles, poly(lactic-co-glycolic acid) particles, PLGA/PEG co-polymers (PLGA-PEG) particles and tri-block PLGA/PEG/PLGA copolymers particles, and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

The present invention preferably refers to a vaccine comprising
a) at least two glycoconjugates of a synthetic *Streptococcus pneumoniae* oligosaccharide having any one of the formula **(II)** or **(III),**
b) at least one adjuvant, wherein the at least one adjuvant is selected from monophosphoryl lipid A (MPLA), resiquimod, a mixture of monophosphoryl lipid A and resiquimod, and isolated bacterial RNA;
   wherein the at least one glycoconjugate and the at least one adjuvant are encapsulated in biocompatible and biodegradable polymer particles. Preferably, the biocompatible and biodegradable polymer particles are selected from the polylactic acid (PLA) particles, polyglycolic acid (PGA) particles, poly(lactic-co-glycolic acid) particles, PLGA/PEG co-polymers (PLGA-PEG) particles and tri-block PLGA/PEG/PLGA copolymers particles,
and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

The present invention preferably refers to a vaccine comprising
a) at least one glycoconjugate of a synthetic *Streptococcus pneumoniae* oligosaccharide having any one of the formula **(II)** or **(III),**
b) at least one adjuvant selected from monophosphoryl lipid A (MPLA), CRX-524, resiquimod, imiquimod, SM360320, GSK2245035, a mixture of monophosphoryl lipid A and resiquimod, isolated bacterial RNA and CPG7909;
   wherein the at least one glycoconjugate and the at least one adjuvant are encapsulated in biocompatible and biodegradable polymer particles; and
   the biocompatible and biodegradable polymer particles are selected from the polylactic acid (PLA) particles, polyglycolic acid (PGA) particles, poly(lactic-co-glycolic acid) particles, PLGA/PEG co-polymers (PLGA-PEG) particles and tri-block PLGA/PEG/PLGA copolymers particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

Also preferably, the present invention refers to a vaccine comprising
a) at least one glycoconjugate of a synthetic *Streptococcus pneumoniae* oligosaccharides having any one of the formulae **(II),** or **(III),**
b) at least one adjuvant, wherein the at least one adjuvant is selected from monophosphoryl lipid A (MPLA), resiquimod, a mixture of monophosphoryl lipid A and resiquimod, isolated bacterial RNA;
   wherein the at least one glycoconjugate and the at least one adjuvant are encapsulated in biocompatible and biodegradable polymer particles; and the biocompatible and biodegradable polymer particles are selected from the polylactic acid (PLA) particles, polyglycolic acid (PGA) particles, poly(lactic-co-glycolic acid) particles, PLGA/PEG co-polymers (PLGA-PEG) particles and tri-block PLGA/PEG/PLGA copolymers particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

Also more preferably, the present invention refers to a vaccine comprising
a) at least one glycoconjugate of synthetic *Streptococcus pneumoniae* oligosaccharides having any one of the formulae **(II),** or **(III),**
b) at least one adjuvant, wherein the at least one adjuvant is selected from monophosphoryl lipid A (MPLA), resiquimod, a mixture of monophosphoryl lipid A and resiquimod, isolated bacterial RNA;
   wherein the at least one glycoconjugate and the at least one adjuvant are encapsulated in biocompatible and biodegradable polymer particles; and the biocompatible and biodegradable polymer particles are poly(lactic-co-glycolic acid) particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

Still more preferably, the present invention refers to a vaccine comprising
a) at least one glycoconjugate of synthetic *Streptococcus pneumoniae* oligosaccharides having any one of the formulae **(IIa),** or (**IIIa**),
b) at least one adjuvant, wherein the at least one adjuvant is selected from monophosphoryl lipid A (MPLA), resiquimod, a mixture of monophosphoryl lipid A and resiquimod, isolated bacterial RNA;
   wherein the at least one glycoconjugate and the at least one adjuvant are encapsulated in biocompatible and biodegradable polymer particles; and
   the biocompatible and biodegradable polymer particles are selected from the polylactic acid (PLA) particles, polyglycolic acid (PGA) particles, poly(lactic-co-glycolic acid) particles, PLGA/PEG co-polymers (PLGA-PEG) particles and tri-block PLGA/PEG/PLGA copolymers particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

Also still more preferably, the present invention refers to a vaccine comprising
a) at least one glycoconjugate of synthetic *Streptococcus pneumoniae* oligosaccharides having any one of the formulae **(IIa),** or (**IIIa**),
b) at least one adjuvant, wherein the at least one adjuvant is selected from monophosphoryl lipid A (MPLA), resiquimod, a mixture of monophosphoryl lipid A and resiquimod, isolated bacterial RNA;
   wherein the at least one glycoconjugate and the at least one adjuvant are encapsulated in biocompatible and biodegradable polymer particles; and
   the biocompatible and biodegradable polymer particles are poly(lactic-co-glycolic acid) particles,
   and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

### Further Immunogenic Components

In some embodiment, the vaccine of the present invention additionally comprises at least one immunogenic component, wherein the at least one immunogenic component is selected from or consisting of:
detoxified pneumolysin (Ply), an immunogenic fragment of detoxified pneumolysin (Ply);
a pneumococcal surface antigen A, an immunogenic fragment of a pneumococcal surface antigen A,
a pneumococcal surface protein A, an immunogenic fragment of a pneumococcal surface protein A, and
a glycosphingolipid.

Thus, the present invention is directed to a vaccine comprising
a) at least one glycoconjugate of a synthetic *Streptococcus pneumoniae* oligosaccharide, and a carrier protein;
b) at least one adjuvant, wherein the at least one adjuvant is selected from agonists of toll-like receptors 1, 2, 4, 6, 7, 8, or 9,
c) at least one immunogenic component, wherein the at least one immunogenic component is selected from the group comprising or consisting of:
   detoxified pneumolysin (Ply), an immunogenic fragment of detoxified pneumolysin (Ply);
   a pneumococcal surface antigen A, an immunogenic fragment of a pneumococcal surface antigen A,
   a pneumococcal surface protein A, an immunogenic fragment of a pneumococcal surface protein A, and
   a glycosphingolipid,
   wherein the at least one glycoconjugate and the at least one adjuvant are encapsulated in biocompatible and biodegradable polymer particles.

The at least one glycoconjugate of synthetic *Streptococcus pneumoniae* oligosaccharide is preferably, a glycoconjugate of synthetic oligosaccharides of *Streptococcus pneumoniae serotype 1,* a glycoconjugate of synthetic oligosaccharides of *Streptococcus pneumoniae serotype 2,* a glycoconjugate of synthetic oligosaccharides of *Streptococcus pneumoniae serotype 3,* a glycoconjugate of synthetic oligosaccharides of *Streptococcus pneumoniae serotype 4,* a glycoconjugate of synthetic oligosaccharides of *Streptococcus pneumoniae serotype 5, or* a glycoconjugate of synthetic oligosaccharides of *Streptococcus pneumoniae serotype 8.* More preferably, the at least one glycoconjugate of synthetic *Streptococcus pneumoniae* oligosaccharides is a glycoconjugate of synthetic oligosaccharides of *Streptococcus pneumoniae serotype* 2 or a glycoconjugate of synthetic oligosaccharides of *Streptococcus pneumoniae serotype* 3, still more preferably a glycoconjugate of synthetic oligosaccharides of *Streptococcus pneumoniae serotype* 3. Most preferably, the at least one glycoconjugate of synthetic *Streptococcus pneumoniae* oligosaccharide is a glycoconjugate of synthetic oligosaccharides of *Streptococcus pneumoniae serotype* 3 having the formula **(III).**

The carrier protein is selected from diphtheria toxoid, detoxified pneumolysin, an immunogenic fragment of detoxified pneumolysin, pneumococcal surface protein A (PspA), an immunogenic fragment of pneumococcal surface protein A, and tetanus toxoid (TT). Preferably, the carrier protein is selected from CRM₁₉₇, detoxified pneumolysin, an immunogenic fragment of detoxified pneumolysin, pneumococcal surface protein A (PspA), an immunogenic fragment of pneumococcal surface protein A, and tetanus toxoid (TT); more preferably, the carrier protein is CRM₁₉₇.

The at least one adjuvant is selected from monophosphoryl lipid A (MPLA), CRX-524, 1*H*-imidazo[4,5-c]quinoline compounds, 8-oxoadenine compounds, a mixture of monophosphoryl lipid A and one of the 1*H*-imidazo[4,5-c]quinoline compounds, isolated bacterial RNA and cytosine-phosphate-guanosine(CpG) oligodeoxynucleotides;
Preferably, the at least one adjuvant is selected from monophosphoryl lipid A (MPLA), CRX-524, resiquimod, imiquimod, SM360320, GSK2245035, a mixture of monophosphoryl lipid A and resiquimod, isolated bacterial RNA, and CPG7909; Still more preferably, the at least one adjuvant is selected from monophosphoryl lipid A (MPLA), resiquimod, a mixture of monophosphoryl lipid A and resiquimod, and isolated bacterial RNA.

The biocompatible and biodegradable polymer particles are preferably selected from polylactic acid (PLA) particles, polyglycolic acid (PGA) particles, poly(lactic-co-glycolic acid) particles, PLGA/PEG co-polymers (PLGA-PEG) particles and tri-block PLGA/PEG/PLGA copolymers particles, and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

Thus, a particular preferred vaccine according to the present invention comprises:
a) at least one glycoconjugate of synthetic oligosaccharides of *Streptococcus pneumoniae* serotype 3 having the formula **(III);**

   **[H-(C)_{c3}-(D-C)_{c2}-(D)_{c1}-O-L³-NH-W³]ₘ₃-carrier protein** **(III)**

   wherein
   C represents: 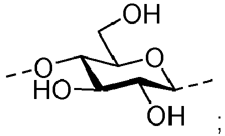
   D represents: 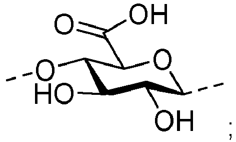
   c2 is an integer selected from 1, 2, 3, 4, 5, 6, 7, 8, 9 and 10;
   c1 and c3 represent independently of each other an integer 0 or 1;
   L³ represents a linker;
   m³ is an integer between 2 and 18;
   -W³- is selected from: 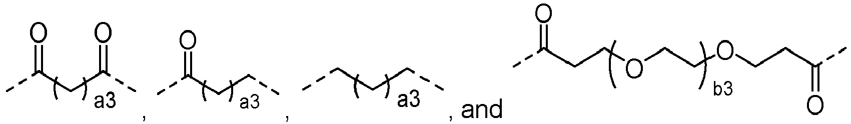
      a3 represents an integer from 1 to 10;
      b3 represents an integer from 1 to 4; and
      carrier protein has the meaning as defined above; the carrier protein preferably selected from CRM₁₉₇, detoxified pneumolysin, an immunogenic fragment of detoxified pneumolysin, and pneumococcal surface protein A, and an immunogenic fragment of pneumococcal surface protein A, most preferably the carrier protein is CRM₁₉₇.
b) at least one adjuvant, wherein the at least one adjuvant is selected from monophosphoryl lipid A (MPLA), resiquimod, a mixture of monophosphoryl lipid A and resiquimod, and isolated bacterial RNA,
c) at least one immunogenic component, wherein the at least one immunogenic component is selected from the group comprising or consisting of:
   detoxified pneumolysin (Ply), an immunogenic fragment of detoxified pneumolysin (Ply);
   a pneumococcal surface antigen A, an immunogenic fragment of a pneumococcal surface antigen A,
   a pneumococcal surface protein A, an immunogenic fragment of a pneumococcal surface protein A, and
   a glycosphingolipid.,
wherein the at least one glycoconjugate and the at least one adjuvant are encapsulated in biocompatible and biodegradable polymer particles, and
the biocompatible and biodegradable polymer particles are selected from the polylactic acid (PLA) particles, polyglycolic acid (PGA) particles, poly(lactic-co-glycolic acid) (PLGA) particles, PLGA/PEG co-polymers (PLGA-PEG) particles and tri-block PLGA/PEG/PLGA copolymers particles, and
optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

### Further glycoconjugates of capsular polysaccharides of S. pneumoniae

The vaccine of the invention may also comprise glycoconjugates of *S. pneumoniae* capsular saccharides individually conjugated to a carrier protein.
The *S. pneumoniae* capsular saccharides individually conjugated to a carrier protein may be selected from a *Streptococcus pneumoniae* of serotypes 1, 2, 3, 4, 5, 6A, 6B, 7A, 7B, 7C, 8, 9A, 9L, 9N, 9V, 10A, 10B, 10C, 10F, 11A, 11 B, 11C, 11D, 11F, 12A, 12B, 12F, 13, 14, 15A, 15B, 15C, 15F, 16A, 16F, 17A, 17F, 18A, 18B, 18C, 18F, 19A, 19B, 19C, 19F, 20, 2 1, 22A, 22F, 23A, 23B, 23F, 24A, 24B, 24F, 25A, 25F, 26, 27, 28A, 28F, 29, 31, 32A, 32F, 33A, 33B, 33C, 33D, 33F, 34, 35A, 35B, 35C, 35D, 35F, 36, 37, 38, 39, 40, 41A, 41F, 42, 43, 44, 45, 46, 47A, 47F or 48 capsular saccharide.
Preferably, the *S. pneumoniae* capsular saccharides individually conjugated to a carrier protein are selected from a *Streptococcus pneumoniae* serotypes 1, 3, 4, 5, 6A, 6B, 7F, 9V, 14, 18C, 19A, 19F, and 23F.

In one embodiment, the vaccine according to any one of the claims 1 to 11, further comprising
one or more capsular polysaccharides from *Streptococcus pneumoniae* serotypes 1, 3, 4, 5, 6A, 6B, 7F, 9V, 14, 18C, 19A, 19F and 23F individually conjugated to CRM₁₉₇ carrier protein.

Thus, the present invention is directed to a vaccine comprising
a) at least one glycoconjugate of a synthetic *Streptococcus pneumoniae* oligosaccharide and a carrier protein;
b) at least one adjuvant, wherein the at least one adjuvant is selected from agonists of toll-like receptors 1, 2, 4, 6, 7, 8, or 9,
d) one or more glycoconjugates of *S. pneumoniae* capsular saccharides individually conjugated to a carrier protein
   wherein the at least one glycoconjugate and the at least one adjuvant are encapsulated in biocompatible and biodegradable polymer particles.

Preferably, the present invention is directed to a vaccine comprising
a) at least one glycoconjugate of synthetic *Streptococcus pneumoniae* oligosaccharides;
b) at least one adjuvant, wherein the at least one adjuvant is selected from agonists of toll-like receptors 1, 2, 4, 6, 7, 8, or 9,
c) at least one immunogenic component, wherein the at least one immunogenic component is selected from the group comprising or consisting of:
   detoxified pneumolysin (Ply), an immunogenic fragment of detoxified pneumolysin (Ply);
   a pneumococcal surface antigen A, an immunogenic fragment of a pneumococcal surface antigen A,
   a pneumococcal surface protein A, an immunogenic fragment of a pneumococcal surface protein A, and
   a glycosphingolipid,
d) one or more glycoconjugates of *S. pneumoniae* capsular saccharides individually conjugated to a carrier protein
   wherein the at least one glycoconjugate and the at least one adjuvant are encapsulated in biocompatible and biodegradable polymer particles.

The at least one glycoconjugate of synthetic *Streptococcus pneumoniae* oligosaccharide is preferably selected from a glycoconjugate of synthetic oligosaccharides of *Streptococcus pneumoniae serotype* 1, a glycoconjugate of synthetic oligosaccharides of *Streptococcus pneumoniae serotype 2,* a glycoconjugate of synthetic oligosaccharides of *Streptococcus pneumoniae serotype 3,* a glycoconjugate of synthetic oligosaccharides of *Streptococcus pneumoniae serotype 4,* a glycoconjugate of synthetic oligosaccharides of *Streptococcus pneumoniae serotype 5, or* a glycoconjugate of synthetic oligosaccharides of *Streptococcus pneumoniae serotype 8.,* More preferably, the at least one glycoconjugate of synthetic *Streptococcus pneumoniae* oligosaccharide is a glycoconjugate of synthetic oligosaccharides of *Streptococcus pneumoniae serotype* 2 or a glycoconjugate of synthetic oligosaccharides of *Streptococcus pneumoniae serotype* 3, still more preferably a glycoconjugate of synthetic oligosaccharides of *Streptococcus pneumoniae serotype* 3. Most preferably, the at least one glycoconjugate of synthetic *Streptococcus pneumoniae* oligosaccharide is a glycoconjugate of synthetic oligosaccharides of *Streptococcus pneumoniae serotype* 3 having the formula **(III).**

The carrier protein is selected from diphtheria toxoid, detoxified pneumolysin, an immunogenic fragment of detoxified pneumolysin, pneumococcal surface protein A (PspA), an immunogenic fragment of pneumococcal surface protein A, and tetanus toxoid (TT). Preferably, the carrier protein is selected from CRM₁₉₇, detoxified pneumolysin, an immunogenic fragment of detoxified pneumolysin, pneumococcal surface protein A (PspA), an immunogenic fragment of pneumococcal surface protein A, and tetanus toxoid (TT); more preferably, the carrier protein is CRM₁₉₇.

The at least one adjuvant is selected from monophosphoryl lipid A (MPLA), CRX-524, 1*H*-imidazo[4,5-c]quinoline compounds, 8-oxoadenine compounds, a mixture of monophosphoryl lipid A and one of the 1*H*-imidazo[4,5-c]quinoline compounds, isolated bacterial RNA and cytosine-phosphate-guanosine(CpG) oligodeoxynucleotides; preferably, the at least one adjuvant is selected from monophosphoryl lipid A (MPLA), CRX-524, resiquimod, imiquimod, SM360320, GSK2245035, a mixture of monophosphoryl lipid A and resiquimod, isolated bacterial RNA and CPG7909; most preferably, the at least one adjuvant is selected from monophosphoryl lipid A (MPLA), resiquimod, a mixture of monophosphoryl lipid A and resiquimod, and isolated bacterial RNA.

The biocompatible and biodegradable polymer particles are preferably selected from the polylactic acid (PLA) particles, polyglycolic acid (PGA) particles, poly(lactic-co-glycolic acid) particles, PLGA/PEG co-polymers (PLGA-PEG) particles and tri-block PLGA/PEG/PLGA copolymers particles, and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm. Most preferably, the biocompatible and biodegradable polymer particles are poly(lactic-co-glycolic acid) particles.

At least four serotypes in form of glycoconjugates of *S. pneumoniae* capsular saccharides individually conjugated to a carrier protein are included in the vaccine of the present invention, e.g. 6B, 14, 19F and 23F. More preferably, at least 7 serotypes are included in the vaccine of the present invention, e.g. 4, 6B, 9V, 14, 18C, 19F and 23F.

Preferably, the vaccine of the present invention further comprises 10 or more, 11 or more, 12 or more, 13 or more, 14 or more, 15 or more, 16 or more, 17 or more, 19 or more, or 20 capsular saccharides from different *S. pneumoniae* serotypes and suitably conjugated to a carrier protein.

Preferably, the vaccine may be an 12-valent vaccine. For example, a 12-valent vaccine may comprise capsular saccharides from serotypes 1, 4, 5, 6A, 6B, 7F, 9V, 14, 18C, 19F and 23F, individually conjugated to a carrier protein, and a synthetic glycoconjugate of serotype 3 as disclosed herein.

Preferably, the vaccine may be an 13-valent or 14-valent vaccine. A 13 or 14-valent paediatric (infant) vaccine may also include the 12 valent formulation (described above) supplemented with serotypes 19A, or 22F, whereas a 14-valent elderly vaccine may include the 11 valent formulation supplemented with serotypes 19A and 22F, 8 and 12F, or 8 and 15, or 8 and 19A, or 8 and 22F, or 12F and 15, or 12F and 19A, or 12F and 22F, or 15 and 19A, or 15 and 22F.

A 14-valent vaccine may include preferably glycocojugates of oligosaccharides / capsular polysaccharides of the serotypes 1, 3, 4, 5, 6A, 6B, 7F, 9V, 14, 18C, 19A, 19F and 23F. Said 14-valent vaccines further contain one or more glycoconjugates of synthetic oligosaccharides of *S*. *pneumoniae serotypes* 1, 3, 4, and 5.

A 14-valent vaccine may also preferably include glycocojugates of oligosaccharides / capsular polysaccharides of the serotypes 1, 2, 3, 4, 5, 6A, 6B, 7F, 9V, 14, 18C, 19A, 19F and 23F.

Preferably, the vaccine may be a 15-valent vaccine.
A 15 -valent pediatric vaccine may include the 12 valent formulation described above supplemented with serotypes 3, 19A and 22F; serotypes 8, 19A and 22F; serotypes 12F, 19A and 22F; serotypes 15, 19A and 22F; serotypes 3, 8, 19A and 22F; serotypes 3, 12F, 19A and 22F; serotypes 3, 15, 19A and 22F.
A 15-valent vaccine may include preferably glycocojugates of oligosaccharides / capsular polysaccharides of the serotypes :
1, 3, 4, 5, 6A, 6B, 7F, 9V, 11A, 14, 18C, 19A, 19F, 22F and 23F; or
1, 2, 3, 4, 5, 6A, 6B, 7F, 9V, 12F, 14, 18C, 19A, 19F, and 23F; or
1, 2, 3, 4, 5, 6A, 6B, 7F, 9V, 14, 15B, 18C, 19A, 19F, and 23F.

Preferably, the vaccine may be a 17-valent vaccine. A 17 valent vaccine may include the 12 valent formulation described above supplemented with serotypes 3, 15B, 19A, 22F and 23F. A 16 valent vaccine may include the 12 valent formulation described above supplemented with serotypes 3, 15B, 19A, 22F and 33F.

Preferably, the vaccine may be a 18-valent vaccine. A 18-valent vaccine may include the 12 valent formulation described above supplemented with serotypes 3, 12F, 15B, 19A, 22F and 33F. A 18-valent vaccine may include preferably glycocojugates of oligosaccharides / capsular polysaccharides of the serotypes 1, 3, 4, 5, 6A, 6B, 7F, 9V, 12F, 14, 15B, 18C, 19A, 19F, 22F, 23F and 33F. (17 values).

Preferably, the vaccine may be a 20-valent vaccine. A 20 valent vaccine may include the 12 valent formulation described above supplemented with serotypes 8, 10A, 11A, 12F, 15B, 19A, 22F and 23F. A 20 valent vaccine may include the 12 valent formulation described above supplemented with serotypes 8, 10A, 11A, 12F, 15B, 19A, 22F and 33F. In an embodiment, the vaccine may be a 21-valent vaccine. A 21 valent vaccine may include the 12 valent formulation described above supplemented with serotypes 3, 8, 10A, 11A, 12F, 15B, 19A, 22F and 23F. A 21 valent vaccine may include the 12 valent formulation described above supplemented with serotypes 3, 8, 10A, 11A, 12F, 15B, 19A, 22F and 33F.

In an embodiment, the vaccine may be a 22-valent vaccine. Preferably, the vaccine may be a 22-valent, 23-valent, or 24-valent vaccine. Optionally, the vaccine may be a 25-valent vaccine.

Thus, the present invention is directed to a vaccine comprising
a) a glycoconjugate of synthetic oligosaccharides of *Streptococcus pneumoniae* serotype 3 and a carrier protein;
b) at least one adjuvant, wherein the at least one adjuvant is selected from agonists of toll-like receptors 1, 2, 4, 6, 7, 8, or 9,
c) one or more glycoconjugates of capsular polysaccharides from *S. pneumoniae* 1, 4, 5, 6A, 6B, 7F, 9V, 14, 18C, 19A, 19F and 23F individually conjugated to a carrier protein,
   wherein the at least one glycoconjugate and the at least one adjuvant are encapsulated in biocompatible and biodegradable polymer particles.

Preferably, the present invention is directed to a vaccine comprising
a) a glycoconjugate of synthetic oligosaccharides of *Streptococcus pneumoniae* serotype 3 and a carrier protein;
b) at least one adjuvant, wherein the at least one adjuvant is selected from agonists of toll-like receptors 1, 2, 4, 6, 7, 8, or 9,
c) at least one immunogenic component, wherein the at least one immunogenic component is selected from the group comprising or consisting of:
   detoxified pneumolysin (Ply), an immunogenic fragment of detoxified pneumolysin (Ply);
   a pneumococcal surface antigen A, an immunogenic fragment of a pneumococcal surface antigen A,
   a pneumococcal surface protein A, an immunogenic fragment of a pneumococcal surface protein A, and
   a glycosphingolipid.,
d) one or more glycoconjugates of capsular polysaccharides from *Streptococcus pneumoniae* individually conjugated to a carrier protein
   wherein the at least one glycoconjugate and the at least one adjuvant are encapsulated in biocompatible and biodegradable polymer particles.

More preferably, the present invention is directed to a vaccine comprising
a) a glycoconjugate of synthetic oligosaccharides of *Streptococcus pneumoniae* serotype 3 and a carrier protein;
b) at least one adjuvant, wherein the at least one adjuvant is selected from monophosphoryl lipid A (MPLA), resiquimod, a mixture of monophosphoryl lipid A and resiquimod, and isolated bacterial RNA,
c) at least one immunogenic component, wherein the at least one immunogenic component is selected from the group comprising or consisting of:
   detoxified pneumolysin (Ply), an immunogenic fragment of detoxified pneumolysin (Ply);
   a pneumococcal surface antigen A, an immunogenic fragment of a pneumococcal surface antigen A,
   a pneumococcal surface protein A, an immunogenic fragment of a pneumococcal surface protein A, and
   a glycosphingolipid.,
d) one or more glycoconjugates of capsular polysaccharides from *Streptococcus pneumoniae* individually conjugated to a carrier protein;
   wherein the at least one glycoconjugate and the at least one adjuvant are encapsulated in biocompatible and biodegradable polymer particles.

Thus, particular preferred vaccine of the present invention comprises
a) at least one glycoconjugate of synthetic oligosaccharides of *Streptococcus pneumoniae* serotype 3 having the formula **(III)**

   **[H-(C)_{c3}-(D-C)_{c2}-(D)_{c1}-O-L³-NH-W³]ₘ₃- carrier protein** **(III)**

   wherein
   C represents: 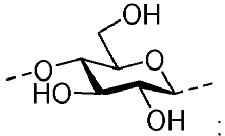
   D represents: 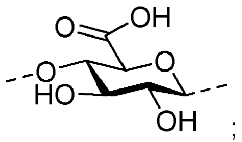
   c2 is an integer selected from 1, 2, 3, 4, 5, 6, 7, 8, 9 and 10;
   c1 and c3 represent independently of each other an integer 0 or 1;
   L³ represents a linker;
   m³ is an integer between 2 and 18;
   -W³- is selected from: 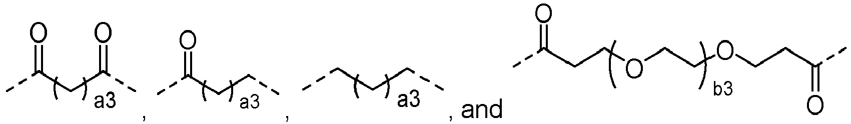
      a3 represents an integer from 1 to 10;
      b3 represents an integer from 1 to 4; and
      the carrier protein is CRM₁₉₇;
b) at least one adjuvant, wherein the at least one adjuvant is selected from monophosphoryl lipid A (MPLA), resiquimod, a mixture of monophosphoryl lipid A and resiquimod, and isolated bacterial RNA,
c) at least one immunogenic component, wherein the at least one immunogenic component is selected from the group comprising or consisting of:
   detoxified pneumolysin (Ply), an immunogenic fragment of detoxified pneumolysin (Ply);
   a pneumococcal surface antigen A,, an immunogenic fragment of a pneumococcal surface antigen A,
   a pneumococcal surface protein A, an immunogenic fragment of a pneumococcal surface protein A, and
   a glycosphingolipid.,
d) one or more glycoconjugates of capsular saccharides from *S. pneumoniae* serotypes 1, 4, 5, 6A, 6B, 7F, 9V, 14, 18C, 19A, 19F and 23F individually conjugated to a carrier protein CRM₁₉₇,
wherein the at least one glycoconjugate and the at least one adjuvant are encapsulated in biocompatible and biodegradable polymer particles, and
the biocompatible and biodegradable polymer particles are selected from the polylactic acid (PLA) particles, polyglycolic acid (PGA) particles, poly(lactic-co-glycolic acid) particles, PLGA/PEG co-polymers (PLGA-PEG) particles and tri-block PLGA/PEG/PLGA copolymers particles, and optionally the biocompatible and biodegradable polymer particles have an average diameter in a range of 100 nM to 10000 nM, preferably 100 nM to 5000 nM, more preferably 100 nM to 1000 nM, most preferably 150 to 500 nm.

### PREPARATION OF CONJUGATED CAPSULAR POLYSACCHARIDES

The capsular polysaccharides (CPS) from *S. pneumoniae* serotypes 1, 3, 4, 5, 6A, 6B, 7F, 9V, 14, 18C, 19A, 19F and 23F are either purchased from American Type Culture Collection (ATCC) or are isolated from bacteria and purified by known methods (see EP0072513A2) such as centrifugation, precipitation, ultra-filtration, and column chromatography. Further, they may be sized to some degree before forming conjugates with carrier proteins. Sizing of these full length capsular polysaccharides means that their size is reduced and it may be achieved by known methods (see for example EP497524 and EP497525), such as acid hydrolysis treatment, hydrogen peroxide treatment, sizing by emulsiflex® followed by a hydrogen peroxide treatment to generate oligosaccharide fragments or preferably by microfluidization. Sizing of polysaccharides is advantageous to filtering conjugated products due to the reduced viscosity of the sized polysaccharides. Sized polysaccharides have typically a molecular weight of 130 to 400 kDa depending on the serotype.

Each of the capsular polysaccharides from *S. pneumoniae* serotypes is conjugated to a carrier protein. Thus, each capsular polysaccharide from a *S. pneumoniae* serotype is covalently coupled or linked either directly or *via* a spacer to a carrier protein.

A spacer as used herein is preferably bifunctional. The spacer is optionally heterobifunctional or homobifunctional, having for example a reactive amino group and a reactive carboxylic acid group, 2 reactive amino groups or two reactive carboxylic acid groups. The spacer has for example between 4 and 20, 4 and 12, 5 and 10 carbon atoms. A possible spacer is adipic acid dihydrazide (ADH). Other spacers include B-propionamido, nitrophenyl-ethylamine, haloalkyl halides, glycosidic linkages, hexane diamine and 6-aminocaproic acid.

The above-described glycoconjugates of the capsular polysaccharide of *S. pneumoniae* can be obtained by conjugation of capsular polysaccharide to a carrier protein by using any known coupling technique. The conjugation method may rely on activation of the saccharide with 1-cyano-4-dimethylamino pyridinium tetrafluoroborate (CDAP) to form a cyanate ester. The activated saccharide may thus be coupled directly or *via* a spacer group to an amino group on the carrier protein. For example, the spacer could be cystamine or cysteamine to give a thiolated polysaccharide, which could be coupled to the carrier *via* a thioether linkage obtained after reaction with a maleimide-activated carrier protein (for example using GMBS) or a haloacetylated carrier protein (for example using iodoacetimide [e.g. ethyl iodoacetimide HCl] or N-succinimidyl bromoacetate or SIAB, or SIA, or SBAP). Preferably, the cyanate ester (optionally made by CDAP chemistry) is coupled with hexane diamine or ADH and the amino-derivatised saccharide is conjugated to the carrier protein using carbodiimide (e.g. EDAC or EDC) chemistry via a carboxyl group on the protein carrier. Such conjugation methods are described in WO 93/15760, WO 95/08348 and WO 96/29094.

Other suitable conjugation techniques rely on the use of carbodiimides, hydrazides, active esters, norborane, p-nitrobenzoic acid, *N*-hydroxysuccinimide, S-NHS (N-hydroxysulfosuccinimide), EDC, TSTU (2-succinimide-1,1,3,3-tetramethyluronium tetrafluoroborate) (WO 98/42721). Conjugation may involve a carbonyl linker which may be formed by reaction of a free hydroxyl group of the saccharide with CDI (1,1'-carbonyldiimidazole) followed by reaction with a protein to form a carbamate linkage. This may involve reduction of the anomeric terminus to a primary hydroxyl group, optional protection/deprotection of the primary hydroxyl group, reaction of the primary hydroxyl group with CDI to form a CDI carbamate intermediate and coupling the CDI carbamate intermediate with an amino group on a protein.

The conjugates can also be prepared by direct reductive amination methods as described in US 4673574. Other preparation methods are described in EP0161188, EP208375 and EP0477508.

A further method involves the coupling of a cyanogen bromide (or CDAP) activated saccharide derivatised with adipic acid dihydrazide (ADH) to the protein carrier by carbodiimide condensation, for example using EDAC.

In an embodiment, a hydroxyl group (preferably an activated hydroxyl group for example a hydroxyl group activated to make a cyanate ester [e.g. with CDAP]) on a saccharide is linked to an amino or carboxylic group on a protein either directly or indirectly (through a spacer). Where a spacer is present, a hydroxyl group on a saccharide is preferably linked to an amino group on a linker, for example by using CDAP conjugation. A further amino group in the linker for example ADH) may be conjugated to a carboxylic acid group on a protein, for example by using carbodiimide chemistry, for example by using EDAC. In an embodiment, the pneumococcal capsular saccharide(s) is conjugated to the spacer first before the spacer is conjugated to the carrier protein. Alternatively, the spacer may be conjugated to the carrier before conjugation to the saccharide.

A combination of techniques may also be used, with some conjugates being prepared by CDAP, and some by reductive amination.

In general the following types of chemical groups on a protein carrier can be used for conjugation:
- Carboxyl (for instance *via* aspartic acid or glutamic acid). In one embodiment this group is linked to amino groups on saccharides directly or to an amino group on a spacer with carbodiimide chemistry e.g. with EDAC.
- Amino group (for instance *via* lysine). In one embodiment this group is linked to carboxyl groups on saccharides directly or to a carboxyl group on a spacer with carbodiimide chemistry e.g. with EDAC. In another embodiment this group is linked to hydroxyl groups activated with CDAP or CNBr on saccharides directly or to such groups on a spacer; to saccharides or spacers having an aldehyde group; to saccharides or spacers having a succinimide ester group.
- Sulfhydryl (for instance *via* cysteine). In one embodiment this group is linked to a bromo or chloro acetylated saccharide or linker with maleimide chemistry. In one embodiment this group is activated/modified with bis diazobenzidine.
- Hydroxyl group (for instance *via* tyrosine). In one embodiment this group is activated/modified with bis diazobenzidine.
- Imidazolyl group (for instance *via* histidine). In one embodiment this group is activated/modified with bis diazobenzidine.
- Guanidyl group (for instance *via* arginine).
- Indolyl group (for instance *via* tryptophan).

On a saccharide, in general the following groups can be used for conjugation: OH, COOH or NH₂. Aldehyde groups can be generated after different treatments known in the art such as: periodate, acid hydrolysis, hydrogen peroxide, etc.

Examples of direct coupling approaches include, but are not limited to:
CPS-OH + CNBr or CDAP → CPS-cyanate ester + NH₂-carrier protein → conjugate;
CPS-aldehyde + NH₂-carrier protein → Schiff base + NaCNBH₃ → conjugate; CPS-COOH + NH₂-carrier protein + EDAC → conjugate;
CPS-NH₂ + carrier protein-COOH + EDAC → conjugate;

Indirect coupling *via* spacer approaches include, but are not limited to:
CPS-OH + CNBr or CDAP → CPS-cyanate ester + NH₂-NH₂ → CPS-NH₂ + COOH-carrier protein + EDAC → conjugate;
CPS-OH + CNBr or CDAP → CPS-cyanate ester + NH₂-SH → CPS-SH + SH-carrier protein (native protein with an exposed cysteine or obtained after modification of amino groups of the protein by SPDP for instance) → CPS-S-S-carrier protein;
CPS-OH + CNBr or CDAP → CPS-cyanate ester + NH₂-SH → CPS-SH + maleimide-carrier protein (modification of amino groups) → conjugate;
CPS-OH + CNBr or CDAP → CPS-cyanate ester + NH₂-SH → CPS-SH + haloacetylated-carrier protein → conjugate;
CPS-COOH + EDAC + NH₂-NH₂ → CPS-NH₂ + EDAC + COOH-carrier protein → conjugate;
CPS-COOH + EDAC+ NH₂-SH → CPS-SH + SH-carrier protein (native protein with an exposed cysteine or obtained after modification of amino groups of the protein by SPDP for instance) → saccharide-S-S-carrier protein;
CPS-COOH + EDAC+ NH₂-SH → CPS-SH + maleimide-carrier protein (modification of amino groups) → conjugate;
CPS-COOH + EDAC + NH₂-SH → CPS-SH + haloacetylated-carrier protein → conjugate;
CPS-aldehyde + NH₂-NH₂ → CPS-NH₂ + EDAC + carrier protein-COOH → conjugate.

In a preferred embodiment, CDAP (1-cyano-dimethylaminopyridinium tetrafluoroborate) cyanylating reagent is used for the synthesis of capsular polysaccharide-protein conjugates. This coupling method avoids hydrolysis of the alkaline sensitive polysaccharides and allows direct coupling to the carrier protein. The polysaccharide is dissolved in water or a saline solution. CDAP is dissolved in acetonitrile and added immediately to the polysaccharide solution. The CDAP reacts with the hydroxyl groups of the polysaccharide to form a cyanate ester in an activation step. Afterwards, the carrier protein is added. Amino groups of lysine react with the activated polysaccharide to form an isourea covalent linkage. After the conjugation reaction, a large excess of glycine is added to quench residual activated functions. The formed conjugate is then passed through a gel permeation to remove unreacted carrier protein and residual reagents.

Another preferred conjugation method is the reductive amination, wherein the capsular polysaccharide is oxidized with sodium periodate in an activation step and subsequently brought to reaction with the amino group of a carrier protein in the presence of sodium borohydride or sodium cyanoborohydride. The reductive amination can be performed in aqueous media or in DMSO. The crude conjugate is then passed through a HA or DEAE column and filtered sterile

### Type of covalent conjugation

In an embodiment, the pneumococcal saccharide of the present invention is covalently linked (either directly or through a linker) to an amino acid residue of a carrier protein. In an embodiment, the pneumococcal saccharide is covalently linked to a carrier protein through a chemical linkage obtainable using a chemical conjugation method, optionally selected from the group consisting of carbodiimide chemistry, reductive animation, cyanylation chemistry (for example CDAP chemistry), maleimide chemistry, hydrazide chemistry, ester chemistry, and N-hydroxysuccinimide chemistry either directly or via a linker. As used herein, the term **"directly linked"** means that the two entities are connected via a chemical bond, preferably a covalent bond. As used herein, the term **"indirectly linked"** means that the two entities are connected via a linking moiety (as opposed to a direct covalent bond). In certain embodiments the linker is adipic acid dihydrazide. In an embodiment, the chemical conjugation method is selected from the group consisting of carbodiimide chemistry, reductive animation, cyanylation chemistry (for example CDAP chemistry), maleimide chemistry, hydrazide chemistry, ester chemistry, and Nhydroysuccinimide chemistry. Conjugates can be prepared by direct reductive amination methods as described in, US20071 0 184072 (Hausdorff) US 4365170 (Jennings) and US 4673574 (Anderson). Other methods are described in EP-0-161-188, EP-208375 and EP-0-477508. The conjugation method may alternatively rely on activation of the pneumococcal saccharide with 1-cyano-4-dimethylamino pyridinium tetrafluoroborate (CDAP) to form a cyanate ester. Such conjugates are described in PCT published application WO 93/15760 Uniformed Services University and WO 95/08348 and WO 96/29094. See also Chu C. et al. Infect. Immunity, 1983, pages 245-256.

In general the following types of chemical groups on a carrier protein can be used for conjugation:
A) Carboxyl (for instance via aspartic acid or glutamic acid). In one embodiment this group is linked to amino groups on saccharides directly or to an amino group on a linker with carbodiimide chemistry e.g. with EDAC.
B) Amino group (for instance via lysine). In one embodiment this group is linked to carboxyl groups on saccharides directly or to a carboxyl group on a linker with carbodiimide chemistry e.g. with EDAC. In another embodiment this group is linked to hydroxyl groups activated with CDAP or CNBr on saccharides directly or to such groups on a linker; to saccharides or linkers having an aldehyde group; to saccharides or linkers having a succinimide ester group.
C) Sulphydryl (for instance via cysteine). In one embodiment this group is linked to a bromo or chloro acetylated saccharide or linker with maleimide chemistry. In one embodiment this group is activated/modified with bis diazobenzidine.
D) Hydroxyl group (for instance via tyrosine). In one embodiment this group is activated/modified with bis diazobenzidine.
E) Imidazolyl group (for instance via histidine). In one embodiment this group is activated/modified with bis diazobenzidine.
F) Guanidyl group (for instance via arginine).
G) Indolyl group (for instance via tryptophan). On a saccharide, in general the following groups can be used for a coupling: OH, COOH or NH₂. Aldehyde groups can be generated after different treatments such as: periodate, acid hydrolysis, hydrogen peroxide, etc.

### Uses

An adjuvant is employed in vaccines to help to create a stronger and more efficient immune response and provide protection from the disease. The adjuvant is an essential part of a vaccine. Several adjuvants characterized by various mechanisms and activation of different immune profiles (cell-mediated or humoral and T-helper 1 or T-helper 2 (Tₕ1/Tₕ2) immune responses) are currently used.

The effect of different adjuvant formulations on the antibody response upon vaccination with ST3-tetrasaccharide CRM₁₉₇ conjugate vaccine was tested in examples 4 and 5 of the present disclosure. In order to improve the immunogenicity of semi-synthetic ST3-glycoconjugates, different adjuvants were added and incorporated into biodegradable poly(lactic-co-glycolid-acid) (PLGA) particles. This approach allowed for the physical combination of adjuvants and antigens, and it ensured the targeted uptake of the vaccine by professional phagocytes.

Screening experiments in mice showed promising results for agonists of TLR7/8, namely resimiquimod (R848) and bacterial RNA, as well as MPLA. It was found that agonists of TLR7/8, namely resimiquimod (R848) and bacterial RNA, as well as MPLA, incorporated into biodegradable PLGA particles were able to increase the immune response against ST3-tetrasaccharide and ST3 capsular polysaccharide **(****Figures 6, 7****)**. Morever, the *in vitro* opsonophagocytic killing assay showed that the serum of immunized mice were able to kill *Streptococcus pneumoniae* bacteria *in vitro,* and the best results were achieved with R848 and PLGA particles**(****Figure 8****)**.

Therefore, the present invention is directed to a vaccine comprising
a) at least one glycoconjugate of synthetic *Streptococcus pneumoniae* oligosaccharides and a carrier protein;
b) at least one adjuvant, wherein the at least one adjuvant is selected from agonists of toll-like receptors 1, 2, 4, 6, 7, 8, or 9,
   wherein the at least one glycoconjugate and the at least one adjuvant are encapsulated in biocompatible and biodegradable polymer particles,
for use in the prevention and/or treatment of a disease caused by *Streptococcus pneumoniae* in a human or an animal host.

Preferably, the vaccine of the present invention is useful in the prevention and/or treatment of a disease caused by *Streptococcus pneumoniae* serotype 3.

More preferably, the vaccine of the present invention is useful in the prevention and/or treatment of a disease caused by *Streptococcus pneumoniae,* wherein the disease caused by Streptococcus pneumoniae includes pneumonia, meningitis, otitis media, bacteremia and acute exacerbation of chronic bronchitis, invasive pneumococcal disease, exacerbation of chronic obstructive pulmonary disease, sinusitis, arthritis and conjunctivitis.

Preferably, in said vaccine of invention for the abovementioned use, the biocompatible and biodegradable polymer particles are selected from polylactic acid particles, polyglycolic acid (PGA) particles, poly(lactic-co-glycolic acid) (PLGA) particles, PLGA/PEG co-polymers (PLGA-PEG) particles and tri-block PLGA/PEG/PLGA copolymers particles. More preferably, the biocompatible and biodegradable polymer particles are poly(lactic-co-glycolic acid) particles.

Preferably, in said vaccine of invention for the abovementioned use, the carrier protein is selected from CRM₁₉₇, detoxified pneumolysin, or an immunogenic fragment of detoxified pneumolysin, pneumococcal surface protein A (PspA), or an immunogenic fragment of pneumococcal surface protein A, and tetanus toxoid (TT); more preferably the carrier protein is CRM₁₉₇.

Preferably, in said vaccine of invention for the abovementioned use, the at least one adjuvant is monophosphoryl lipid A, an 1*H*-imidazo[4,5-c]quinoline compound, preferably resiquimod, a mixture of monophosphoryl lipid A and resiquimod, or isolated bacterial RNA.

More preferably, a ratio of the at least one glycoconjugate and the at least one adjuvant is comprised between 1 : 1 and 1 : 100 (w/w), 1 : 1 and 1 : 75, 1 : 1 and 1 : 50, 1:1 and 1 : 25, 1 : 1 and 1 : 10:, more preferably between 1 : 1 and 1 : 100.

Preferably, in said vaccine of invention for the above mentioned use, the synthetic *Streptococcus pneumoniae* oligosaccharide is selected from the group of serotypes comprising serotypes 1, 2, 3, 4, 5, and 8, more preferred the synthetic *Streptococcus pneumoniae* oligosaccharide is of *Streptococcus pneumoniae* serotype 3.

More preferably in said vaccine of invention for the abovementioned use, the at least one glycoconjugate has the general formula **(IIII)**

**[H-(C)_{c3}-(D-C)_{c2}-(D)_{c1}-O-L³-NH-W³]ₘ₃- carrier protein** **(III)**

wherein
C represents: 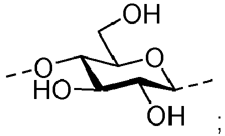
D represents: 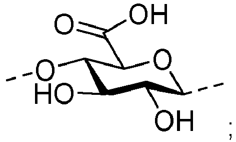
c2 is an integer selected from 1, 2, 3, 4, 5, 6, 7, 8, 9 and 10;
c1 and c3 represent independently of each other an integer 0 or 1;
L³ represents a linker;
m³ is an integer between 2 and 18;
-W³- is selected from: 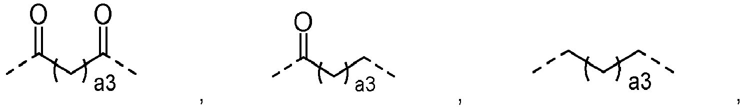 and 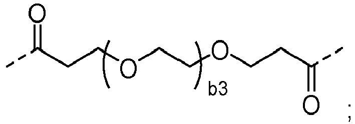
   a3 represents an integer from 1 to 10;
   b3 represents an integer from 1 to 4; and
   carrier protein is selected from CRM₁₉₇, detoxified pneumolysin, or an immunogenic fragment of detoxified pneumolysin, pneumococcal surface protein A (PspA), or an immunogenic fragment of pneumococcal surface protein A, and tetanus toxoid (TT); more preferably the carrier protein is CRM₁₉₇.

In particular, in said vaccine of invention for the abovementioned use, the at least one glycoconjugate has the following formula **(IIIa)** 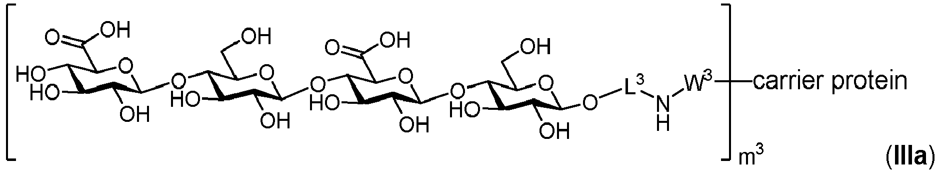
wherein **m³** represents an integer from 3 to 12;
**L³** represents -CH₂CH₂-;
**W³** represents -COCH₂CH₂CH₂CH₂CO-, or -COCH₂CH₂CH₂CH₂CH₂; carrier protein is selected from CRM₁₉₇, detoxified pneumolysin, or an immunogenic fragment of detoxified pneumolysin, pneumococcal surface protein A (PspA), or an immunogenic fragment of pneumococcal surface protein A, and tetanus toxoid (TT); more preferably the carrier protein is CRM₁₉₇; and
the at least one adjuvant is selected from monophosphoryl lipid A, resiquimod, a mixture of monophosphoryl lipid A and resiquimod, or isolated bacterial RNA.

In addition, for the above-mentioned medical use, the vaccine of the present invention further comprises at least one immunogenic component, wherein the at least one immunogenic component is selected from the group consisting of:
detoxified pneumolysin (Ply), or an immunogenic fragment thereof;
a pneumococcal surface antigen A, or an immunogenic fragment thereof,
a pneumococcal surface protein A, or an immunogenic fragment thereof, and a glycosphingolipid.

In particular case, for the above-mentioned medical use, the vaccine of the present invention further comprises one or more glycoconjugates of capsular polysaccharides from *Streptococcus pneumoniae* serotypes 1, 3, 4, 5, 6A, 6B, 7F, 9V, 14, 18C, 19A, 19F and 23F individually conjugated to CRM₁₉₇ carrier protein.

Said vaccine includes a nasal formulation, preferably nasal drops, nasal powder, a nasal gel or a nasal spray.

A physiologically acceptable vehicle may include non-toxic levels of alcohols and salts, 5% dextrose or other sugars, saline, and other pharmaceutically acceptable excipients, and any combination of any of these solvents. Such excipients are well known and described. Preferably used physiologically acceptable **vehicles** are water, buffered saline, succinic acid, polysorbate 80, polyols (e.g., glycerol, propylene glycol, liquid polyethylene glycol) and dextrose solutions.

Vaccines are preferably in aqueous form, particularly at the point of administration, but they can also be presented in non-aqueous liquid forms or in dried forms e.g. as gelatin capsules, or as lyophilisates, etc. Vaccines may include one or more preservatives, such as thiomersal or 2-phenoxyethanol. Mercury-free compositions are preferred, and preservative-free vaccines can be prepared.

Vaccines may include a physiological salt, such as a sodium salt e.g. to control tonicity. Sodium chloride (NaCl) is typical and may be present at between 1 and 20 mg/ml. Other salts that may be present include potassium chloride, potassium dihydrogen phosphate, disodium phosphate dehydrate, magnesium chloride, calcium chloride, etc.

Vaccines can have an osmolality of between 200 mOsm/kg and 400 mOsm/kg.
Vaccines may include compounds (with or without an insoluble metal salt) in plain water (e.g. Water for Injection, w.f.i.), but will usually include one or more buffers. Typical buffers include: a phosphate buffer; a Tris buffer; a borate buffer; a succinate buffer; a histidine buffer (particularly with an aluminium hydroxide adjuvant); or a citrate buffer. Buffer salts will typically be included in the 5 - 20 mM range. Vaccines typically have a pH between 5.0 and 9.5 e.g. between 6.0 and 8.0. Vaccines are preferably sterile and gluten free.

Vaccines are suitable for administration to animal (and, in particular, human) patients, and thus, include both human and veterinary uses. They may be used in a method of raising an immune response in a patient, comprising the step of administering the composition to the patient. The vaccines of the present invention may be administered before a subject is exposed to a *Streptococcus pneumoniae* and/or after a subject is exposed to a *Streptococcus pneumoniae.*

Vaccines may be prepared in unit dose form. In some embodiments a unit dose may have a volume of between 0.1 - 1.0 mL e.g. about 0.5 mL. The invention also provides a delivery device (e.g. syringe, nebulizer, sprayer, inhaler, dermal patch, etc.) containing a vaccine of the invention e.g. containing a unit dose. This device can be used to administer the composition to a vertebrate subject.
The invention also provides a sterile container (e.g. a vial) containing a vaccine of the invention e.g. containing a unit dose.
The invention also provides a unit dose of a vaccine of the invention.
The invention also provides a hermetically sealed container containing a vaccine of the invention. Suitable containers include e.g. a vial.

Vaccines of the invention may be prepared in various forms. For example, the vaccines may be prepared as injectables, either as liquid solutions or suspensions. Solid forms suitable for solution in, or suspension in, liquid vehicles prior to injection can also be prepared (e.g. a lyophilized composition or a spray-freeze dried composition). The vaccine may be prepared for topical administration e.g. as an ointment, cream or powder. The vaccine may be prepared for oral administration e.g. as a tablet or capsule, as a spray, or as a syrup (optionally flavored). The vaccine may be prepared for pulmonary administration e.g. by an inhaler, using a fine powder or a spray. The vaccine may be prepared as a suppository. The vaccine may be prepared for nasal, aural or ocular administration e.g. as a spray or drops. Injectables for intramuscular administration are typical.

Techniques for the formulation and administration of the vaccine of the present invention may be found in "Remington's Pharmaceutical Sciences" Mack Publishing Co., Easton PA.

The **amount of conjugate** in each vaccine dose is selected as an amount that induces an immunoprotective response without significant, adverse effects. Such amount can vary depending upon the *S*. *pneumoniae* serotype. Preferably, each dose will comprise 0.1 to 100 µg of each synthetic oligosaccharide or capsular polysaccharide, more preferably 0.1 to 10 µg, preferably from 0.1 to 5 µg, more preferably from 0.4 to 5 µg.

Said vaccine may be prepared in the form of a suspension or may be lyophilized. The suspension form may be stored frozen. In the lyophilized form, it is preferable to add one or more stabilizers. Optionally, one or more adjuvants may be added as well. Any conventional stabilizers and adjuvants may be included in a vaccine according to this invention.
Vaccination can be performed at any age.
The vaccine can also be administered in form of its pharmaceutically active salt optionally using substantially nontoxic pharmaceutically acceptable carrier, excipients, adjuvants or diluents. The vaccine of the present invention is prepared in a conventional solid or liquid carrier or diluents and a conventional pharmaceutically-made adjuvant at suitable dosage level in a known way. The preferred preparations and formulations are in administrable form, which is suitable for oral application. These administrable forms, for example, include pills, tablets, film tablets, coated tablets, capsules, powders and deposits.

The inventive vaccine may be administered by any appropriate means, including but not limited to inhalation; injection (intravenous, intraperitoneal, intramuscular, subcutaneous); by absorption through epithelial or mucocutaneous linings (oral mucosa, rectal and vaginal epithelial linings, nasopharyngial mucosa, intestinal mucosa); orally, rectally, transdermally, topically, intradermally, intragastrically, intracutaneously, intravaginally, intravasally, intranasally, intrabuccally, percutaneously, sublingually, or any other means available within the pharmaceutical arts.

The vaccine of the present invention will typically be administered in admixture with suitable carrier materials suitably selected with respect to the intended form of administration, i.e. oral tablets, capsules (either solid-filled, semi-solid filled or liquid filled), powders for constitution, oral gels, elixirs, dispersible granules, syrups, suspensions, and the like, and consistent with conventional pharmaceutical practices. Moreover, when desired or needed, suitable binders, lubricants, disintegrating agents and coloring agents may also be incorporated in the mixture.

Suitable binders include starch, gelatin, natural sugars, corn sweeteners, natural and synthetic gums such as acacia, sodium alginate, carboxymethyl-cellulose, polyethylene glycol and waxes. Among the lubricants that may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate. Some of the terms noted above, namely disintegrants, diluents, lubricants, binders and the like, are discussed in more detail below.

Additionally, the vaccine of the present invention may be formulated in sustained release form to provide the rate controlled release of any one or more of the components or active ingredients to optimize the therapeutic effects. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Liquid form preparations include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier such as inert compressed gas, e.g. nitrogen.

For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidifies.

Also included are solid form preparations that are intended to be converted, shortly before use, to liquid form preparations for either oral or parenteral administration. Such liquid forms include solutions, suspensions and emulsions.

The vaccine of the present invention may also be deliverable transdermally. The transdermal compositions may take the form of creams, lotions, aerosols and/or emulsions and can be included in a transdermal patch of the matrix or reservoir type as are conventional in the art for this purpose.

The term capsule refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.

Tablet means compressed or molded solid dosage form containing the active ingredients with suitable diluents. The tablet can be prepared by compression of mixtures or granulations obtained by wet granulation, dry granulation or by compaction well known to a person skilled in the art.

Oral gels refer to the active ingredients dispersed or solubilized in a hydrophilic semi-solid matrix.

Powders for constitution refer to powder blends containing the active ingredients and suitable diluents which can be suspended in water or juices.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluents in the composition can range from about 5 to about 95% by weight of the total composition, preferably from about 25 to about 75%, more preferably from about 30 to about 60% by weight, and most preferably from about 40 to 50% by weight.

The term disintegrants refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 1 to about 40% by weight of the composition, preferably 2 to about 30% by weight of the composition, more preferably from about 3 to 20% by weight of the composition, and most preferably from about 5 to about 10% by weight.

Binders characterize substances that bind or "glue" powders together and make them cohesive by forming granules, thus serving as the "adhesive" in the formulation. Binders add cohesive strength already available in the diluents or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropyl-methylcellulose; polyvinylpyrrolidone; and inorganics such as magnesium aluminum silicate. The amount of binder in the composition can range from about 1 to 30% by weight of the composition, preferably from about 2 to about 20% by weight of the composition, more preferably from about 3 to about 10% by weight, even more preferably from about 3 to about 6% by weight.

Lubricant refers to a substance added to the dosage form to enable the tablet, granules, etc. after it has been compressed, to release from the mold or die by reducing friction or wear. Suitable lubricants include metallic stearates such as magnesium stearate, calcium stearate or potassium stearate; stearic acid; high melting point waxes; and water soluble lubricants such as sodium chloride, sodium benzoate, sodium acetate, sodium oleate, polyethylene glycols and D, L-leucine. Lubricants are usually added at the very last step before compression, since they must be present on the surfaces of the granules and in between them and the parts of the tablet press. The amount of lubricant in the composition can range from about 0.05 to about 15% by weight of the composition, preferably 0.2 to about 5% by weight of the composition, more preferably from about 0.3 to about 3%, and most preferably from about 0.3 to about 1.5% by weight of the composition.

Glidents are materials that prevent caking and improve the flow characteristics of granulations, so that flow is smooth and uniform. Suitable glidents include silicon dioxide and talc. The amount of glident in the composition can range from about 0.01 to 10% by weight of the composition, preferably 0.1% to about 7% by weight of the total composition, more preferably from about 0.2 to 5% by weight, and most preferably from about 0.5 to about 2% by weight.

Coloring agents are excipients that provide coloration to the composition or the dosage form. Such excipients can include food grade dyes and food grade dyes adsorbed onto a suitable adsorbent such as clay or aluminum oxide. The amount of the colouring agent can vary from about 0.01 to 10% by weight of the composition, preferably from about 0.05 to 6% by weight, more preferably from about 0.1 to about 4% by weight of the composition, and most preferably from about 0.1 to about 1%.

Techniques for the formulation and administration of the vaccine of the present invention may be found in "Remington's Pharmaceutical Sciences" Mack Publishing Co., Easton PA.

A therapeutically effective dosage of a vaccine of the present invention refers to that amount of said conjugate that results in an at least a partial immunization against a disease. Toxicity and therapeutic efficacy of such conjugates can be determined by standard pharmaceutical, pharmacological, and toxicological procedures in cell cultures or experimental animals. The dose ratio between toxic and therapeutic effect is the therapeutic index. The actual amount of the composition administered will be dependent on the subject being treated, on the subject's weight, the severity of the affliction, the manner of administration and the judgement of the prescribing physician.

The inventive vaccine comprises an effective amount of an adjuvant i.e. an amount which, when administered to an individual, either in a single dose or as part of a series, is effective for enhancing the immune response to a co-administered *S*. *pneumoniae* antigen or. a *S*. *pneumoniae* conjugate. This amount can vary depending upon the health and physical condition of the individual to be treated, age, the taxonomic group of individual to be treated (e.g. non-human primate, primate, etc.), the capacity of the individual's immune system to synthesize antibodies, the degree of protection desired, the formulation of the vaccine, the treating doctor's assessment of the medical situation, and other relevant factors. The amount will fall in a relatively broad range that can be determined through routine trials.

The vaccine of the present invention comprises an immunologically effective amount of the pneumococcal saccharide conjugate of the invention, as well as any other components. By "immunologically effective amount", it is meant that the administration of that amount to an individual, either as a single dose or as part of a series is effective for treatment or prevention. This amount varies depending on the health and physical condition of the individual to be treated, age, the degree of protection desired, the formulation of the vaccine and other relevant factors. It is expected that the amount will fall in a relatively broad range that can be determined through routine trials. In an embodiment, the vaccine of the invention further comprises a pharmaceutically acceptable excipient, carrier or diluent.

As used herein, the term "effective amount" in the context of administering a therapy (e.g. an immunogenic composition or vaccine of the invention) to a subject refers to the amount of a therapy which has a prophylactic and/or therapeutic effect(s). In certain embodiments, an "effective amount" refers to the amount of a therapy which is sufficient to achieve one, two, three, four, or more of the following effects: (i) reduce or ameliorate the severity of a bacterial infection or symptom associated therewith; (ii) reduce the duration of a bacterial infection or symptom associated therewith; (iii) prevent the progression of a bacterial infection or symptom associated therewith; (iv) cause regression of a bacterial infection or symptom associated therewith; (v) prevent the development or onset of a bacterial infection, or symptom associated therewith; (vi) prevent the recurrence of a bacterial infection or symptom associated therewith; (vii) reduce organ failure associated with a bacterial infection; (viii) reduce hospitalization of a subject having a bacterial infection; (ix) reduce hospitalization length of a subject having a bacterial infection; (x) increase the survival of a subject with a bacterial infection; (xi) eliminate a bacterial infection in a subject; (xii) inhibit or reduce a bacterial replication in a subject; and/or (xiii) enhance or improve the prophylactic or therapeutic effect(s) of another therapy.

As used herein, the term "subject" refers to an animal, in particular a mammal such as a primate (e.g. human).

Pharmaceutically acceptable excipients and carriers can be selected by those of skill in the art. For example, the pharmaceutically acceptable excipient or carrier can include a buffer, such as Tris (trimethamine), phosphate (e.g. sodium phosphate), acetate, borate (e.g. sodium borate), citrate, glycine, histidine and succinate (e.g. sodium succinate), suitably sodium chloride, histidine, sodium phosphate or sodium succinate. The pharmaceutically acceptable excipient may include a salt, for example sodium chloride, potassium chloride or magnesium chloride. Optionally, the pharmaceutically acceptable excipient contains at least one component that stabilizes solubility and/or stability. Examples of solubilizing/stabilizing agents include detergents, for example, laurel sarcosine and/or polysorbate (e.g. TWEEN 80 (Polysorbate-80)). Examples of stabilizing agents also include poloxamer (e.g. poloxamer 124, poloxamer 188, poloxamer 237, poloxamer 338 and poloxamer 407). The pharmaceutically acceptable excipient may include a non-ionic surfactant, for example polyoxyethylene sorbitan fatty acid esters, TWEEN 80 (Polysorbate-80), TWEEN 60 (Polysorbate-60), TWEEN 40 (Polysorbate-40) and TWEEN 20 (Polysorbate-20), or polyoxyethylene alkyl ethers (suitably polysorbate- 80). Alternative solubilizing/stabilizing agents include arginine, and glass forming polyols (such as sucrose, trehalose and the like). The pharmaceutically excipient may be a preservative, for example phenol, 2-phenoxyethanol, or thiomersal. Other pharmaceutically acceptable excipients include sugars (e.g. lactose, sucrose), and proteins (e.g. gelatine and albumin). Pharmaceutically acceptable carriers include water, saline solutions, aqueous dextrose and glycerol solutions. Numerous pharmaceutically acceptable excipients and carriers are described, for example, in Remington's Pharmaceutical Sciences, by E. W. Martin, Mack Publishing Co. Easton, PA, 5th Edition (975). Immunogenic compositions of the invention may also contain diluents such as water, saline, glycerol etc. Additionally, auxiliary substances, such as wetting or emulsifying agents, pH buffering substances, polyols and the like may be present. In an embodiment, the immunogenic composition of the invention additionally comprises one or more buffers, e.g. phosphate buffer and/or sucrose phosphate glutamate buffer. In other embodiments, the immunogenic composition of the invention does not comprise a buffer. In an embodiment, the immunogenic composition of the invention additionally comprises one or more salts, e.g. sodium chloride, calcium chloride, sodium phosphate, monosodium glutamate, and aluminum salts (e.g. aluminum hydroxide, aluminum phosphate, alum (potassium aluminum sulfate), or a mixture of such aluminum salts). In other embodiments, the immunogenic composition of the invention does not comprise a salt.

Another aspect of the present invention relates to pharmaceutical formulations and pharmaceutical compositions containing the vaccine as an active ingredient, together with at least one pharmaceutically acceptable carrier, excipient, solvent and/or diluents.

Further preferred, the pharmaceutical composition is formulated in the form of a lyophilisate or liquid buffer solution.

### Synthesis of S. pneumoniae serotype 2 conjugates of general formula (II)

The conjugates of general formula **II**

**[B*-A_{y+3}-A_{y+2}-A_{y+1}-A_{y}-O-L²-NH-W²]ₘ₂-carrier protein** **(II)**

wherein
y is an integer selected from 1, 2, 3 and 4;
A₁=A₅= 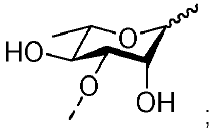
A₂=A₆= 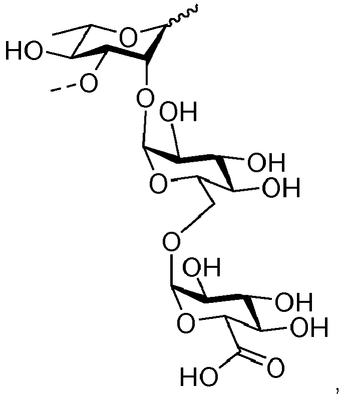
A₃=A₇= 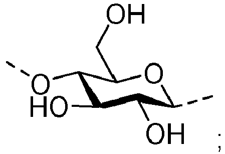
A₄= 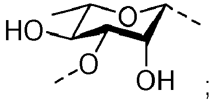
B*- represents: H-, H-A_{y}-, H-A_{y+1}-A_{y}-, H-A_{y+2}-A_{y+1}-A_{y}- or H-A_{y+3}-A_{y+2}-A_{y+1}-A_{y}-;
L² represents a linker and is defined as above;
m² is comprised between about 2 and about 18;
-W²- is selected from: 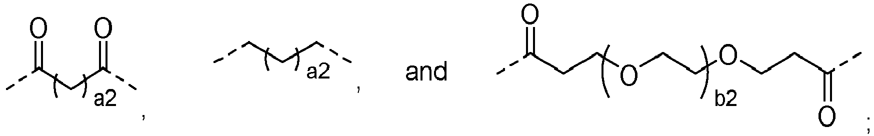
   a2 represents an integer from 1 to 10;
   b2 represents an integer from 1 to 4; and
   carrier protein has the meaning of carrier protein as defined above;
   can be obtained starting from a saccharide of general formula **2*** and carrier protein using coupling methods well known to the skilled person (see **Scheme 1**).

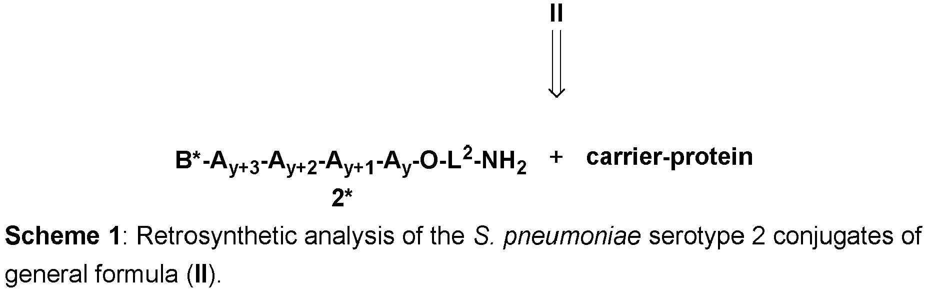

Such coupling methods include, for example, treatment of the saccharide **2*** with a commercially available bifunctional spacer (Thermo Fischer, Sigma Aldrich) such as disuccinimidyl adipate (DSA), bis-(4-nitrophenyl) adipate, bis-(4-nitrophenyl)succinate, disuccinimidyl glutarate (DSG), bis(sulfosuccinimidyl)disuccinimidyl glutarate bis(sulfosuccinimidyl)disuccinimidyl suberate (DSS), bis(sulfosuccinimidyl)suberate (BS³), sebacic acid bis(N-succinimidyl) ester bis(succinimidyl)penta(ethyleneglycol), bis(succinimidyl) tetra(ethyleneglycol), in presence of a base, such as triethylamine, pyridine, followed by reaction of the resulting construct with the carrier protein. Alternatively, saccharide **2*** can be reacted with a suitable dialdehyde, such as glutaraldehyde, under reductive amination conditions, and the resulting construct can be subsequently treated with the carrier protein under reductive amination conditions.

A saccharide of general formula **2*** can be efficiently assembled starting from building blocks **3*, 4*, 5*, 6*, 7*,** as well as amino-alcohol linker **8*** as starting material (**Scheme 2**) and using glycosylation and deprotection method well known to the skilled person in the art. Building block **6*** can be prepared from known building block **9*** (Bundle D. R. et al. ACS Chem. Biol. 2012, 7, 1754). Conversion of building block **9*** to building block **6*** involves installing the Fmoc protecting group at 4^{th} position of the glucoside by reaction with FmocCl and pyridine in dichloromethane, followed by treatment with CAN in a mixture of acetonitrile and water, and finally installation of the trichloroacetimidate leaving group at the anomeric position by treatment with CCl₃CN and DBU in dichloromethane at room temperature.

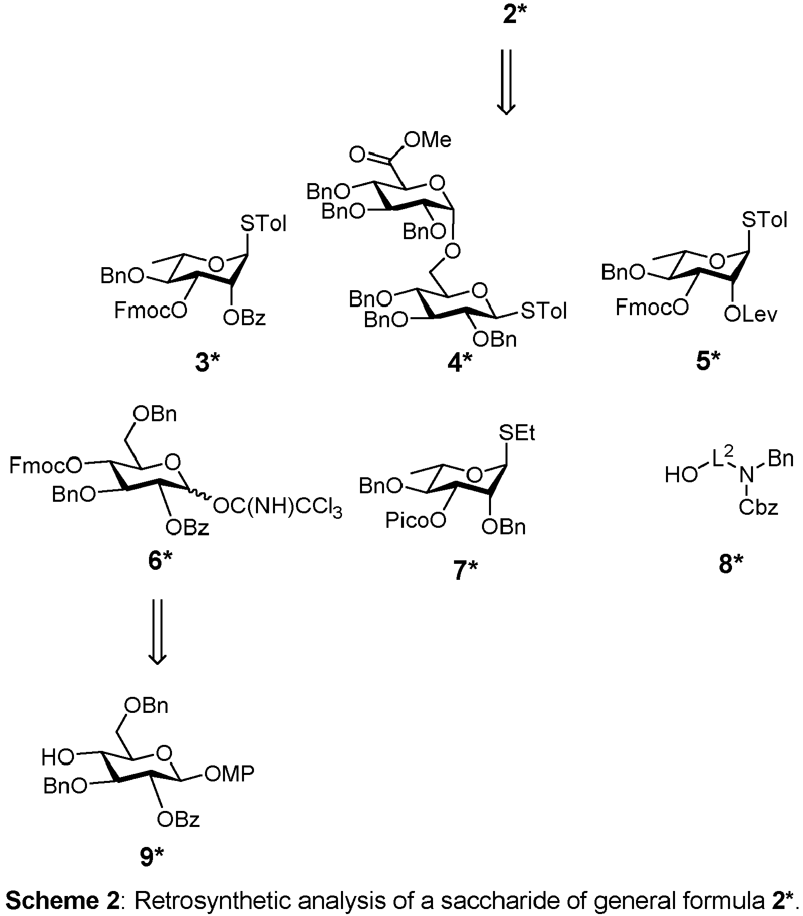

### Synthesis of S. pneumoniae serotype 3 conjugates of general formula (III)

The conjugates of general formula **III**

**[H-(C)_{c3}-(D-C)_{c2}-(D)_{c1}-O-L³-NH-W³]ₘ₃-carrier** protein **(lll)**

wherein C, D, c1, c2, c3, L3, m3, -W³- and carrier protein have the meanings defined herein;
can be generated starting from saccharide **10*** and carrier protein using coupling methods well known to the skilled person.

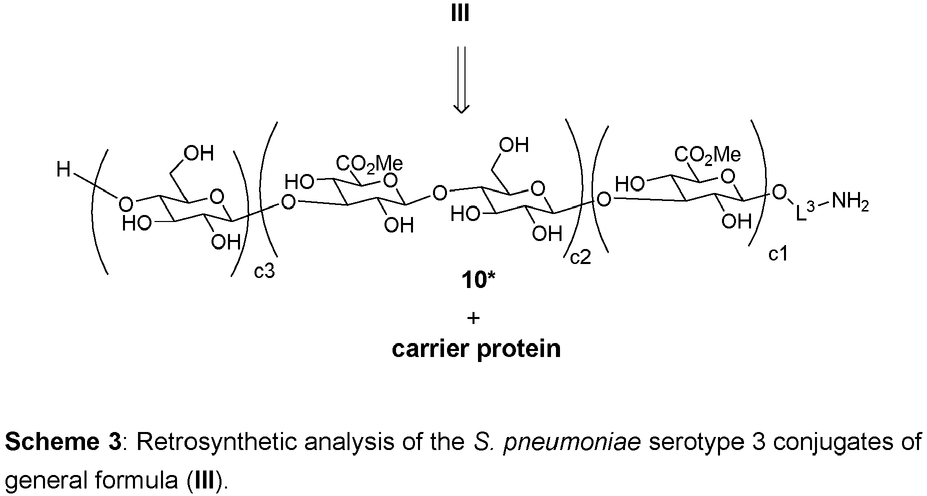

Such coupling methods include, for example, treatment of the saccharide **10*** with a commercially available bifunctional spacer (Thermo Fischer, Sigma Aldrich) such as, disuccinimidyl adipate (DSA), bis-(4-nitrophenyl) adipate, bis-(4-nitrophenyl)succinate, disuccinimidyl glutarate (DSG), bis(sulfosuccinimidyl) disuccinimidyl glutarate bis(sulfosuccinimidyl)disuccinimidyl suberate (DSS), bis(sulfosuccinimidyl)suberate (BS³), sebacic acid bis(N-succinimidyl) ester, bis(succinimidyl)penta(ethyleneglycol), bis(succinimidyl)tetra(ethyleneglycol), in presence of a base, such as trimethylamine or pyridine, followed by reaction of the resulting construct with carrier protein. Alternatively, saccharide **10*** can be reacted with a suitable dialdehyde, such as glutaraldehyde, under reductive amination condition, and the resulting construct can be subsequently treated with the carrier protein under reductive amination conditions.

Saccharides **10*** can be prepared following synthetic methods disclosed in the international patent application WO 2015/040140A1.

### Synthesis of S. pneumoniae serotype 8 conjugates of general formula (VI)

The conjugates of general formula **VI**

**[V*-Uₓ₊₃-Uₓ₊₂-Uₓ₊₁-Uₓ-O-L⁸-NH-W⁸]ₘ₈-carrier** protein **(VI)**

wherein V*-, x, Uₓ₊₃, Uₓ₊₂, Uₓ₊₁, Uₓ, L⁸, -W⁸-, m8 and carrier protein have the meanings defined herein,
can be obtained starting from a saccharide of general formula **1*** and carrier protein using coupling methods well known to the skilled person (see Scheme 4).

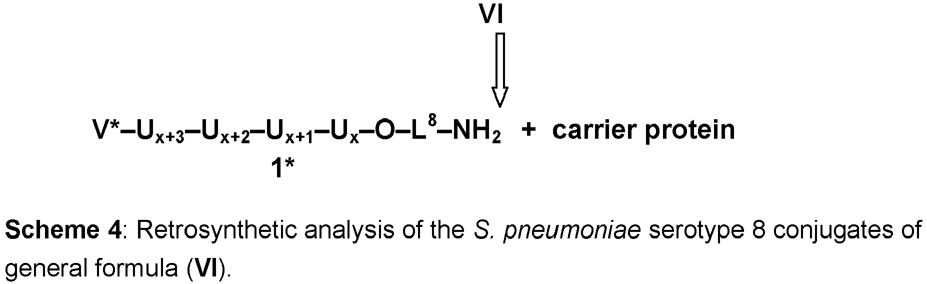

Such coupling methods include, for example, treatment of the saccharide **1*** with a commercially available bifunctional spacer (Thermo Fischer, Sigma Aldrich) such as, disuccinimidyl adipate (DSA), bis-(4-nitrophenyl) adipate, bis-(4-nitrophenyl)succinate, disuccinimidyl glutarate (DSG), bis(sulfosuccinimidyl)disuccinimidyl glutarate bis(sulfosuccinimidyl)disuccinimidyl suberate (DSS), bis(sulfosuccinimidyl)suberate (BS³), sebacic acid bis(N-succinimidyl) ester bis(succinimidyl)penta(ethyleneglycol), bis(succinimidyl) tetra(ethyleneglycol), in presence of a base, such as triethylamine, pyridine, followed by reaction of the resulting construct with carrier protein. Alternatively, saccharide **1*** can be reacted with a suitable dialdehyde, such as glutaraldehyde, under reductive amination condition, and the resulting construct can be subsequently treated with the carrier protein under reductive amination conditions.

A saccharide of general formula **1*** can be synthesized *via* several synthetic routes.

For example, saccharide **1*** can be assembled starting from thioglycoside building blocks BB2, BB3 (Angew. Chem. Int. Ed. 2013, 52, 5858; precursor for the sugar fragment U₂ and U₆), BB4, BB5, and functionalized solid support BB1 (Angew. Chem. Int. Ed. 2013, 52, 5858.) (see Scheme 5) by automated solid phase synthesis.

The synthetic process, which is summarized in **Scheme 5** involves:
- assembly of the desired oligosaccharide, which includes glycosylation with the appropriate thioglycoside (BB2, BB3, BB4 or BB5) by activation with NIS/TfOH; followed by removal of the temporary protecting group Fmoc by treatment with Et₃N;
- cleavage from the solid support; and
- removal of the permanent protecting groups.

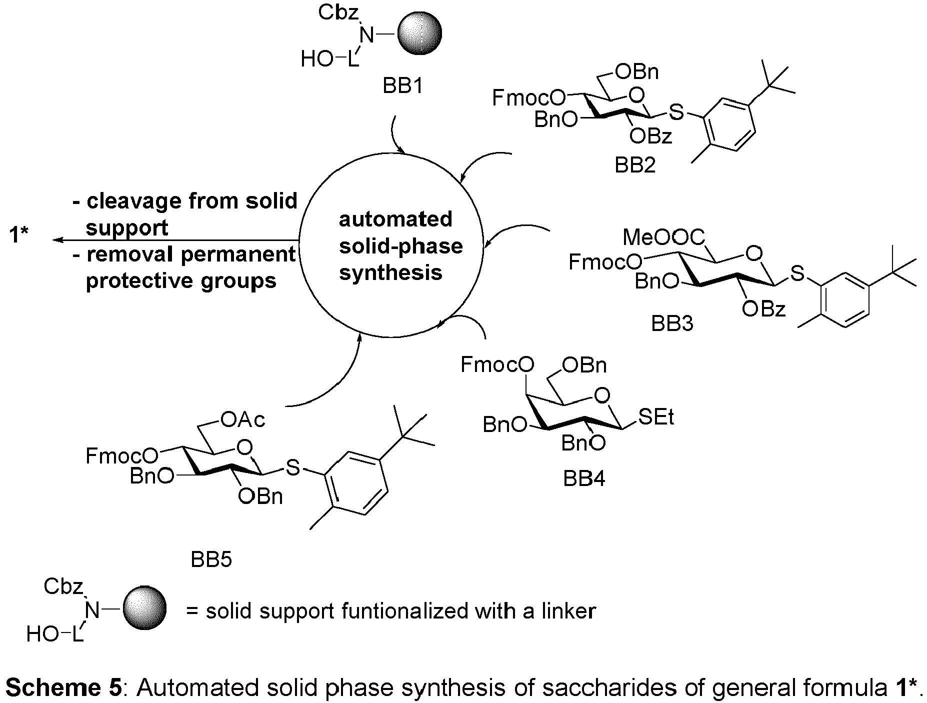

**Description of the figures**

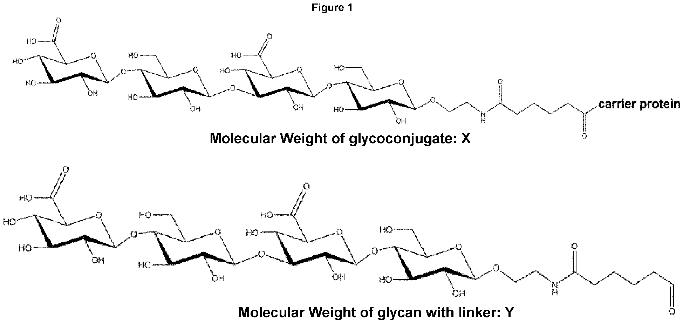

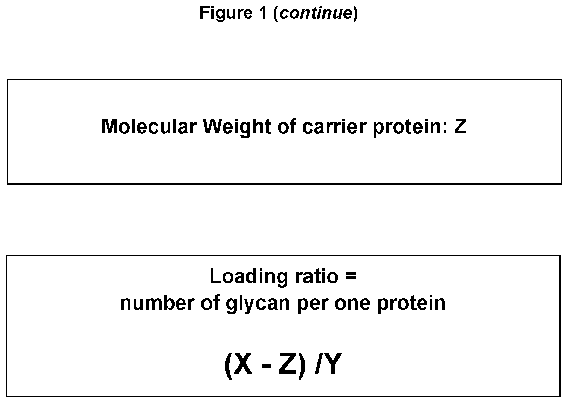

- **Figure** 1: shows calculation of loading ratio glycan on the carrier protein based on the mass of compound evaluated by MALDI-TOF-MS.

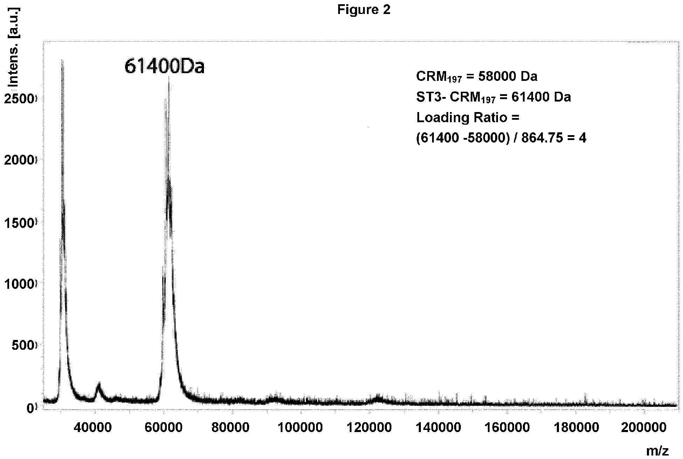

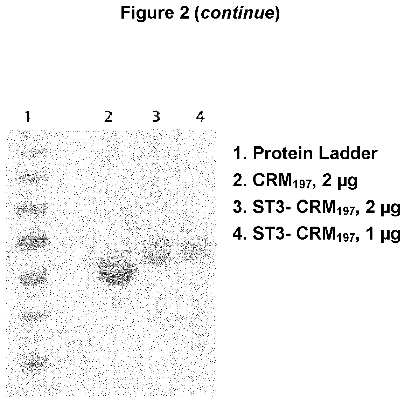

- **Figure** 2: shows characterization of ST3-tetrasaccharide CRM₁₉₇ conjugate. a) MALDI-TOF analysis was carried out to measure the average molecular size of the conjugate and the loading ratio of the glycan on the carrier protein. The recombinant CRM₁₉₇ was used as standard. b) Conjugate visualization by gel shift assay using 10% SDS-PAGE.

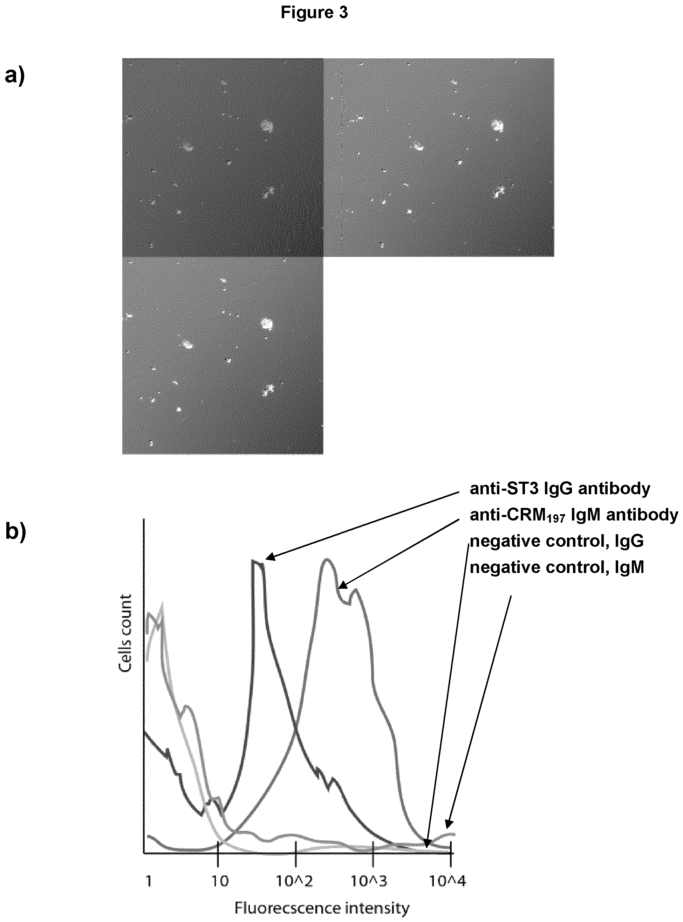

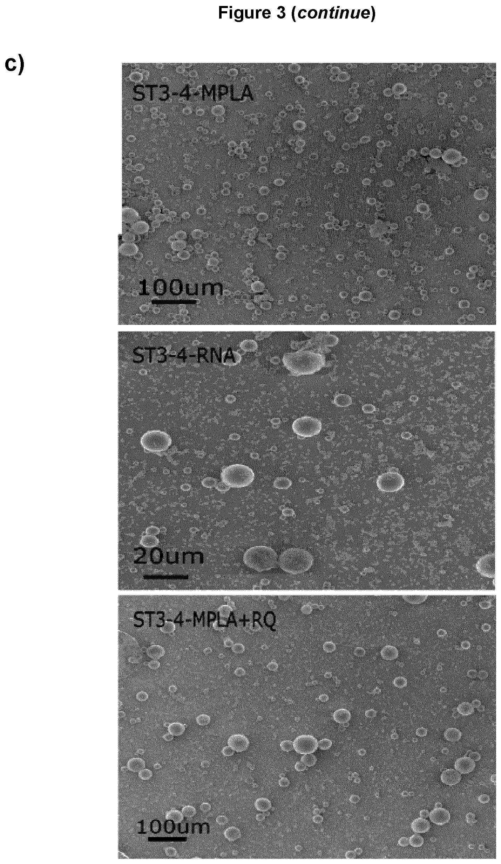

- **Figure** 3: shows exemplary characterization of conjugate formulations with PLGA particles. a) Picture of ST3-tetrasaccharide CRM₁₉₇ conjugate and resiquimod loaded PLGA particles observed under Transmission Electron Microscope (TEM). Scale bars indicated in the pictures. b) Encapsulation of ST3-tetrasaccharide CRM₁₉₇ conjugate into PLGA particles analyzed by Flow Cytometry. The shift in the intensity of the fluorescence signal (x-axis) compare to the secondary antibodies alone (anti-IgG-FITC and anti-IgM-FITC), indicates the detection of the glycan (monoclonal IgG antibodies against ST3-tetrasaccharide) and protein (monoclonal IgM antibodies against CRM₁₉₇) components of the conjugate. The same number of events was recorded for all measurements. c) Representative pictures of particles formulated with ST3- CRM₁₉₇ and MPLA (top), bacterial RNA (middle), MPLA+ resiquimod (RQ) (bottom) observed under TEM. d) Particle size and polydispersity measured by Dynamic Light Scattering (DLS). Representative pictures of particles loaded with ST3-tetrasaccharide CRM₁₉₇ conjugate and resiquimod. Graphs show the particle size by number and intensity. Measurement was repeated three times.

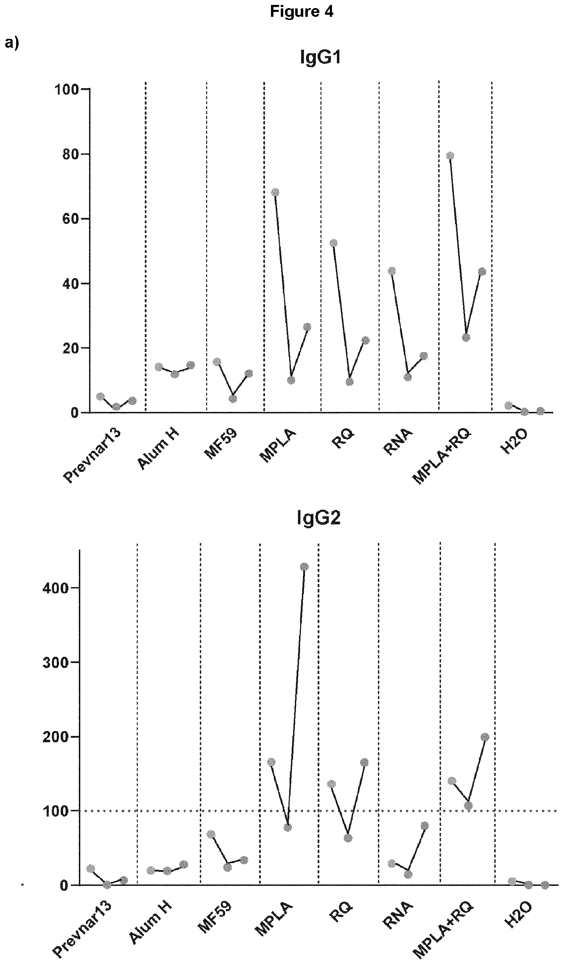

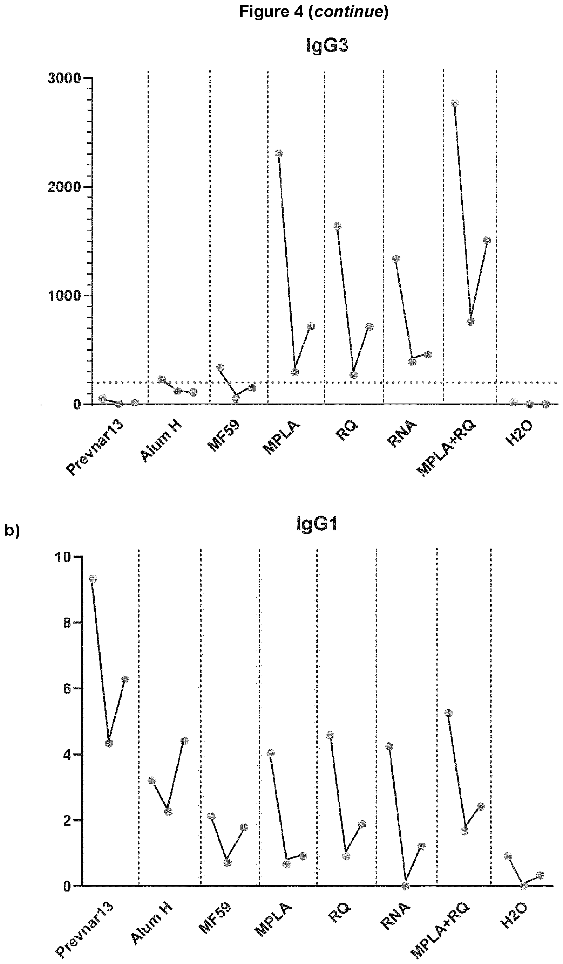

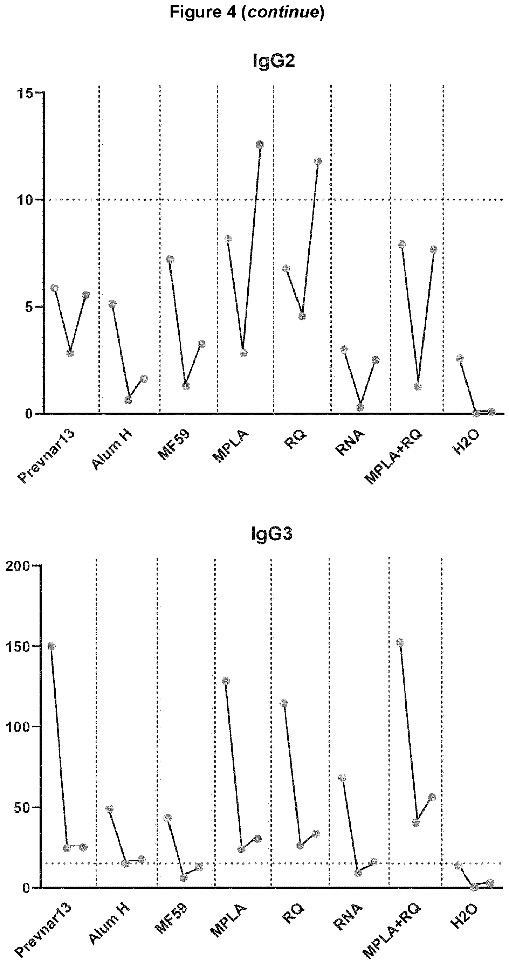

- **Figure 4**: shows immune response analysis of mice immunized with the ST3-tetrasaccharide CRM₁₉₇ conjugate in the different adjuvant formulations. a) Kinetic of ST3-tetrasaccharide specific antibody response in mice immunized with different formulations of ST3-tetrasaccharide CRM₁₉₇ conjugate seven days after the second boost, during the resting period and seven days after the final boost evaluated by glycan array analysis. b) Kinetics of cross-reactive antibody response against native capsular polysaccharide of S. *pneumoniae* serotype 3 from mice vaccinated with ST3-tetrasaccharide CRM₁₉₇ conjugate in various adjuvant formulations. Mean fluorescence intensity (MFI) of eight animals measured in triplicates were plotted. In the graphs, resiquimod is abbreviated as RQ.

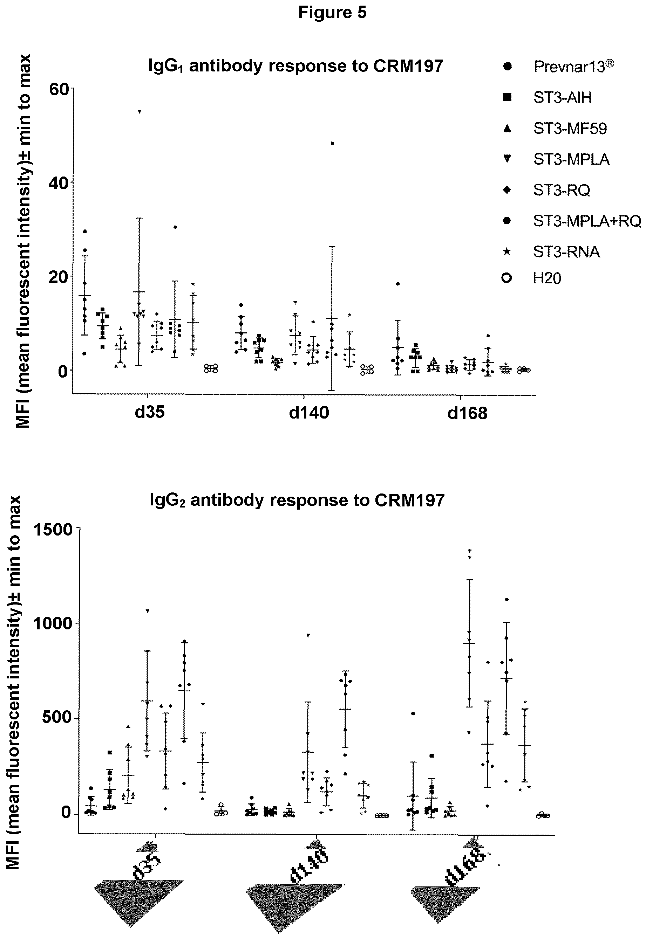

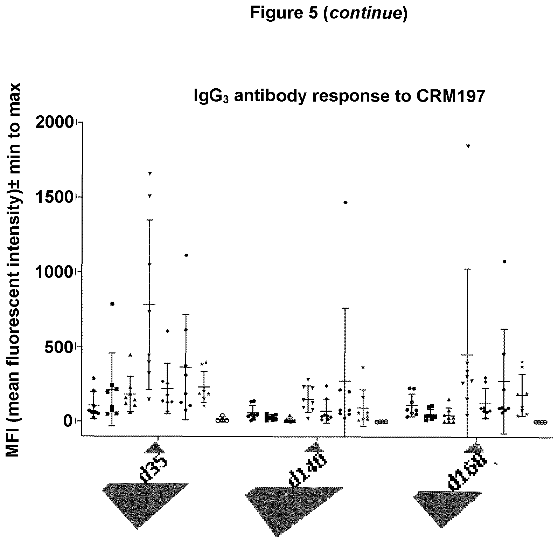

- **Figure 5**: shows evaluation of IgG₁, IgG₂, and IgG₃ antibody response to CRM₁₉₇ by glycan array in mice immunized with ST3-tetrasaccharide CRM₁₉₇ conjugate vaccine using different adjuvant formulations. Data are measurements obtained from single animals on day 35 (after 2^{nd} boost), day 148 (during the resting period) and day 168 (after final boost). Graphs show mean fluorescence intensity ± SD, *n* = 8. In the graphs, resiquimod is abbreviated as RQ.

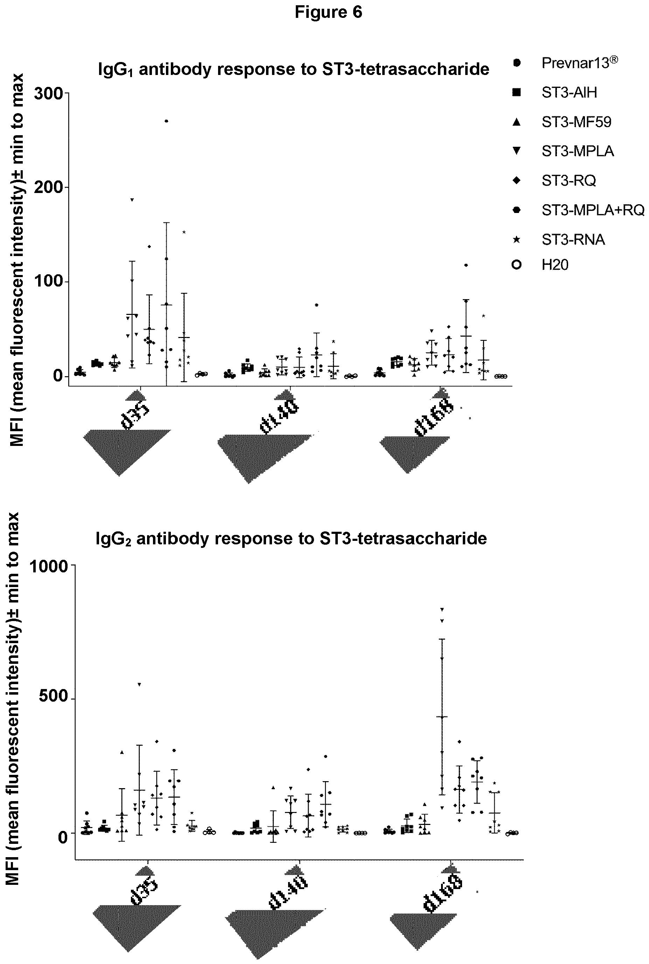

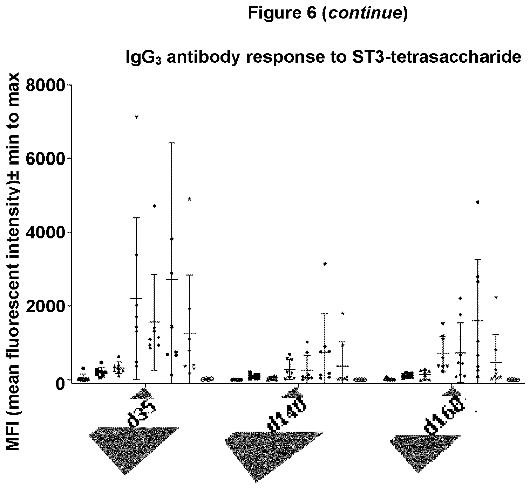

- **Figure 6**: shows evaluation of IgG₁, IgG₂, and IgG₃ antibody response to ST3-tetrasaccharide in mice immunized with ST3-tetrasaccharide CRM₁₉₇ conjugate using different adjuvant formulations. Data are glycan array measurements obtained from single animals on day 35 (after 2nd boost), day 148 (during the resting period) and day 168 (after final boost). Graphs show mean fluorescence intensity ± SD, *n* = 8. In the graphs, resiquimod is abbreviated as RQ.

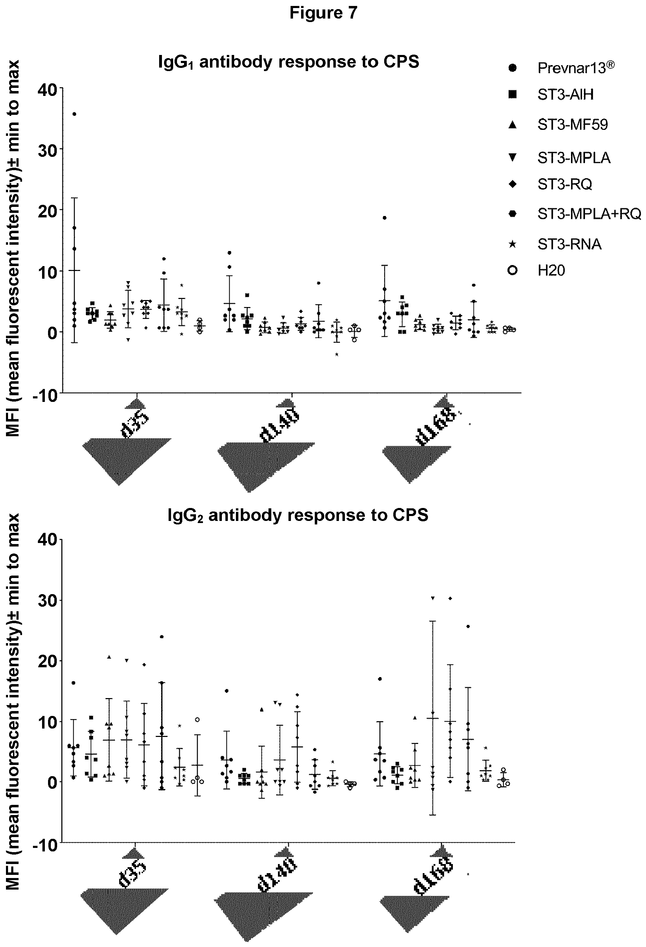

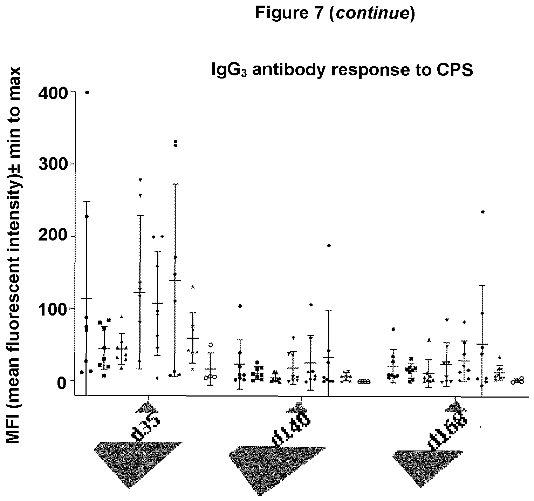

- **Figure** 7: shows evaluation of IgG₁, IgG₂, and IgG₃ antibody response to native CPS of *S*. *pneumoniae* serotype 3 in mice immunized with ST3-tetrasaccharide CRM₁₉₇ conjugate with different adjuvant formulations. Data are glycan array measurements obtained from single animals on day 35 (after the second boost), day 148 (during the resting period) and day 168 (after final boost). Graphs show mean fluorescence intensity ± SD, *n* = 8. In the graphs, resiquimod is abbreviated as RQ.

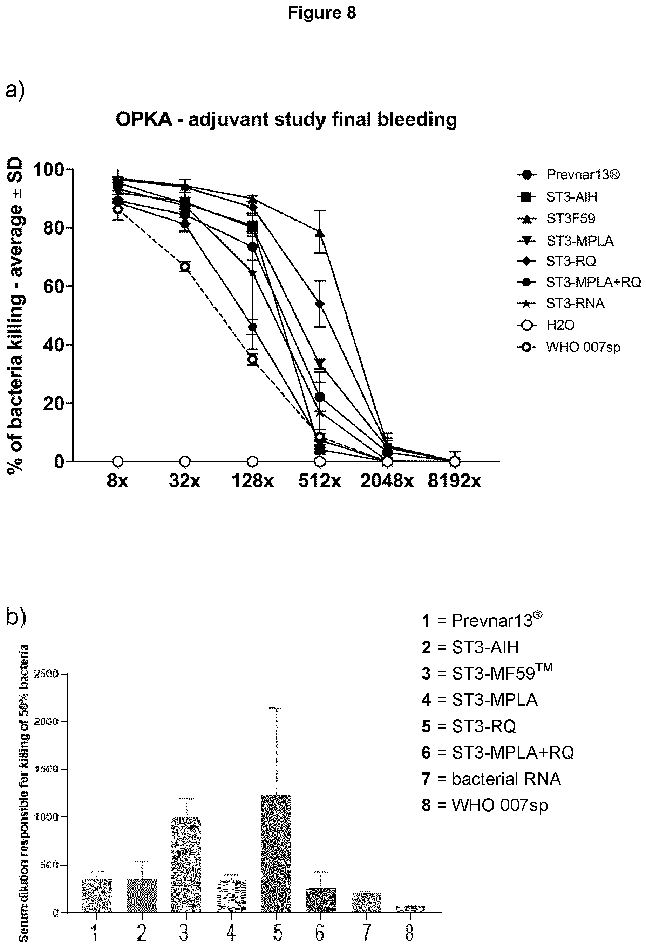

- **Figure** 8: shows in-vitro opsonophagocytic killing assay (OPKA) of sera from mice immunized with ST3-tetrasaccharide CRM₁₉₇ conjugate with different adjuvant formulations. a) A comparison of OPKA activity of pooled sera from mice immunized with ST3-tetrasaccharide CRM₁₉₇ conjugate in different adjuvants formulation. The commercial vaccine Prevnar13® and WHO human reference serum 007sp were used as controls. Data are means ± SD of CFU reduction relative to negative control wells (samples lacking either antibody or complement) of two independent experiments. b) Evaluation of serum dilution responsible for the killing of 50% bacteria by the in-vitro opsonophagocytic killing assay. Graph shows mean ± SD of results from two independent experiment estimated through non-linear interpolation of the dilution-killing OPKA data. Data were analyzed by two way ANOVA, and p-value of ≤ 0.05 was considered significant. In the graphs, resiquimod is abbreviated as RQ.

### EXAMPLES

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the scope of the invention as described in the following claims.

### Methods

### Animal experiments

Mice experiments were conducted in accordance to the guideline of the ethic committee at the University of Greifswald, the German regulations of the Society for Laboratory Animal Science (GV-SOLAS) and the European Health Law of the Federation of Laboratory Animal Science Associations (FELASA). The trial was approved by the Landesamt für Landwirtschaft, Lebensmittelsicherheit und Fischerei Mecklenburg-Vorpommern (LALLF M-V, Rostock, Germany) and the LALLF M-V ethical board (LALLF M-V permit no. 7221.3-1-061/17). All efforts were made to minimize the discomfort of the animals and ensure the highest ethical standards.

### Vaccine formulation with adjuvants and immunization

Six to eight week old female C57BL/6 mice (purchased from Janvier Labs, Saint-Berthevin, France) were immunized s.c. with 100 µL volume of ST3-tetrasaccharide CRM₁₉₇ conjugate in different adjuvant formulations, so that 60 animals were divided into eight experimental groups (Table 1). Immunization was performed according to the long-term prime + boost + boost + final boost vaccination regime (as shown in Table 3).

**Table 1. The experimental group used in the evaluation of adjuvant formulation on the effect of ST3-tetrasaccharide CRM₁₉₇ conjugated vaccine.**

| | **Group name** | **Adjuvant** | **Adjuvant** dose | **Ratio glycoconjugate**/ **adjuvant** | **Adjuvant provider** | **Formulation** |
|---|---|---|---|---|---|---|
| **1** | ST3-AIH | Aluminium hydroxide | 0.125 mg | 1 : 312.5 (w/w) | Alhydrogel® 10% (Brenntag, Denmark) | adsorption |
| **2** | ST3-MF59 | MF59™ | 50 µl | 1 : 1 (v/v) | AddaVax™ (InvivoGen, San Diego, USA) | emulsion |
| **3** | ST3-MPLA | Monophosporyl lipid A (MPLA) | 10 µg | 1 : 25 (w/w) | PHAD®, Avanti Polar Lipids (Sigma Aldrich, St. Louis, USA) | PLGA particles |
| **4** | ST3-R848 | Resiquimod (R848) | 10 µg | 1 : 25 (w/w) | InvivoGen, San Diego, USA | PLGA particles |
| **5** | ST3-MPLA + R848 | MPLA + R848 | 10 µg + 10 µg | 1 : 25 (w/w) | as for groups 3, 4 | PLGA particles |
| **6** | ST3-RNA | Isolated bacterial RNA | 5 µg | 1 : 12.5 (w/w) | - | PLGA particles |
| **7** | Prevnar13 ® | Aluminum hydroxide | 100 µl, i.e. 1/5 of the human dose | | Pfizer, New York, USA | solution |
| **8** | Empty particles | - | 100 µl | | - | PLGA particles |

All experimental groups received an amount of S. *pneumoniae* serotype 3 tetrasaccharide CRM₁₉₇ conjugate corresponding to a dose of 0.4 µg of ST3-tetrasaccharide. The conjugate was prepared as described in **Example 1.**

The ST3-tetrasaccharide formulated with Aluminium hydroxide was prepared as follows. An amount of conjugate corresponding to a dose of 0.4 µg of ST3-tetrasaccharide antigen per injection was mixed with 125 µg Aluminum Hydroxide (Al(OH)₃) from Alum gel (Alhydrogel, Brenntag, Denmark) in total volume of 100 µL of phosphate buffer (PBS pH 7.4, PAN-Biotech, Germany). Different antigen doses were calculated based on a glycan:protein ratio evaluated by MALDI-TOF-MS **(****Figure 1****).** The vaccine was rotated overnight at 4°C. The conjugates were filter sterilized using 0.22 µm small volume syringe filters. Prior to the formulation, the adjuvant was aliquoted and kept sterile, every time a fresh aliquot was used.

ST3-tetrasaccharide-CRM₁₉₇ formulated with MF59™ was formed by emulsification of the conjugate with MF59™ AddaVax™ (InvivoGen, San Diego, USA) at 1 : 1 volume ratio. The water-in-oil emulsion was created by the **"two-syringe"** technique directly before injection. Briefly, two glass syringes containing antigen and adjuvant were connected with a double-ended syringe connector. The emulsion was formed by forcing a mixture of oil-in-water adjuvant and the antigen back and forth until the material became homogeneous and viscous. The syringes were carefully disconnected, and the antigen-adjuvant emulsion was transferred to the plastic immunization syringe.
The preparation of the vaccines in the form of PLGA particles are described in the next paragraph.

Bacterial RNA from *E*. *coli* was isolated from mid-log phase cultures of DH5alpha *E. coli* using Trizol (Lige technologies, Karlsruhe, Germany) as described by Ugolini M., et al., 2018, Nat Immunol 19, 386 - 396.

### Preparation of PLGA particles and encapsulation with the S. pneumoniae olygosaccharide conjugate and the adjuvant.

Poly(lactic-co-glycolic acid) particles (PLGA) were fabricated using a water/oil/water (W1 /O/ W2) double emulsion solvent evaporation technique. The conjugate ST3-tetrasaccharide-CRM₁₉₇ was added to the internal aqueous phase (IAP, cargo). MPLA adjuvant and R848, both individually or combined, were added in the organic phase (OP). Bacterial RNA was added to the inner aqueous phase (IAP). A two-step approach was employed while 100 µL of the internal aqueous phase containing cargo (IAP or W1) was emulsified into 2 mL of the organic phase (OP or O; 50 mg/mL lactide:glycolide 50:50, 0.49 dL/g in Dichloromethane) in a 10 mL glass vial using a sonicator fitted with a microtip (output control 4, 40 W, 1 minute), resulting in the primary emulsion (W1 / O). Microparticles were fabricated by emulsifying the primary emulsion, to the external aqueous phase (EAP or W2 8 mL (2% Polyvinylalcohol-PVA, Mw 31 - 50K), 98-99 % hydrolysis in 10% sucrose) by using a homogenizer with S 18 N-10G probe (10,000 rpm, 8 min) resulting in the secondary emulsion (W1 /O / W2). The resulting secondary emulsion was poured into a glass beaker, covered with an aluminum foil punched with holes and stirred overnight at 400 rpm under a sterile bench to allow solvent evaporation and particle precipitation. The particles were collected by centrifugation at 13000 x *g* for 15 min, washed with nanopure water and lyophilized for 48 - 72 hours to obtain a fine powder. The vials were hermetically sealed and stored at 4°C until further use. The supernatant from washing steps was collected, and protein concentration was measured to assess the amount of non-capsulated antigen.

### Particle characterization

### Flow cytometry analysis (FACS) of antigen-loaded PLGA particles

Lyophilized particles were re-suspended in PBS and washed by centrifugation (3000 x g, 5 min, 4°C). Monoclonal antibodies recognizing SP3-tetrasaccharide (7A9 and 5F6 received from Pirofski's group, New York, USA) and CRM₁₉₇ (produced in-house) diluted in 1% BSA/PBS were added to the particles and incubated overnight at 4°C while rotating. The particles were washed three times with PBS and stained with anti-mouse IgG FITC (BD Biosciences, Heidelberg, Germany) and IgM- FITC (BioLegend, California, USA) diluted 1 : 100 in PBS for one hour. After three washing steps with PBS, the particles were subjected to flow cytometry using a FACS Canto II instrument (BD Biosciences, Heidelberg, Germany). 2,000 events were counted for each measurement.

### Confocal microscopy analysis of antigen-loaded PLGA particles

The encapsulation of antigen on the PLGA particles was checked by confocal microscopy analysis. Briefly, 10 mg of the particles were washed three times with PBS and incubated with the corresponding primary and secondary antibody as described above. The samples were washed three times with PBS, placed onto a glass microscope slide and covered with a coverslip. Images were acquired using a confocal microscope (LSM-700 Carl Zeiss AG).

### Estimation of encapsulated antigens onto PLGA particles

The supernatant collected after washing the particles with PBS was used to evaluate the concentration of non-capsulated glycoconjugate by BCA assay and NanoDrop.

### Transmission Electron Microscopy (TEM) of particles containing ST3-tetrasaccharide CRM₁₉₇ conjugate and different adjuvants

High-resolution imaging was performed using a Tecnai F20 X-Twin transmission electron microscope at GFZ Potsdam. The TEM is equipped with a field emission gun as an electron emitter. TEM bright-field images were acquired as energy-filtered images using a Gatan imaging filter GIF. A 20 eV window was applied to the zero-loss peak. Data were evaluated with the Gatan Digital Micrograph software package.

### Dynamic Light Scattering (DLS) Analysis

The size distribution of nanoparticles was analyzed by dynamic light scattering (DLS) measurement technique using the Zetasizer µV Nano (Malvern). Vesicles (10 µL) or 1 mg/mL nanoparticle suspension (10 µL) was diluted in 1 mL PBS or water, and three measurements were performed at 25° C, each measurement comprising of 15 scans for 14 min. Microparticle size distribution was analyzed using a Mastersizer with each measurement performed in triplicates with a size range of 0.02 - 2000 µm.

### Protein analysis: Sodium Dodecyl Sulfate Polyacrylamide Gel Electrophoresis (SDS-PAGE)

Polyacrylamide gel electrophoresis of glycoconjugates was performed according to the standard protocol. Discontinuous SDS-PAGE was prepared according to Lämmli's protocol, using a MiniProtean system (Bio-Rad, Hercules, USA). An alkaline separating gel (375 mM Tris/HCI pH 8.8, 10 to 12% (w/v) of a 29:1 acrylamide/*N*,*N'*-methylenebisacrylamide mixture) and an acidic stacking gel (100 mM Tris/HCI pH 6.8, 4.5% (w/v) of a 29:1 acrylamide/*N*,*N*'-methylenebisacrylamide mixture), polymerized by the addition of TEMED and 10% (w/v) ammonium peroxodisulfate, were used. The glycoconjugates samples were dissolved in SDS-PAGE loading buffer (200 mM Tris-CI (pH 6.8), 400 mM DTT, 8% SDS, 0.4% bromophenol blue, 40% (v/v) glycerol). An amount of 1-2 µg of glycoconjugate and CRM₁₉₇ (as a positive control) was loaded per lane and PageRuler Plus Prestained Protein Ladder 10 to 250 kDa (Thermo Scientific) was used as a size marker (2 µL per lane). All samples were run at 120 V and 25 mA for 90 min and stained with 0.5 % (w/v) Coomassie Brilliant Blue R-250 in 50 % (v/v) methanol and 10 % (v/v) acetic acid for 30 min. Stained gels were destained with 50 % (v/v) methanol and 10 % (v/v) acetic acid.

### Matrix-assisted laser desorption/ionization with time-of-flight detection mass spectrometry MALDI-TOF MS.

Mass spectra were acquired with an Autoflex Speed MALDI-TOF system (Bruker Daltonics; Bremen, Germany). Samples were spotted using the dried droplet technique with 2,5-dihydroxyacetophenone (DHAP) as matrix on MTP 384 ground steel target plates (Bruker Daltonics). Samples were prepared by mixing protein sample with DHAP matrix and 2% (v/v) trifluoroacetic acid (TFA) before spotting. The mass spectrometer was operated in linear positive mode. Mass spectra were acquired over an m/z range from 30,000 to 210,000 and data was analyzed with the FlexAnalysis software provided with the instrument.

### Glycan array serum screening

Individual synthetic oligosaccharide fragments of *S.pneumoniae* capsular polysaccharide CPS, native CPS and CRM₁₉₇ were dissolved in sterile printing buffer (50 mM sodium phosphate buffer, NaPi, pH 8.5) to the final concentration of 100 - 200 µM (volume around 20 - 50 µL). Compounds were spotted onto CodeLink N-hydroxysuccinimide-activated glass slides (SurModics Inc., Eden Prairie, USA) in two or three replicates using a contact-free piezoelectric microarray spotter (Scienion, Berlin, Germany). After the spotting was finished, slides were incubated for 16 to 24 h in a humidity box at room temperature to allow the completion of the coupling reaction and quenched for 1 h at room temperature (using 100 mM ethanolamine in 0.1 M NaPi pH 9) to suppress the free reactive groups on the microarray surface. The slide was washed with ddH₂O water, dried by centrifugation (5 min at 300x g) and stored at 4°C until use.

Directly before the assay, the slide was blocked with the blocking buffer (1 % (w/v) BSA in PBS) for one hour at room temperature or overnight at 4°C. A FlexWell grid (FlexWell 64, Grace Bio-Labs, Bend, US) was attached and 20 - 40 µL of the serum samples were applied into wells. The slide with serum samples was incubated for 1h at 37°C in a light-protected humidified box. The microarray was washed three times by applying 50 µL of washing buffer (PBS + 0.1% Tween-20) into every well. The secondary fluorescently labeled antibodies diluted in sample buffer (1% BSA (w/v) in PBS) were added (see secondary antibodies dilution in **Table 2)** and incubated for 1h at 37°C. The slide was washed three times with 50 µL PBS + 0.1% Tween-20 and ddH₂O and dried by centrifugation (5 min at 300x g).
The fluorescence read-out was performed using an Axon GenePix 4300A microarray scanner and GenePix Pro 7 software (Molecular Devices, Sunnyvale, CA, USA). Image analysis was carried out with the GenePix Pro 8 software (Graphpad Software Inc., La Jolla, USA). The photomultiplier tube (PMT) voltage was adjusted such that scans were free of saturation signals.

In order to differentiate the IgG₁, IgG₂, and IgG₃ isotype antibodies, test serum samples from immunized animals were labeled using mouse secondary monoclonal antibodies anti-IgG₁, IgG₂, or IgG₃, each labeled with different fluorescent dyes, and intensity was measured at 300 PMT.

**Table 2. Fluorescently labeled antibodies used for glycan array and Elisa**

| **Assay** | **Antibody** | **Provider** | **Catalog number** | **Dilution** |
|---|---|---|---|---|
| Glycan array | Anti-mouse IgG (H+L) FITC | Life Technologies, Carlsbad, CA, USA | A-31574 | 1:400 |
| | Anti-mouse IgG1 Alexa Fluor®594 | Life Technologies, Carlsbad, CA, USA | A-21125 | 1:400 |
| | Anti-mouse IgG2a (y2a) AlexaFluor®647 | Life Technologies, Carlsbad, CA, USA | A-21241 | 1:400 |
| | Anti-mouse IgG3 (y3) AlexaFluor®488 | Life Technologies, Carlsbad, CA, USA | A-21151 | 1:200 |
| | Anti-mouse IgM (µ chain) Alexa Fluor® 546 | Life Technologies, Carlsbad, CA, USA | A-21045 | 1:200 |
| Elisa | Anti-mouse IgG (Fc-specyfic) HRP | Dianova, Hamburg, Germany | 115-035-164 | 1:10,000 |
| | Anti-mouse IgM (µ chain) HRP | Life Technologies, Carlsbad, CA, USA | M-31507 | 1:3000 |

### Enzyme-Linked Immunosorbent Assay (ELISA) for capsular polysaccharide.

ELISA was performed using high-binding 96-well polystyrene microtiter plates (Corning, USA) coated with different capsular polysaccharides (SSI Diagnostica, Kopenhagen) at concentration 10 µg/mL (50 µL per well) in PBS (overnight incubation at 4°C). The plates were washed three times with PBS + 0.1% Tween-20 and blocked with 1% BSA-PBS at RT for 1 h. After three washing steps with PBS + 0.1% Tween-20, plates were incubated with serial dilutions of serum in duplicate or triplicate for 1 h at 37°C. The plates were washed with PBS + 0.1% Tween-20 and treated with horseradish peroxidase (HRP)-labeled secondary antibody diluted accordingly to **Table 2** in 1% BSA-PBS followed by incubation for 1 h at 37°C. The plates were washed three times with PBS + 0.1% Tween-20 and the color was developed using HRP substrate 3,3',5,5'-tetramethylbenzidine (TMB substrate; BD Biosciences, San Jose, USA). The reaction was stopped by quenching with 2% H₂SO₄. The absorbance was recorded at 450 nm using a standard ELISA plate reader.

### In-vitro opsonophagocytic killing assay (OPKA).

The *in-vitro* opsonophagocytic killing assay was performed using a standard protocol. Briefly, effector human leukemia HL-60 cells, were differentiated at concentration of 4×10⁵cells/mL in complete RPMI cell culture medium (90% RPMI 1640, 10% FCS, 1 mM L-glutamine and penicillin-streptomycin solution; PAN Biotech, Germany) added of 0.8 % dimethylformamide (DMF; 99.8% purity; Fisher Scientific, Fair Lawn, N.J., USA) for 5 - 6 days at 37°C in the presence of 5% CO₂. After differentiation, the cells were harvested by centrifugation (300 × g, 5 min) and then viable cells were counted by using 1% trypan blue exclusion and resuspended in opsonophagocytic buffer (HBSS with Ca²⁺ and Mg²⁺, 0.1% gelatin, and 10% FBS; HyClone) at a density of 1x10⁷ cells/mL directly before use.

The frozen stock of *Streptococcus pneumoniae* serotype 3 previously grown in growth medium at 37°C / 5% CO₂ to mid-log phase (OD₆₀₀= 0.3 - 0.4), was diluted in the assay buffer to a final density of 10⁶ CFU/ml. Individual or pooled sera samples (10 µL per well) were heat-inactivated and aliquoted in round bottom non-treated 96-well plates at four-fold dilution intervals (native serum follow by 1:8 to 1:8192 dilutions) and treated with the bacterial suspensions (20 µL per well) to initiate opsonization (incubation for 15 min at 37°C). After preopsonization, 10 µL of external complement source (baby rabbit complement, CedarLane, Ontario, Canada) as well as 4×10⁵ differentiated HL-60 cells in a volume of 40 µL were added to each well (phagocyte: bacteria ratio 400:1) and plates were incubated for 45 min at 37°C in 5% CO₂ environment (preferably with shaking at 220 rpm). The phagocytic reaction was stopped by putting the plate on ice for 20 min. Viable extracellular pneumococci were determined by plating aliquots (5 µL) from each well on Columbia Agar plates with 5% (v/v) sheep blood (BD, New Jersey, USA) and incubating at 37°C in 5% CO_{2.} for several hours to allow bacteria growing (6-8 in case of *S.pneumoniae* serotype 3). Visible colony forming units (CFU) were counted. Negative controls lacking either antibody, HL-60 or complement, as well as standard control WHO 007sp typing serum (Human Anti-Pneumococcal capsule Reference Serum, NIBSC, Herts, UK) were used. The assay was repeated two to three times independently. Percentage killing of bacteria was calculated as CFU reduction relative to negative control wells. Serum dilution responsible for 50% killing of bacteria was estimated through non-linear interpolation of the dilution-killing OPKA data.

### Example 1 Preparation of S. pneumoniae synthetic saccharide conjugate.

S. *pneumoniae* serotype 3 synthetic tetrasaccharide (ST3) was conjugated to CRM₁₉₇ using a standardized protocol as described below.
*S.pneumoniae* tetrasaccharide (ST3-tetrasaccharide) **(1)** and bis (4-nitrophenyl) adipate **(2)** were combined at 1 to 6.5 equivalent in DMSO and pyridine in v/v ratio 1.6. Then 180 equivalents of triethylamine were added, and the solution was stirred for three hours at room temperature. The resulting solution was frozen in liquid nitrogen and subsequently lyophilized to give a crude white solid. Excess bis (4-nitrophenyl) adipate was removed by washing the crude solid with chloroform (3 x 1 mL) and dichloromethane (3 x 1 mL) until no more bis (4-nitrophenyl) adipate was observed by the Thin-layer Chromatography (TLC). The resulting compound (3) was taken forward to the next reaction without additional purification.

To a pre-weighed vial of CRM₁₉₇ carrier protein **(4;** Pfenex, California, USA) was added autoclaved water. The solution was transferred to an Amicon 10K filter and centrifuged for 8 min at 10,000 rpm. The filtrate was discarded, and the remaining solution was added to the filter and centrifuged for 8 min at 10,000 rpm. The CRM₁₉₇ vial was washed with autoclaved water, transferred to the filter and centrifuged for 8 min at 10,000 rpm. The protein vial was then washed with phosphate buffer (pH 8) **(4),** transferred to the filter and centrifuged for 8 min at 10,000 rpm. Subsequently, the filter was removed, turned upside down into a clean vial and centrifuged for 2 min at 1,000 rpm, giving a colorless filtrate containing CRM₁₉₇.

The filtrate was added to a vial containing the tetrasaccharide-linker construct **(3).** The resulting solution **(5)** was stirred for 18 h, after which the solution was transferred to an Amicon 10K filter and washed with sodium phosphate buffer pH 8 (2 x 400 µL). The sample was centrifuged for 8 min at 10,000 rpm, followed by additional washing with autoclaved water (3 x 400 µL). Prior to the third wash, autoclaved water (200 µL) was added to the filter with thorough mixing. A small sample (10 µL) was taken for MALDI-TOF-MS analysis. Autoclaved water (200 µL) was added to the filter and centrifuged for 8 min at 10,000 rpm. The sample was washed with PBS (400 µL). Afterward, the filter was removed and turned upside down into a clean vial and centrifuged for 2 min at 1,000 rpm. The filtrate was diluted with PBS (350 µL) and stored at 5 °C.

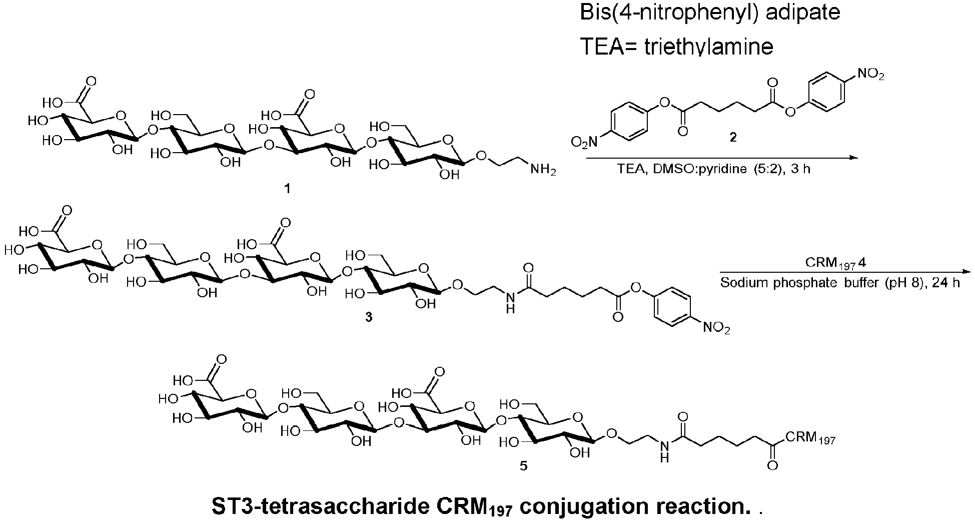

At the end of the procedure, glycoconjugates were characterized by MALDI-TOF and by a gel shift assay **(****Figure 2**). The average loading ratio of four glycans per protein was obtained. The loading ratio can vary from batch to batch depending on, e.g., the amount of glycan used in the particular reaction.

### Example 2. Formulation of ST3-tetrasaccharide CRM₁₉₇ Conjugate Vaccine with different adjuvants

Given that carbohydrate antigens are weaker immunogens than proteins, the proper choice of the adjuvant formulation is the major challenge during the carbohydrate vaccine development process, to improve immunogenic and protective potential as well as safety. Therefore, the ST3-tetrasaccharide CRM₁₉₇ conjugate, obtained as described in **Example 1**, was formulated with various adjuvants **(Table 1**).

The particular vaccine formulation was decided according to the chemical and biological properties of the adjuvant. All experimental groups contained the same dose of antigen equal to 0.4 µg of synthetic ST3-tetrasaccharide conjugated to the CRM₁₉₇.

The amount of adjuvant was decided on the basis of commercially available vaccine formulation or published data. Given the long-term success of "gold standard" aluminum adjuvant, the ST3-tetrasaccharide CRM₁₉₇ conjugate formulated with aluminium hydroxide was used as control group (**Table 1**)**.** The antigen was adsorbed onto 125 µg alum (Al(OH)₃) as an aluminum source (Alhydrogel, Brenntag, Denmark).

The adjuvants tested were Toll-Like receptors (TLRs) ligands: monophosphoryl lipid A (MPLA), a derivative of lipopolysaccharide, resiquimod (R848), and bacterial RNA (groups 3 - 6). MPLA and resiquimod (TLR4 and TLR7/8 activators, respectively) were chosen to prove if synergistic effects may result in a more effective or longer-lasting immune response. As these adjuvants can not be chemically attached to the antigen, nanoparticles were selected as a delivery system.
Poly(lactic-co-glycolic acid) (PLGA) particles made of biodegradable approved for human-use polyester offers the possibility to delivery the antigen and immunostimulatory molecules to the immune cells in more natural spherical structures. Therefore, these particles were used in the formulations of ST3-tetrasaccharide - CRM₁₉₇ conjugate with the TLR ligands MPLA, resiquimod, and bacterial RNA. Particles were prepared by a water/oil/water double emulsion solvent evaporation technique. For experimental groups 3 to 6, the ST3-tetrasaccharide CRM₁₉₇ conjugate was encapsulated together with the different adjuvants into biodegradable PLGA particles. The immunogenic potency of groupds 1 - 6 was compared to that of Prevnar13® (group 7), and to that of dummy control particles (group 8), prepared without antigen or adjuvant.
Moreover, it was tested a formulation containing the ST3-tetrasaccharide CRM₁₉₇ conjugate adjuvanted with MF59™ (group 2); to prepare the vaccine, an emulsion was prepared by the "two-syringes" method in 1 : 1 volume ratio of antigen and adjuvant, as described in the method section.

### Example 3. Characterization of the formulations with PLGA particles.

Particle characterization is a crucial step before further development for applications. Therefore, the presence of the adjuvant and antigen, size and distribution of the particles were determined for group 3 - 6.
The flow-through solutions after washing steps were monitored by Nano-Drop and BCA assay for the presence of the non-encapsulated conjugate. In this analysis, the ST3-tetrasaccharide CRM₁₉₇ conjugate was not detected at any point, so that the encapsulation efficiency was assumed to be 100 %.

The encapsulation and localization of the antigen on the particles were analysed by flow cytometry and confocal microscopy analysis. ST3-tetrasaccharide CRM₁₉₇ conjugate encapsulated particles were incubated with monoclonal antibodies specific for ST3 or CRM₁₉₇, followed by detection using a FITC conjugated secondary antibody. Confocal microscopy analysis revealed the specific surface localization of ST3-tetrasaccharide CRM₁₉₇ on the particles (**Figure 3a**). The presence of carbohydrate and protein components on the particles were analysed by flow cytometry. The shift of the fluorescence signal to the right confirmed the presence of both carbohydrate and protein components on the particles (**Figure 3b**)**.** Surface localization of the ST3-tetrasaccharide antigen is an important feature considering that polysaccharide specific immune responses are primarily mediated by cross-linking of the B cell receptor.

The dimension of particles loaded with antigen and adjuvant was analyzed by dynamic light scattering (DLS) measurement and scanning electron microscopy (SEM). The size distribution by intensity and number indicated that the average magnitude of the particles encapsulated with ST3-tetrasaccharide CRM₁₉₇ and adjuvant was between 300 - 500 nm depending on the formulation (**Figure 3c** **and** d).

### Example 4. Immune response induced by immunization with the different formulations containing the ST3-tetrasaccharide CRM₁₉₇.

Six to eight-week-old C57BL/6 mice were s.c immunized with different vaccine formulations, described in Example 2, containing an amount of ST3-tetrasaccharide CRM₁₉₇ conjugate corresponding to a dose of 0.4 µg of ST3-tetrasaccharide antigen per injection. Animals were vaccinated according to the long-term immunization regime prime + boost + boost + final boost (Table 3). Blood was collected, and the antibody response was analyzed.

**Table 3. Immunization regime for adjuvant formulation study**

| **days** | **procedure** |
|---|---|
| **d0** | **Immunization,** bleeding |
| **7** | Bleeding |
| **14** | **1^{st} boost** |
| **21** | Bleeding |
| **28** | **2^{nd} boost** |
| **35** | Bleeding |
| **42** | Bleeding |
| **49-140** | Resting period |
| **140** | Bleeding |
| **161** | **Final boost** |
| **168** | Bleeding |
| **175** | Dissection |

It is known that the IgG subclasses induced after immunization of mice are an indirect measurement of Th₂-type versus Th₁-type immune response. The production of IgG₁-type antibodies is principally induced by Tₕ2-type cytokines, whereas the IgG₂ and IgG₃ reflect the involvement of Tₕ1-type cytokines. A specific IgG subclass can contribute to the clearance of encapsulated bacteria by particular mechanisms. Murine IgG₃ is highly protective, while the IgG₂a, IgG₂b, and IgG₁ isotypes are poorly or non-protective and have a significantly lower affinity. Mice IgG₂ display the strongest binding to Fc receptors and together with IgG₃ can activate the complement better than IgG₁ (IgG₃>IgG₂b>IgG₂a>IgG₁). Therefore, an immune response with a broad subclass distribution may be beneficial against encapsulated bacteria. In human IgG₃ and IgG₁ are the best complement activators.

The effect of adjuvant formulation on the Tₕ1 and Tₕ2 profile of humoral immune responses was analyzed by determining the level of IgG₁, IgG₂, and IgG₃ antibodies recognizing the carrier protein CRM₁₉₇ (**Figure 5**)**,** the synthetic ST3-tetrasaccharide (**Figure 6**), or native capsular polysaccharide (CPS) of *S.pneumoniae* serotype 3 (**Figure 7**)**.** To this aim, blood samples from single animals were screened by glycan arrays using secondary antibodies recognising IgG₁, IgG₂ or IgG₃ conjugated to different fluorescent dyes. Moreover, the levels of IgG₁, IgG₂, and IgG₃ antibodies were analysed at different time points to analyse the influence of the adjuvant formulation on the immune response kinetic (**Figure 4**)**.**
It was found that the different adjuvants influence the antibody response kinetic, probably because they differ in the mode of action. MF59™ promptly stimulates an early immune response while alum entraps the antigen and slows down antibody production two weeks after priming. The analysis revealed that the kinetic of the humoral response induced by ST3-tetrasaccharide CRM₁₉₇ conjugate strongly depends on the adjuvant formulation. From the results, MPLA seemed to induce the best kinetic response of antibody titer and IgG₂ levels.
Animals immunized with ST3-tetrasaccharide CRM₁₉₇ conjugate in combination with Tₕ1 stimulating adjuvants (MPLA, R848, bacterial RNA as well as a combination of MPLA and R848) encapsulated in PLGA particles showed high anti-ST3-tetrasaccharide IgG1 and IgG₃ titer after the second boost (**Figure 6**)**.** The same groups demonstrated an excellent IgG₂ response just after the final boost. These results suggest the presence of oligosaccharide specific memory B-cells, which are responsable for a quick and specific recognition of previously encountered antigens and a corresponding high immune response. Thus, using TLR ligands MPLA, R848, RNA as adjuvants substantially shifted the immune response toward a Tₕ1 phenotype.

The profile of IgG₁, IgG₂, and IgG₃ antibodies specific for *S.pneumoniae* serotype 3 capsular polysaccharide (CPS) (**Figure 7**) differed from that specific for ST3-tetrasaccharide. The vaccine formulated with alum and MF59™ evoked a prevalence of the IgG₁ subclass, indicative of a Tₕ2-biased response, while the MPLA, R848, and RNA strongly indicated a Tₕ1-biased response by induction of IgG₂ and IgG₃ subclasses. Improved production of IgG₂ antibodies was similarly observed for the formulation of ST3-tetrasaccharide CRM₁₉₇ conjugate with MF59™ even though the adjuvant is considered more Tₕ2.

In summary, the choice of adjuvant played a significant role in the level of involvement of the Tₕ1 and Tₕ2 responses in mice immunized with ST3-tetrasaccharide CRM₁₉₇ conjugate.

### Example 5. Bacterial killing mediated by the serum of immunized animals.

Polysaccharide specific antibodies are the primary protective mechanism against pneumococcal bacteria. To determine antibody-mediated bacterial killing *in-vitro,* an opsonophagocytic killing assay (OPKA) was performed. This very well-established method evaluates the vaccine-induced immunoprotection by measuring the reduction of viable bacteria mediated by the serum of immunized animals.

The OPKA results (**Figure 8**) indicated that the R848 with PLGA particles and MF59™ are the best candidates for the formulation of the ST3-tetrasaccharide CRM₁₉₇ conjugate, as the serum dilutions responsible for 50% killing of bacteria were the highest between the different tested formulations, with values of 1236 and 998, respectively. The other tested groups showed similar results as Prevnar13®. The serum dilution for the MPLA group was 341; for the combination of MPLA and R848 group was 260 and for the bacterial RNA group was 203. The serum dilution for the control Prevnar13® vaccinated group equaled 357. For the aluminium hydroxide group, the serum dilution was 360.

## Claims

1. A vaccine comprising
a) at least one glycoconjugate of a synthetic *Streptococcus pneumoniae* oligosaccharide and a carrier protein;
b) at least one adjuvant, wherein the at least one adjuvant is selected from agonists of toll like receptors 1, 2, 4, 6, 7, 8, or 9,
wherein the at least one glycoconjugate and the at least one adjuvant are encapsulated in biocompatible and biodegradable polymer particles.

2. The vaccine according to claim 1, wherein the biocompatible and biodegradable polymer particles are selected from poly(lactic-co-glycolic acid) particles, polylactic acid particles, polyglycolic acid particles, poly(lactic-co-glycolic acid) / polyethylene glycol co-polymers particles and tri-block poly(lactic-co-glycolic acid)/ polyethylene glycol/ poly(lactic-co-glycolic acid)/ copolymers particles.

3. The vaccine according to claim 1 or 2, wherein the carrier protein is selected from CRM₁₉₇, detoxified pneumolysin, an immunogenic fragment of detoxified pneumolysin, pneumococcal surface protein A, and an immunogenic fragment of pneumococcal surface protein A.

4. The vaccine according to any one of the claims 1 to 3, wherein the carrier protein is CRM₁₉₇.

5. The vaccine according to any one of the claims 1 to 4, wherein the at least one adjuvant is selected from monophosphoryl lipid A, an 1*H*-imidazo[4,5-c]quinoline compound, preferably resiquimod, a mixture of monophosphoryl lipid A and resiquimod, and isolated bacterial RNA.

6. The vaccine according to any one of the claims 1 to 5, wherein a ratio of the at least one glycoconjugate and the at least one adjuvant is comprised between 1 : 1 and 1 : 100 (w/w).

7. The vaccine according to any one of the claims 1 to 6, wherein the synthetic *Streptococcus pneumoniae* oligosaccharide is selected from the group of serotypes comprising serotypes 1, 2, 3, 4, 5, and 8.

8. The vaccine according to any one of the claims 1 to 7, wherein the synthetic *Streptococcus pneumoniae* oligosaccharide is of *Streptococcus pneumoniae* serotype 3.

9. The vaccine according to any one of the claims 1 to 8, wherein the at least one glycoconjugate has the general formula (**IIII**)
**[H-(C)_{c3}-(D-C)_{c2}-(D)_{c1}-O-L³-NH-W³]ₘ₃- carrier protein** **(III)**
wherein
C represents: 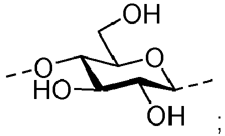
D represents: 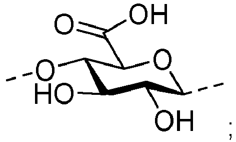
c2 is an integer selected from 1, 2, 3, 4, 5, 6, 7, 8, 9 and 10;
c1 and c3 represent independently of each other an integer 0 or 1;
L³ represents a linker;
m³ is an integer between 2 and 18;
-W³- is selected from: 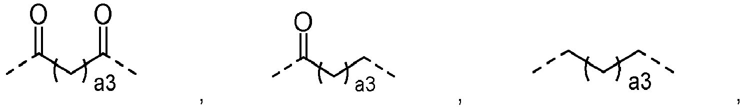 and 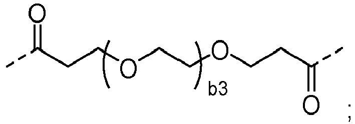
a3 represents an integer from 1 to 10;
b3 represents an integer from 1 to 4; and
carrier protein has the meaning as defined in claim 3 or 4.

10. The vaccine according to any one of the claims 1 to 9, wherein the at least one glycoconjugate has the following formula (**IIIa**) 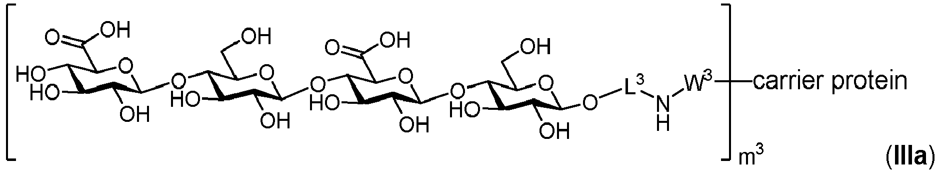
wherein **m³** represents an integer from 3 to 12;
**L³** represents -CH₂CH₂-;
**W³** represents -COCH₂CH₂CH₂CH₂CO-, or -COCH₂CH₂CH₂CH₂CH₂;
carrier protein has the meaning as defined in claim 3 or 4; and
the at least one adjuvant is selected from monophosphoryl lipid A, resiquimod, a mixture of monophosphoryl lipid A and resiquimod, and isolated bacterial RNA.

11. The vaccine according to any of the claims 1 to 10, further comprising at least one immunogenic component, wherein the at least one immunogenic component is selected from the group consisting of:
detoxified pneumolysin (Ply), or an immunogenic fragment thereof;
a pneumococcal surface antigen A, or an immunogenic fragment thereof,
a pneumococcal surface protein A, or an immunogenic fragment thereof,
and a glycosphingolipid.

12. The vaccine according to any one of the claims 1 to 11, further comprising one or more glycoconjugates of capsular polysaccharides from *Streptococcus pneumoniae* serotypes 1, 3, 4, 5, 6A, 6B, 7F, 9V, 14, 18C, 19A, 19F and 23F individually conjugated to CRM₁₉₇ carrier protein.

13. The vaccine according to any one of the claims 1 to 12 for use in the prevention and/or treatment of a disease caused by *Streptococcus pneumoniae* in a human or an animal host.

14. The vaccine for use according to claim 13, for use in the prevention and/or treatment of a disease caused by *Streptococcus pneumoniae* serotype 3.

15. The vaccine for use according to claim 13 or 14, wherein the disease caused by *Streptococcus pneumoniae* includes pneumonia, meningitis, otitis media, bacteremia and acute exacerbation of chronic bronchitis, invasive pneumococcal disease, exacerbation of chronic obstructive pulmonary disease, sinusitis, arthritis and conjunctivitis.
